# EUROPEAN PATENT APPLICATION

(11) **EP 1 273 915 A2**
(43) Date of publication of application: **08.01.2003**
(21) Application number: 02013807.9
(22) Date of filing: 21.06.2002
(51) Int. Cl.: G01N 33/52

(54) **Biochemical analysis kit and method for exposing a stimulable phosphor sheet**

(30) Priority: 02.07.2001 JP 2001201196
(71) Applicant: FUJI PHOTO FILM CO., LTD., Kanagawa-ken (JP)
(72) Inventor: Ogura, Nobuhiko, Kaisei-machi, Ashigarakami-gun, Kanagawa (JP)
(74) Representative: Klunker . Schmitt-Nilson . Hirsch

(57) **Abstract**

A biochemical analysis kit includes a biochemical analysis unit including a substrate formed with a plurality of absorptive regions to be spaced apart from each other and a stimulable phosphor sheet including a support formed with a plurality of stimulable phosphor layer regions to be spaced apart from each other in substantially the same pattern as that of the plurality of absorptive regions formed in the substrate of the biochemical analysis unit. According to the thus constituted biochemical analysis kit, it is possible to produce biochemical analysis data having excellent quantitative characteristics with high resolution by selectively labeling the absorptive regions with a radioactive labeling substance or a labeling substance which generates chemiluminescence emission when it contacts a chemiluminescent substrate, superposing a stimulable phosphor sheet formed with stimulable phosphor layer regions on the biochemical analysis unit, exposing the stimulable phosphor layer regions to the radioactive labeling substance contained in the absorptive regions or chemiluminescence emission released from the absorptive regions to record radiation data or chemiluminescence data in the stimulable phosphor layer regions, stimulating the stimulable phosphor layer regions with a stimulating ray and detecting stimulated emission released from the stimulable phosphor layer regions.

## Description

### BACKGROUND OF THE INVENTION

The present invention relates to a biochemical analysis kit and a method for exposing a stimulable phosphor sheet and, particularly, to a biochemical analysis kit and a method for exposing a stimulable phosphor sheet, which can prevent noise caused by the scattering of electron beams (β rays) released from a radioactive labeling substance selectively contained in a plurality of spot-like regions of a biochemical analysis unit from being generated in biochemical analysis data and produce biochemical analysis data having an excellent quantitative characteristic by reading radiation data with high resolution even in the case of forming a plurality of spot-like regions containing specific binding substances, which can specifically bind with a substance derived from a living organism and whose sequence, base length, composition and the like are known, in the biochemical analysis unit at a high density, selectively labeling the plurality of spot-like regions of the biochemical analysis unit with a radioactive labeling substance to record radiation data therein, facing the thus prepared biochemical analysis unit toward a stimulable phosphor layer of a stimulable phosphor sheet to expose the stimulable phosphor layer to a radioactive labeling substance, irradiating the thus exposed stimulable phosphor layer with a stimulating ray, and photoelectrically detecting stimulated emission released from the stimulable phosphor layer to produce biochemical analysis data, and can also prevent noise caused by the scattering of chemiluminescence emission selectively released from a plurality of spot-like regions of a biochemical analysis unit from being generated in biochemical analysis data and produce biochemical analysis data having an excellent quantitative characteristic by reading radiation data with high resolution even in the case of forming a plurality of spot-like regions containing specific binding substances, which can specifically bind with a substance derived from a living organism and whose sequence, base length, composition and the like are known, in the biochemical analysis unit at a high density, selectively labeling the plurality of spot-like regions of the biochemical analysis unit with a labeling substance which generates chemiluminescence emission when it contacts a chemiluminescent substrate to record chemiluminescence data therein, bringing the plurality of spot-like regions of the biochemical analysis unit into contact with a chemiluminescent substrate, thereby causing the plurality of spot-like regions of the biochemical analysis unit to release chemiluminescence emission, facing the biochemical analysis unit releasing chemiluminescence emission toward a stimulable phosphor layer of a stimulable phosphor sheet to expose the stimulable phosphor layer to chemiluminescence emission, irradiating the thus exposed stimulable phosphor layer with a stimulating ray, and photoelectrically detecting stimulated emission released from the stimulable phosphor layer to produce biochemical analysis data.

### DESCRIPTION OF THE PRIOR ART

An autoradiographic analyzing system using as a detecting material for detecting radiation a stimulable phosphor which can absorb, store and record the energy of radiation when it is irradiated with radiation and which, when it is then stimulated by an electromagnetic wave having a specified wavelength, can release stimulated emission whose light amount corresponds to the amount of radiation with which it was irradiated is known, which comprises the steps of introducing a radioactively labeled substance into an organism, using the organism or a part of the tissue of the organism as a specimen, superposing the specimen and a stimulable phosphor sheet formed with a stimulable phosphor layer for a certain period of time, storing and recording radiation energy in a stimulable phosphor contained in the stimulable phosphor layer, scanning the stimulable phosphor layer with an electromagnetic wave to excite the stimulable phosphor, photoelectrically detecting the stimulated emission released from the stimulable phosphor to produce digital image signals, effecting image processing on the obtained digital image signals, and reproducing an image on displaying means such as a CRT or the like or a photographic film (see, for example, Japanese Patent Publication No. 1-60784, Japanese Patent Publication No. 1-60782, Japanese Patent Publication No. 4-3952 and the like).

There is further known chemiluminescence analysis system comprising the steps of employing, as a detecting material for light, a stimulable phosphor which can absorb and store the energy of light upon being irradiated therewith and release a stimulated emission whose amount is proportional to that of the received light upon being stimulated with an electromagnetic wave having a specific wavelength range, selectively labeling a fixed high molecular substance such as a protein or a nucleic acid sequence with a labeling substance which generates chemiluminescence emission when it contacts a chemiluminescent substance, contacting the high molecular substance selectively labeled with the labeling substance and the chemiluminescent substance, storing and recording the chemiluminescence emission in the wavelength of visible light generated by the contact of the chemiluminescent substance and the labeling substance in the stimulable phosphor contained in a stimulable phosphor layer formed on a stimulable phosphor sheet, scanning the stimulable phosphor layer with an electromagnetic wave to excite the stimulable phosphor, photoelectrically detecting the stimulated emission released from the stimulable phosphor to produce digital signals, effecting data processing on the obtained digital signals, and reproducing data on displaying means such as a CRT or a recording material such as a photographic film (see for example, U.S. Patent No. 5,028,793, UK Patent Application 2,246,197 A and the like).

Unlike the system using a photographic film, according to these systems using the stimulable phosphor as a detecting material, development, which is chemical processing, becomes unnecessary. Further, it is possible reproduce a desired image by effecting image processing on the obtained image data and effect quantitative analysis using a computer. Use of a stimulable phosphor in these processes is therefore advantageous.

On the other hand, a fluorescence analyzing system using a fluorescent substance as a labeling substance instead of a radioactive labeling substance in the autoradiographic analyzing system is known. According to this system, it is possible to study a genetic sequence, study the expression level of a gene, and to effect separation or identification of protein or estimation of the molecular weight or properties of protein or the like. For example, this system can perform a process including the steps of distributing a plurality of DNA fragments on a gel support by means of electrophoresis after a fluorescent dye was added to a solution containing a plurality of DNA fragments to be distributed, or distributing a plurality of DNA fragments on a gel support containing a fluorescent dye, or dipping a gel support on which a plurality of DNA fragments have been distributed by means of electrophoresis in a solution containing a fluorescent dye, thereby labeling the electrophoresed DNA fragments, exciting the fluorescent dye by a stimulating ray to cause it to release fluorescence emission, detecting the released fluorescence emission to produce an image and detecting the distribution of the DNA fragments on the gel support. This system can also perform a process including the steps of distributing a plurality of DNA fragments on a gel support by means of electrophoresis, denaturing the DNA fragments, transferring at least a part of the denatured DNA fragments onto a transfer support such as a nitrocellulose support by the Southern-blotting method, hybridizing a probe prepared by labeling target DNA and DNA or RNA complementary thereto with the denatured DNA fragments, thereby selectively labeling only the DNA fragments complementary to the probe DNA or probe RNA, exciting the fluorescent dye by a stimulating ray to cause it to release fluorescence emission, detecting the released fluorescence emission to produce an image and detecting the distribution of the target DNA on the transfer support. This system can further perform a process including the steps of preparing a DNA probe complementary to DNA containing a target gene labeled by a labeling substance, hybridizing it with DNA on a transfer support, combining an enzyme with the complementary DNA labeled by a labeling substance, causing the enzyme to contact a fluorescent substance, transforming the fluorescent substance to a fluorescent substance having fluorescence emission releasing property, exciting the thus produced fluorescent substance by a stimulating ray to release fluorescence emission, detecting the fluorescence emission to produce an image and detecting the distribution of the target DNA on the transfer support. This fluorescence detecting system is advantageous in that a genetic sequence or the like can be easily detected without using a radioactive substance.

Similarly, there is known a chemiluminescence detecting system comprising the steps of fixing a substance derived from a living organism such as a protein or a nucleic acid sequence on a support, selectively labeling the substance derived from a living organism with a labeling substance which generates chemiluminescence emission when it contacts a chemiluminescent substrate, contacting the substance derived from a living organism and selectively labeled with the labeling substance and the chemiluminescent substrate, photoelectrically detecting the chemiluminescence emission in the wavelength of visible light generated by the contact of the chemiluminescent substrate and the labeling substance to produce digital image signals, effecting image processing thereon, and reproducing a chemiluminescent image on a display means such as a CRT or a recording material such as a photographic film, thereby obtaining information relating to the high molecular substance such as genetic information

Further, a micro-array analyzing system has been recently developed, which comprises the steps of using a spotting device to drop at different positions on the surface of a carrier such as a slide glass plate, a membrane filter or the like specific binding substances which can specifically bind with a substance derived from a living organism such as a cell, virus, hormone, tumor marker, enzyme, antibody, antigen, abzyme, other protein, a nuclear acid, cDNA, DNA, RNA or the like and whose sequence, base length, composition and the like are known, thereby forming a number of independent spots, specifically binding the specific binding substances using a hybridization method or the like with a substance derived from a living organism such as a cell, virus, hormone, tumor marker, enzyme, antibody, antigen, abzyme, other protein, a nuclear acid, cDNA, DNA or mRNA by extraction, isolation or the like and optionally further subjected to chemical processing, chemical modification or the like and which is labeled with a labeling substance such as a fluorescent substance, dye or the like, thereby forming a micro-array, irradiating the micro-array with a stimulating ray, photoelectrically detecting light such as fluorescence emission released from a labeling substance such as a fluorescent substance, dye or the like, and analyzing the substance derived from a living organism. This micro-array analyzing system is advantageous in that a substance derived from a living organism can be analyzed in a short time period by forming a number of spots of specific binding substances at different positions of the surface of a carrier such as a slide glass plate at a high density and hybridizing them with a substance derived from a living organism and labeled with a labeling substance.

In addition, a macro-array analyzing system using a radioactive labeling substance as a labeling substance has been further developed, which comprises the steps of using a spotting device to drop at different positions on the surface of a carrier such as a membrane filter or the like specific binding substances which can specifically bind with a substance derived from a living organism such as a cell, virus, hormone, tumor marker, enzyme, antibody, antigen, abzyme, other protein, a nuclear acid, cDNA, DNA, RNA or the like and whose sequence, base length, composition and the like are known, thereby forming a number of independent spots, specifically binding the specific binding substance using a hybridization method or the like with a substance derived from a living organism such as a cell, virus, hormone, tumor marker, enzyme, antibody, antigen, abzyme, other protein, a nuclear acid, cDNA, DNA or mRNA by extraction, isolation or the like and optionally further subjected to chemical processing, chemical modification or the like and which is labeled with a radioactive labeling substance, thereby forming a macro-array, superposing the macro-array and a stimulable phosphor sheet formed with a stimulable phosphor layer, exposing the stimulable phosphor layer to the radioactive labeling substance, irradiating the stimulable phosphor layer with a stimulating ray to excite the stimulable phosphor, photoelectrically detecting the stimulated emission released from the stimulable phosphor to produce biochemical analysis data, and analyzing the substance derived from a living organism.

However, in the macro-array analyzing system using a radioactive labeling substance as a labeling substance, when the stimulable phosphor layer is exposed to a radioactive labeling substance, since the radiation energy of the radioactive labeling substance contained in spot-like regions formed on the surface of a carrier such as a membrane filter is very large, electron beams (β rays) released from the radioactive labeling substance contained in the individual spot-like regions are scattered in the carrier such as a membrane filter, thereby impinging on regions of the stimulable phosphor layer that should be exposed only to the radioactive labeling substance contained in neighboring spot-like regions, or electron beams released from the radioactive labeling substance adhering to the surface of the carrier such as a membrane filter between neighboring spot-like regions impinge on the stimulable phosphor layer, to generate noise in biochemical analysis data produced by photoelectrically detecting stimulated emission, thus making data of neighboring spot-like regions hard to separate and lowering resolution, and to lower the accuracy of biochemical analysis when a substance derived from a living organism is analyzed by quantifying the radiation amount of each spot. The degradation of the resolution and accuracy of biochemical analysis is particularly pronounced when spots are formed close to each other at a high density.

In order to solve these problems by preventing noise caused by the scattering of electron beams released from radioactive labeling substance contained in neighboring spot-like regions, it is inevitably required to increase the distance between neighboring spot-like regions and this makes the density of the spot-like regions lower and the test efficiency lower.

Furthermore, in the field of biochemical analysis, it is often required to analyze a substance derived from a living organism by forming at different positions on the surface of a carrier such as a membrane filter or the like a plurality of spot-like regions containing specific binding substances which can specifically bind with a substance derived from a living organism such as a cell, virus, hormone, tumor marker, enzyme, antibody, antigen, abzyme, other protein, a nuclear acid, cDNA, DNA, RNA or the like and whose sequence, base length, composition and the like are known, specifically binding, using a hybridization method or the like, the specific binding substances contained in the plurality of spot-like regions with a substance derived from a living organism labeled with a labeling substance which generates chemiluminescence emission when it contacts a chemiluminescent substrate, thereby selectively labeling the plurality of spot-like regions, causing the plurality of spot-like regions to come into contact with a chemiluminescent substrate, exposing the stimulable phosphor layer of a stimulable phosphor sheet to chemiluminescence emission in the wavelength of visible light generated by the contact of the chemiluminescent substance and the labeling substance, thereby storing the energy of chemiluminescence emission in the stimulable phosphor layer, irradiating the stimulable phosphor layer with a stimulating ray, and photoelectrically detecting stimulated emission released from the stimulable phosphor layer, thereby effecting biochemical analysis. In this case, chemiluminescence emission released from any particular spot-like region is scattered in the carrier such as a membrane filter, thereby impinging on regions of the stimulable phosphor layer that should be exposed only to the chemiluminescence emission released from neighboring spot-like regions to generate noise in biochemical analysis data produced by photoelectrically detecting stimulated emission, thus making data of neighboring spot-like regions hard to separate and lowering resolution, and to lower the quantitative characteristics of biochemical analysis data.

### SUMMARY OF THE INVENTION

It is therefore an object of the present invention to provide a biochemical analysis kit and a method for exposing a stimulable phosphor sheet, which can prevent noise caused by the scattering of electron beams (β rays) released from a radioactive labeling substance selectively contained in a plurality of spot-like regions of a biochemical analysis unit from being generated in biochemical analysis data and produce biochemical analysis data having an excellent quantitative characteristic by reading radiation data with high resolution even in the case of forming a plurality of spot-like regions containing specific binding substances, which can specifically bind with a substance derived from a living organism and whose sequence, base length, composition and the like are known, in the biochemical analysis unit at a high density, selectively labeling the plurality of spot-like regions of the biochemical analysis unit with a radioactive labeling substance to record radiation data therein, facing the thus prepared biochemical analysis unit toward a stimulable phosphor layer of a stimulable phosphor sheet to expose the stimulable phosphor layer to a radioactive labeling substance, irradiating the thus exposed stimulable phosphor layer with a stimulating ray, and photoelectrically detecting stimulated emission released from the stimulable phosphor layer to produce biochemical analysis data, and can also prevent noise caused by the scattering of chemiluminescence emission selectively released from a plurality of spot-like regions of a biochemical analysis unit from being generated in biochemical analysis data and produce biochemical analysis data having an excellent quantitative characteristic by reading radiation data with high resolution even in the case of forming a plurality of spot-like regions containing specific binding substances, which can specifically bind with a substance derived from a living organism and whose sequence, base length, composition and the like are known, in the biochemical analysis unit at a high density, selectively labeling the plurality of spot-like regions of the biochemical analysis unit with a labeling substance which generates chemiluminescence emission when it contacts a chemiluminescent substrate to record chemiluminescence data therein, bringing the plurality of spot-like regions of the biochemical analysis unit into contact with a chemiluminescent substrate, thereby causing the plurality of spot-like regions of the biochemical analysis unit to release chemiluminescence emission, facing the biochemical analysis unit releasing chemiluminescence emission toward a stimulable phosphor layer of a stimulable phosphor sheet to expose the stimulable phosphor layer to chemiluminescence emission, irradiating the thus exposed stimulable phosphor layer with a stimulating ray, and photoelectrically detecting stimulated emission released from the stimulable phosphor layer to produce biochemical analysis data.

The above other objects of the present invention can be accomplished by a biochemical analysis kit comprising a biochemical analysis unit including a substrate formed with a plurality of absorptive regions to be spaced apart from each other and a stimulable phosphor sheet including a support formed with a plurality of stimulable phosphor layer regions to be spaced apart from each other in substantially the same pattern as that of the plurality of absorptive regions formed in the substrate of the biochemical analysis unit.

According to one application of the present invention, since the plurality of stimulable phosphor layer regions are formed to be spaced apart from each other in the support of the stimulable phosphor sheet in substantially the same pattern as that of the plurality of absorptive regions formed in the substrate of the biochemical analysis unit, in the case of spotting a solution containing specific binding substances whose sequence, base length, composition and the like are known onto the plurality of absorptive regions of the biochemical analysis unit, thereby absorbing the specific binding substances in the plurality of absorptive regions, specifically binding the specific binding substances absorbed in the plurality of absorptive regions with a substance derived from a living organism and labeled with a radioactive labeling substance by means of hybridization or the like, thereby selective labeling the plurality of absorptive regions of the biochemical analysis unit with the radioactive labeling substance and recording radiation data therein, superposing a stimulable phosphor sheet on the thus prepared biochemical analysis unit, and exposing the plurality of stimulable phosphor layer regions formed in the support of the stimulable phosphor sheet to the radioactive labeling substance selectively contained in the plurality of absorptive regions of the biochemical analysis unit, electron beams (β rays) released from the radioactive labeling substance contained in the individual absorptive regions of the biochemical analysis unit can be effectively prevented from entering stimulable phosphor layer regions other than the stimulable phosphor layer region to be exposed to electron beams (β rays) released from the radioactive labeling substance contained in the absorptive region. Therefore, it is possible to produce biochemical analysis data having an excellent quantitative characteristic with high resolution by scanning the plurality of thus exposed stimulable phosphor layer regions with a stimulating ray and photoelectrically detecting stimulated emission released from the plurality of stimulable phosphor layer regions.

According to another application of the present invention, since the plurality of stimulable phosphor layer regions are formed to be spaced apart from each other in the support of the stimulable phosphor sheet in substantially the same pattern as that of the plurality of absorptive regions formed in the substrate of the biochemical analysis unit, in the case of spotting a solution containing specific binding substances whose sequence, base length, composition and the like are known onto the plurality of absorptive regions of the biochemical analysis unit, thereby absorbing the specific binding substances in the plurality of absorptive regions, specifically binding the specific binding substances absorbed in the plurality of absorptive regions with a substance derived from a living organism and labeled with a labeling substance which generates chemiluminescence emission when it contacts a chemiluminescent substrate by means of hybridization or the like, thereby selective labeling the plurality of absorptive regions of the biochemical analysis unit with the labeling substance which generates chemiluminescence emission when it contacts a chemiluminescent substrate and recording chemiluminescence data therein, bringing the thus prepared biochemical analysis unit into contact with a chemiluminescent substrate, thereby causing the plurality of absorptive regions of the biochemical analysis unit to release chemiluminescence emission, superposing a stimulable phosphor sheet on the biochemical analysis unit releasing chemiluminescence emission, and exposing the plurality of stimulable phosphor layer regions formed in the support of the stimulable phosphor sheet to the chemiluminescence emission selectively released from the plurality of absorptive regions of the biochemical analysis unit, chemiluminescence emission released from the individual absorptive regions of the biochemical analysis unit can be effectively prevented from entering stimulable phosphor layer regions other than the stimulable phosphor layer region to be exposed to chemiluminescence emission released from the absorptive region. Therefore, it is possible to produce biochemical analysis data having an excellent quantitative characteristic with high resolution by scanning the plurality of thus exposed stimulable phosphor layer regions with a stimulating ray and photoelectrically detecting stimulated emission released from the plurality of stimulable phosphor layer regions.

In a preferred aspect of the present invention, the substrate of the biochemical analysis unit is formed with the plurality of absorptive regions in a regular pattern.

In a preferred aspect of the present invention, the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit.

According to this preferred aspect of the present invention, since the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit, the biochemical analysis unit and the stimulable phosphor sheet can be positioned in a desired manner by forming at least two erect positioning pins on the substrate of an exposure device at positions corresponding to the at least two positioning through-holes formed in the substrate of the biochemical analysis unit and the at least two positioning through-holes formed in the support of the stimulable phosphor sheet, inserting the at least two positioning pins into the at least two positioning through-holes formed in the substrate of the biochemical analysis unit and the at least two positioning through-holes formed in the support of the stimulable phosphor sheet and superposing the biochemical analysis unit and the stimulable phosphor sheet and, therefore, the plurality of stimulable phosphor layer regions formed in the support of the stimulable phosphor sheet can be exposed to a radioactive labeling substance selectively contained in the plurality of absorptive regions of the biochemical analysis unit in a desired manner.

Further, according to this preferred aspect of the present invention, since the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit, the biochemical analysis unit and the stimulable phosphor sheet can be positioned in a desired manner by forming at least two erect positioning pins on the substrate of an exposure device at positions corresponding to the at least two positioning through-holes formed in the substrate of the biochemical analysis unit and the at least two positioning through-holes formed in the support of the stimulable phosphor sheet, inserting the at least two positioning pins into the at least two positioning through-holes formed in the substrate of the biochemical analysis unit and the at least two positioning through-holes formed in the support of the stimulable phosphor sheet and superposing the biochemical analysis unit and the stimulable phosphor sheet and, therefore, the plurality of stimulable phosphor layer regions formed in the support of the stimulable phosphor sheet can be exposed to chemiluminescence emission released from the plurality of absorptive regions of the biochemical analysis unit in a desired manner.

Furthermore, according to this preferred aspect of the present invention, since the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit, the biochemical analysis unit and the stimulable phosphor sheet can be positioned in a desired manner by providing a light emitting means and a light receiving means in an exposure device, superposing the biochemical analysis unit and the stimulable phosphor sheet, setting them in the exposure device and detecting light emitted from the light emitting means and transmitted through the at least two positioning through-holes formed in the substrate of the biochemical analysis unit and the at least two positioning through-holes formed in the support of the stimulable phosphor sheet and, therefore, the plurality of stimulable phosphor layer regions formed in the support of the stimulable phosphor sheet can be exposed to a radioactive labeling substance selectively contained in the plurality of absorptive regions of the biochemical analysis unit in a desired manner.

Moreover, according to this preferred aspect of the present invention, since the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit, the biochemical analysis unit and the stimulable phosphor sheet can be positioned in a desired manner by providing a light emitting means and a light receiving means in an exposure device, superposing the biochemical analysis unit and the stimulable phosphor sheet, setting them in the exposure device and detecting light emitted from the light emitting means and transmitted through the at least two positioning through-holes formed in the substrate of the biochemical analysis unit and the at least two positioning through-holes formed in the support of the stimulable phosphor sheet and, therefore, the plurality of stimulable phosphor layer regions formed in the support of the stimulable phosphor sheet can be exposed to chemiluminescence emission released from the plurality of absorptive regions of the biochemical analysis unit in a desired manner.

Further, according to this preferred aspect of the present invention, since the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit, the biochemical analysis unit and the stimulable phosphor sheet can be positioned in a desired manner by setting one of the biochemical analysis unit and the stimulable phosphor sheet on the substrate of an exposure device, producing an image of the at least two positioning through-holes formed in the one of the biochemical analysis unit and the stimulable phosphor sheet using an imaging device, storing data corresponding to the image in a memory, superposing the other one of the biochemical analysis unit and the stimulable phosphor sheet on the surface of the one of the biochemical analysis unit and the stimulable phosphor sheet, producing an image of the at least two positioning through-holes formed in the other one of the biochemical analysis unit and the stimulable phosphor sheet using an imaging device, comparing the thus produced image with the image of the at least two positioning through-holes formed in the one of the biochemical analysis unit and the stimulable phosphor sheet based on the data stored in the memory, and moving the other one of the biochemical analysis unit and the stimulable phosphor sheet so that the at least two positioning through-holes formed in the substrate of the biochemical analysis unit and the at least two positioning through-holes formed in the support of the stimulable phosphor sheet coincide with each other in images and, therefore, the plurality of stimulable phosphor layer regions formed in the support of the stimulable phosphor sheet can be exposed to a radioactive labeling substance selectively contained in the plurality of absorptive regions of the biochemical analysis unit in a desired manner.

Furthermore, according to this preferred aspect of the present invention, since the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit, the biochemical analysis unit and the stimulable phosphor sheet can be positioned in a desired manner by setting one of the biochemical analysis unit and the stimulable phosphor sheet on the substrate of an exposure device, producing an image of the at least two positioning through-holes formed in the one of the biochemical analysis unit and the stimulable phosphor sheet using an imaging device, storing data corresponding to the image in a memory, superposing the other one of the biochemical analysis unit and the stimulable phosphor sheet on the surface of the one of the biochemical analysis unit and the stimulable phosphor sheet, producing an image of the at least two positioning through-holes formed in the other one of the biochemical analysis unit and the stimulable phosphor sheet using an imaging device, comparing the thus produced image with the image of the at least two positioning through-holes formed in the one of the biochemical analysis unit and the stimulable phosphor sheet based on the data stored in the memory, and moving the other one of the biochemical analysis unit and the stimulable phosphor sheet so that the at least two positioning through-holes formed in the substrate of the biochemical analysis unit and the at least two positioning through-holes formed in the support of the stimulable phosphor sheet coincide with each other in images and, therefore, the plurality of stimulable phosphor layer regions formed in the support of the stimulable phosphor sheet can be exposed to chemiluminescence emission released from the plurality of absorptive regions of the biochemical analysis unit in a desired manner.

In a preferred aspect of the present invention, the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit.

According to this preferred aspect of the present invention, since the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit, the biochemical analysis unit and the stimulable phosphor sheet can be positioned in a desired manner by forming at least two erect positioning pins on the substrate of an exposure device at positions corresponding to the at least two positioning notches formed in the substrate of the biochemical analysis unit and the at least two positioning notches formed in the support of the stimulable phosphor sheet, inserting the at least two positioning pins into the at least two positioning notches formed in the substrate of the biochemical analysis unit and the at least two positioning notches formed in the support of the stimulable phosphor sheet and superposing the biochemical analysis unit and the stimulable phosphor sheet and, therefore, the plurality of stimulable phosphor layer regions formed in the support of the stimulable phosphor sheet can be exposed to a radioactive labeling substance selectively contained in the plurality of absorptive regions of the biochemical analysis unit in a desired manner.

Further, according to this preferred aspect of the present invention, since the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit, the biochemical analysis unit and the stimulable phosphor sheet can be positioned in a desired manner by forming at least two erect positioning pins on the substrate of an exposure device at positions corresponding to the at least two positioning notches formed in the substrate of the biochemical analysis unit and the at least two positioning notches formed in the support of the stimulable phosphor sheet, inserting the at least two positioning pins into the at least two positioning notches formed in the substrate of the biochemical analysis unit and the at least two positioning notches formed in the support of the stimulable phosphor sheet and superposing the biochemical analysis unit and the stimulable phosphor sheet and, therefore, the plurality of stimulable phosphor layer regions formed in the support of the stimulable phosphor sheet can be exposed to chemiluminescence emission released from the plurality of absorptive regions of the biochemical analysis unit in a desired manner.

Furthermore, according to this preferred aspect of the present invention, since the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit, the biochemical analysis unit and the stimulable phosphor sheet can be positioned in a desired manner by providing a light emitting means and a light receiving means in an exposure device, superposing the biochemical analysis unit and the stimulable phosphor sheet, setting them in the exposure device and detecting light emitted from the light emitting means and transmitted through the at least two positioning notches formed in the substrate of the biochemical analysis unit and the at least two positioning notches formed in the support of the stimulable phosphor sheet and, therefore, the plurality of stimulable phosphor layer regions formed in the support of the stimulable phosphor sheet can be exposed to a radioactive labeling substance selectively contained in the plurality of absorptive regions of the biochemical analysis unit in a desired manner.

Moreover, according to this preferred aspect of the present invention, since the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit, the biochemical analysis unit and the stimulable phosphor sheet can be positioned in a desired manner by providing a light emitting means and a light receiving means in an exposure device, superposing the biochemical analysis unit and the stimulable phosphor sheet, setting them in the exposure device and detecting light emitted from the light emitting means and transmitted through the at least two positioning notches formed in the substrate of the biochemical analysis unit and the at least two positioning notches formed in the support of the stimulable phosphor sheet and, therefore, the plurality of stimulable phosphor layer regions formed in the support of the stimulable phosphor sheet can be exposed to chemiluminescence emission released from the plurality of absorptive regions of the biochemical analysis unit in a desired manner.

Further, according to this preferred aspect of the present invention, since the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit, the biochemical analysis unit and the stimulable phosphor sheet can be positioned in a desired manner by setting one of the biochemical analysis unit and the stimulable phosphor sheet on the substrate of an exposure device, producing an image of the at least two positioning notches formed in the one of the biochemical analysis unit and the stimulable phosphor sheet using an imaging device, storing data corresponding to the image in a memory, superposing the other one of the biochemical analysis unit and the stimulable phosphor sheet on the surface of the one of the biochemical analysis unit and the stimulable phosphor sheet, producing an image of the at least two positioning notches formed in the other one of the biochemical analysis unit and the stimulable phosphor sheet using an imaging device, comparing the thus produced image with the image of the at least two positioning notches formed in the one of the biochemical analysis unit and the stimulable phosphor sheet based on the data stored in the memory, and moving the other one of the biochemical analysis unit and the stimulable phosphor sheet so that the at least two positioning notches formed in the substrate of the biochemical analysis unit and the at least two positioning notches formed in the support of the stimulable phosphor sheet coincide with each other in images and, therefore, the plurality of stimulable phosphor layer regions formed in the support of the stimulable phosphor sheet can be exposed to a radioactive labeling substance selectively contained in the plurality of absorptive regions of the biochemical analysis unit in a desired manner.

Furthermore, according to this preferred aspect of the present invention, since the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit, the biochemical analysis unit and the stimulable phosphor sheet can be positioned in a desired manner by setting one of the biochemical analysis unit and the stimulable phosphor sheet on the substrate of an exposure device, producing an image of the at least two positioning notches formed in the one of the biochemical analysis unit and the stimulable phosphor sheet using an imaging device, storing data corresponding to the image in a memory, superposing the other one of the biochemical analysis unit and the stimulable phosphor sheet on the surface of the one of the biochemical analysis unit and the stimulable phosphor sheet, producing an image of the at least two positioning notches formed in the other one of the biochemical analysis unit and the stimulable phosphor sheet using an imaging device, comparing the thus produced image with the image of the at least two positioning notches formed in the one of the biochemical analysis unit and the stimulable phosphor sheet based on the data stored in the memory, and moving the other one of the biochemical analysis unit and the stimulable phosphor sheet so that the at least two positioning notches formed in the substrate of the biochemical analysis unit and the at least two positioning notches formed in the support of the stimulable phosphor sheet coincide with each other in images and, therefore, the plurality of stimulable phosphor layer regions formed in the support of the stimulable phosphor sheet can be exposed to chemiluminescence emission released from the plurality of absorptive regions of the biochemical analysis unit in a desired manner.

In a preferred aspect of the present invention, at least one positioning convex portion is formed on the surface of the substrate of the biochemical analysis unit and at least one positioning recess having a complementary shape to that of the at least one positioning convex portion is formed on the surface of the support of the stimulable phosphor sheet at a position corresponding to that of the at least one positioning convex portion.

According to this preferred aspect of the present invention, since at least one positioning convex portion is formed on the surface of the substrate of the biochemical analysis unit and at least one positioning recess having a complementary shape to that of the at least one positioning convex portion is formed on the surface of the support of the stimulable phosphor sheet at a position corresponding to that of the at least one positioning convex portion, the biochemical analysis unit and the stimulable phosphor sheet can be positioned in a desired manner by fitting the at least one positioning convex portion formed on the surface of the substrate of the biochemical analysis unit into the at least one positioning recess formed on the surface of the support of the stimulable phosphor sheet and, therefore, the plurality of stimulable phosphor layer regions formed in the support of the stimulable phosphor sheet can be exposed to a radioactive labeling substance selectively contained in the plurality of absorptive regions of the biochemical analysis unit in a desired manner.

Furthermore, according to this preferred aspect of the present invention, since at least one positioning convex portion is formed on the surface of the substrate of the biochemical analysis unit and at least one positioning recess having a complementary shape to that of the at least one positioning convex portion is formed on the surface of the support of the stimulable phosphor sheet at a position corresponding to that of the at least one positioning convex portion, the biochemical analysis unit and the stimulable phosphor sheet can be positioned in a desired manner by fitting the at least one positioning convex portion formed on the surface of the substrate of the biochemical analysis unit into the at least one positioning recess formed on the surface of the support of the stimulable phosphor sheet and, therefore, the plurality of stimulable phosphor layer regions formed in the support of the stimulable phosphor sheet can be exposed to chemiluminescence emission released from the plurality of absorptive regions of the biochemical analysis unit in a desired manner.

In a preferred aspect of the present invention, at least one positioning recess is formed on the surface of the substrate of the biochemical analysis unit and at least one positioning convex portion having a complementary shape to that of the at least one positioning recess is formed on the surface of the support of the stimulable phosphor sheet at a position corresponding to that of the at least one positioning recess.

According to this preferred aspect of the present invention, since at least one positioning recess is formed on the surface of the substrate of the biochemical analysis unit and at least one positioning convex portion having a complementary shape to that of the at least one positioning recess is formed on the surface of the support of the stimulable phosphor sheet at a position corresponding to that of the at least one positioning recess, the biochemical analysis unit and the stimulable phosphor sheet can be positioned in a desired manner by fitting the at least one positioning convex portion formed on the surface of the support of the stimulable phosphor sheet into the at least one positioning recess formed on the surface of the substrate of the biochemical analysis unit and, therefore, the plurality of stimulable phosphor layer regions formed in the support of the stimulable phosphor sheet can be exposed to a radioactive labeling substance selectively contained in the plurality of absorptive regions of the biochemical analysis unit in a desired manner.

Further, according to this preferred aspect of the present invention, since at least one positioning recess is formed on the surface of the substrate of the biochemical analysis unit and at least one positioning convex portion having a complementary shape to that of the at least one positioning recess is formed on the surface of the support of the stimulable phosphor sheet at a position corresponding to that of the at least one positioning recess, the biochemical analysis unit and the stimulable phosphor sheet can be positioned in a desired manner by fitting the at least one positioning convex portion formed on the surface of the support of the stimulable phosphor sheet into the at least one positioning recess formed on the surface of the substrate of the biochemical analysis unit and, therefore, the plurality of stimulable phosphor layer regions formed in the support of the stimulable phosphor sheet can be exposed to chemiluminescence emission released from the plurality of absorptive regions of the biochemical analysis unit in a desired manner.

In a preferred aspect of the present invention, the plurality of absorptive regions are formed in the substrate of the biochemical analysis unit in a predetermined positional relationship to a first reference edge of the substrate of the biochemical analysis unit and a second reference edge of the substrate of the biochemical analysis unit perpendicular to the first reference edge and the plurality of stimulable phosphor layer regions are formed in the support of the stimulable phosphor sheet in a predetermined positional relationship to a first reference edge of the support of the stimulable phosphor sheet corresponding to the first reference edge of the substrate of the biochemical analysis unit and a second reference edge of the support of the stimulable phosphor sheet corresponding to the substrate of the biochemical analysis unit.

According to this preferred aspect of the present invention, since the plurality of absorptive regions are formed in the substrate of the biochemical analysis unit in a predetermined positional relationship to a first reference edge of the substrate of the biochemical analysis unit and a second reference edge of the substrate of the biochemical analysis unit perpendicular to the first reference edge and the plurality of stimulable phosphor layer regions are formed in the support of the stimulable phosphor sheet in a predetermined positional relationship to a first reference edge of the support of the stimulable phosphor sheet corresponding to the first reference edge of the substrate of the biochemical analysis unit and a second reference edge of the support of the stimulable phosphor sheet corresponding to the substrate of the biochemical analysis unit, the biochemical analysis unit and the stimulable phosphor sheet can be positioned in a desired manner by superposing the biochemical analysis unit and the stimulable phosphor sheet so that the first reference edge of the substrate of the biochemical analysis unit and the first reference edge of the support of the stimulable phosphor sheet align with one of two erect reference pins formed on the support of an exposure device and that the second reference edge of the substrate of the biochemical analysis unit and the second reference edge of the support of the stimulable phosphor sheet align with the other one of the two erect reference pins formed on the support of the exposure device and, therefore, the plurality of stimulable phosphor layer regions formed in the support of the stimulable phosphor sheet can be exposed to a radioactive labeling substance selectively contained in the plurality of absorptive regions of the biochemical analysis unit in a desired manner.

Further, according to this preferred aspect of the present invention, since the plurality of absorptive regions are formed in the substrate of the biochemical analysis unit in a predetermined positional relationship to a first reference edge of the substrate of the biochemical analysis unit and a second reference edge of the substrate of the biochemical analysis unit perpendicular to the first reference edge and the plurality of stimulable phosphor layer regions are formed in the support of the stimulable phosphor sheet in a predetermined positional relationship to a first reference edge of the support of the stimulable phosphor sheet corresponding to the first reference edge of the substrate of the biochemical analysis unit and a second reference edge of the support of the stimulable phosphor sheet corresponding to the substrate of the biochemical analysis unit, the biochemical analysis unit and the stimulable phosphor sheet can be positioned in a desired manner by superposing the biochemical analysis unit and the stimulable phosphor sheet so that the first reference edge of the substrate of the biochemical analysis unit and the first reference edge of the support of the stimulable phosphor sheet align with one of two erect reference pins formed on the support of an exposure device and that the second reference edge of the substrate of the biochemical analysis unit and the second reference edge of the support of the stimulable phosphor sheet align with the other one of the two erect reference pins formed on the support of the exposure device and, therefore, the plurality of stimulable phosphor layer regions formed in the support of the stimulable phosphor sheet can be exposed to chemiluminescence emission released from the plurality of absorptive regions of the biochemical analysis unit in a desired manner.

In a preferred aspect of the present invention, the plurality of absorptive regions are formed by charging an absorptive material in a plurality of holes formed in the substrate of the biochemical analysis unit.

In a further preferred aspect of the present invention, the plurality of absorptive regions are formed by charging an absorptive material in a plurality of through-holes formed in the substrate of the biochemical analysis unit.

In a further preferred aspect of the present invention, the plurality of absorptive regions are formed by embedding an absorptive material in a plurality of through-holes formed in the substrate of the biochemical analysis unit.

In a further preferred aspect of the present invention, the plurality of absorptive regions are formed by pressing an absorptive membrane containing an absorptive material into a plurality of through-holes formed in the substrate of the biochemical analysis unit.

According to this preferred aspect of the present invention, since the plurality of absorptive regions can be formed only by pressing an absorptive membrane containing an absorptive material into a plurality of through-holes formed in the substrate of the biochemical analysis unit, it is possible to extremely easily produce a biochemical analysis unit formed with a plurality of absorptive regions spaced apart from each other.

In another preferred aspect of the present invention, the plurality of absorptive regions are formed by charging an absorptive material in a plurality of recesses formed in the substrate of the biochemical analysis unit.

In a further preferred aspect of the present invention, the plurality of absorptive regions are formed by embedding an absorptive material in a plurality of recesses formed in the substrate of the biochemical analysis unit.

In a preferred aspect of the present invention, the plurality of stimulable phosphor layer regions are formed by charging stimulable phosphor in a plurality of holes formed in the support of the stimulable phosphor sheet.

In a further preferred aspect of the present invention, the plurality of stimulable phosphor layer regions are formed by charging stimulable phosphor in a plurality of through-holes formed in the support of the stimulable phosphor sheet.

In a further preferred aspect of the present invention, the plurality of stimulable phosphor layer regions are formed by pressing a stimulable phosphor membrane containing stimulable phosphor and a binder into a plurality of through-holes formed in the support of the stimulable phosphor sheet.

According to this preferred aspect of the present invention, since the plurality of stimulable phosphor layer regions can be formed only by pressing a stimulable phosphor membrane containing stimulable phosphor and a binder into a plurality of through-holes formed in the support of the stimulable phosphor sheet, it is possible to extremely easily produce a stimulable phosphor sheet formed with a plurality of stimulable phosphor layer regions spaced apart from each other.

In a further preferred aspect of the present invention, the plurality of stimulable phosphor layer regions are formed by embedding stimulable phosphor in a plurality of through-holes formed in the support of the stimulable phosphor sheet.

In another preferred aspect of the present invention, the plurality of stimulable phosphor layer regions are formed by charging stimulable phosphor in a plurality of recesses formed in the support of the stimulable phosphor sheet.

In a further preferred aspect of the present invention, the plurality of stimulable phosphor layer regions are formed by embedding stimulable phosphor in a plurality of recesses formed in the support of the stimulable phosphor sheet.

In a further preferred aspect of the present invention, the at least two positioning through-holes of the biochemical analysis unit are formed by through-holes in which no absorptive material is charged among the plurality of through-holes formed in the substrate of the biochemical analysis unit and the at least two positioning through-holes of the stimulable phosphor sheet are formed by through-holes formed at positions corresponding to the at least two positioning through-holes of the biochemical analysis unit and in which no stimulable phosphor is charged among the plurality of through-holes formed in the support of the stimulable phosphor sheet.

According to this preferred aspect of the present invention, since the at least two positioning through-holes of the biochemical analysis unit are formed by through-holes in which no absorptive material is charged among the plurality of through-holes formed in the substrate of the biochemical analysis unit and the at least two positioning through-holes are formed by through-holes formed at positions corresponding to the at least two positioning through-holes of the biochemical analysis unit and in which no stimulable phosphor is charged among the plurality of through-holes formed in the support of the stimulable phosphor sheet, the biochemical analysis unit and the stimulable phosphor sheet can be positioned in a desired manner by forming at least two erect positioning pins on the substrate of an exposure device at positions corresponding to the at least two positioning through-holes formed in the substrate of the biochemical analysis unit and the at least two positioning through-holes formed in the support of the stimulable phosphor sheet, inserting the at least two positioning pins into the at least two positioning through-holes formed in the substrate of the biochemical analysis unit and the at least two positioning through-holes formed in the support of the stimulable phosphor sheet and superposing the biochemical analysis unit and the stimulable phosphor sheet and, therefore, the plurality of stimulable phosphor layer regions formed in the support of the stimulable phosphor sheet can be exposed to a radioactive labeling substance selectively contained in the plurality of absorptive regions of the biochemical analysis unit in a desired manner.

Further, according to this preferred aspect of the present invention, since the at least two positioning through-holes of the biochemical analysis unit are formed by through-holes in which no absorptive material is charged among the plurality of through-holes formed in the substrate of the biochemical analysis unit and the at least two positioning through-holes are formed by through-holes formed at positions corresponding to the at least two positioning through-holes of the biochemical analysis unit and in which no stimulable phosphor is charged among the plurality of through-holes formed in the support of the stimulable phosphor sheet, the biochemical analysis unit and the stimulable phosphor sheet can be positioned in a desired manner by forming at least two erect positioning pins on the substrate of an exposure device at positions corresponding to the at least two positioning through-holes formed in the substrate of the biochemical analysis unit and the at least two positioning through-holes formed in the support of the stimulable phosphor sheet, inserting the at least two positioning pins into the at least two positioning through-holes formed in the substrate of the biochemical analysis unit and the at least two positioning through-holes formed in the support of the stimulable phosphor sheet and superposing the biochemical analysis unit and the stimulable phosphor sheet and, therefore, the plurality of stimulable phosphor layer regions formed in the support of the stimulable phosphor sheet can be exposed to chemiluminescence emission released from the plurality of absorptive regions of the biochemical analysis unit in a desired manner.

Furthermore, according to this preferred aspect of the present invention, since the at least two positioning through-holes of the biochemical analysis unit are formed by through-holes in which no absorptive material is charged among the plurality of through-holes formed in the substrate of the biochemical analysis unit and the at least two positioning through-holes are formed by through-holes formed at positions corresponding to the at least two positioning through-holes of the biochemical analysis unit and in which no stimulable phosphor is charged among the plurality of through-holes formed in the support of the stimulable phosphor sheet, the biochemical analysis unit and the stimulable phosphor sheet can be positioned in a desired manner by providing a light emitting means and a light receiving means in an exposure device, superposing the biochemical analysis unit and the stimulable phosphor sheet, setting them in the exposure device and detecting light emitted from the light emitting means and transmitted through the at least two positioning through-holes formed in the substrate of the biochemical analysis unit and the at least two positioning through-holes formed in the support of the stimulable phosphor sheet and, therefore, the plurality of stimulable phosphor layer regions formed in the support of the stimulable phosphor sheet can be exposed to a radioactive labeling substance selectively contained in the plurality of absorptive regions of the biochemical analysis unit in a desired manner.

Moreover, according to this preferred aspect of the present invention, since the at least two positioning through-holes of the biochemical analysis unit are formed by through-holes in which no absorptive material is charged among the plurality of through-holes formed in the substrate of the biochemical analysis unit and the at least two positioning through-holes are formed by through-holes formed at positions corresponding to the at least two positioning through-holes of the biochemical analysis unit and in which no stimulable phosphor is charged among the plurality of through-holes formed in the support of the stimulable phosphor sheet, the biochemical analysis unit and the stimulable phosphor sheet can be positioned in a desired manner by providing a light emitting means and a light receiving means in an exposure device, superposing the biochemical analysis unit and the stimulable phosphor sheet, setting them in the exposure device and detecting light emitted from the light emitting means and transmitted through the at least two positioning through-holes formed in the substrate of the biochemical analysis unit and the at least two positioning through-holes formed in the support of the stimulable phosphor sheet and, therefore, the plurality of stimulable phosphor layer regions formed in the support of the stimulable phosphor sheet can be exposed to chemiluminescence emission released from the plurality of absorptive regions of the biochemical analysis unit in a desired manner.

Further, according to this preferred aspect of the present invention, since the at least two positioning through-holes of the biochemical analysis unit are formed by through-holes in which no absorptive material is charged among the plurality of through-holes formed in the substrate of the biochemical analysis unit and the at least two positioning through-holes are formed by through-holes formed at positions corresponding to the at least two positioning through-holes of the biochemical analysis unit and in which no stimulable phosphor is charged among the plurality of through-holes formed in the support of the stimulable phosphor sheet, the biochemical analysis unit and the stimulable phosphor sheet can be positioned in a desired manner by setting one of the biochemical analysis unit and the stimulable phosphor sheet on the substrate of an exposure device, producing an image of the at least two positioning through-holes formed in the one of the biochemical analysis unit and the stimulable phosphor sheet using an imaging device, storing data corresponding to the image in a memory, superposing the other one of the biochemical analysis unit and the stimulable phosphor sheet on the surface of the one of the biochemical analysis unit and the stimulable phosphor sheet, producing an image of the at least two positioning through-holes formed in the other one of the biochemical analysis unit and the stimulable phosphor sheet using an imaging device, comparing the thus produced image with the image of the at least two positioning through-holes formed in the one of the biochemical analysis unit and the stimulable phosphor sheet based on the data stored in the memory, and moving the other one of the biochemical analysis unit and the stimulable phosphor sheet so that the at least two positioning through-holes formed in the substrate of the biochemical analysis unit and the at least two positioning through-holes formed in the support of the stimulable phosphor sheet coincide with each other in images and, therefore, the plurality of stimulable phosphor layer regions formed in the support of the stimulable phosphor sheet can be exposed to a radioactive labeling substance selectively contained in the plurality of absorptive regions of the biochemical analysis unit in a desired manner.

Furthermore, according to this preferred aspect of the present invention, since the at least two positioning through-holes of the biochemical analysis unit are formed by through-holes in which no absorptive material is charged among the plurality of through-holes formed in the substrate of the biochemical analysis unit and the at least two positioning through-holes are formed by through-holes formed at positions corresponding to the at least two positioning through-holes of the biochemical analysis unit and in which no stimulable phosphor is charged among the plurality of through-holes formed in the support of the stimulable phosphor sheet, the biochemical analysis unit and the stimulable phosphor sheet can be positioned in a desired manner by setting one of the biochemical analysis unit and the stimulable phosphor sheet on the substrate of an exposure device, producing an image of the at least two positioning through-holes formed in the one of the biochemical analysis unit and the stimulable phosphor sheet using an imaging device, storing data corresponding to the image in a memory, superposing the other one of the biochemical analysis unit and the stimulable phosphor sheet on the surface of the one of the biochemical analysis unit and the stimulable phosphor sheet, producing an image of the at least two positioning through-holes formed in the other one of the biochemical analysis unit and the stimulable phosphor sheet using an imaging device, comparing the thus produced image with the image of the at least two positioning through-holes formed in the one of the biochemical analysis unit and the stimulable phosphor sheet based on the data stored in the memory, and moving the other one of the biochemical analysis unit and the stimulable phosphor sheet so that the at least two positioning through-holes formed in the substrate of the biochemical analysis unit and the at least two positioning through-holes formed in the support of the stimulable phosphor sheet coincide with each other in images and, therefore, the plurality of stimulable phosphor layer regions formed in the support of the stimulable phosphor sheet can be exposed to chemiluminescence emission released from the plurality of absorptive regions of the biochemical analysis unit in a desired manner.

The above and other objects of the present invention can be also accomplished by a method for exposing a stimulable phosphor sheet comprising the steps of superposing a biochemical analysis unit including a substrate capable of attenuating radiation energy and formed with a plurality of absorptive regions spaced apart from each other and selectively labeled with a radioactive labeling substance and the stimulable phosphor sheet including a support formed with a plurality of stimulable phosphor layer regions spaced apart from each other so that each of the plurality of absorptive regions of the biochemical analysis unit faces the corresponding stimulable layer region of the stimulable phosphor sheet and exposing the plurality of stimulable phosphor layer regions of the stimulable phosphor sheet to the radioactive labeling substance selectively contained in the plurality of absorptive regions of the biochemical analysis unit.

According to the present invention, since the plurality of stimulable phosphor layer regions are formed to be spaced apart from each other in the support of the stimulable phosphor sheet in substantially the same pattern as that of the plurality of absorptive regions formed in the substrate of the biochemical analysis unit, in the case of spotting a solution containing specific binding substances whose sequence, base length, composition and the like are known onto the plurality of absorptive regions of the biochemical analysis unit, thereby absorbing the specific binding substances in the plurality of absorptive regions, specifically binding the specific binding substances absorbed in the plurality of absorptive regions with a substance derived from a living organism and labeled with a radioactive labeling substance by means of hybridization or the like, thereby selective labeling the plurality of absorptive regions of the biochemical analysis unit with the radioactive labeling substance and recording radiation data therein, superposing a stimulable phosphor sheet on the thus prepared biochemical analysis unit, and exposing the plurality of stimulable phosphor layer regions formed in the support of the stimulable phosphor sheet to the radioactive labeling substance selectively contained in the plurality of absorptive regions of the biochemical analysis unit, electron beams (β rays) released from the radioactive labeling substance contained in the individual absorptive regions of the biochemical analysis unit can be effectively prevented from entering stimulable phosphor layer regions other than the stimulable phosphor layer region to be exposed to electron beams (β rays) released from the radioactive labeling substance contained in the absorptive region. Therefore, it is possible to produce biochemical analysis data having an excellent quantitative characteristic with high resolution by scanning the plurality of thus exposed stimulable phosphor layer regions with a stimulating ray and photoelectrically detecting stimulated emission released from the plurality of stimulable phosphor layer regions.

The above and other objects of the present invention can be also accomplished by a method for exposing a stimulable phosphor sheet comprising the steps of bringing a biochemical analysis unit including a substrate capable of attenuating light energy and formed with a plurality of absorptive regions spaced apart from each other and selectively labeled with a labeling substance which generates chemiluminescence emission when it contacts a chemiluminescent substrate into contact with a chemiluminescent substrate, thereby causing the plurality of absorptive regions to selectively release chemiluminescence emission, superposing the biochemical analysis unit releasing the chemiluminescence emission and the stimulable phosphor sheet including a support formed with a plurality of stimulable phosphor layer regions spaced apart from each other so that each of the plurality of absorptive regions of the biochemical analysis unit faces the corresponding stimulable layer region of the stimulable phosphor sheet and exposing the plurality of stimulable phosphor layer regions of the stimulable phosphor sheet to the chemiluminescence emission selectively released from the plurality of absorptive regions of the biochemical analysis unit.

According to the present invention, since the plurality of stimulable phosphor layer regions are formed to be spaced apart from each other in the support of the stimulable phosphor sheet in substantially the same pattern as that of the plurality of absorptive regions formed in the substrate of the biochemical analysis unit, in the case of spotting a solution containing specific binding substances whose sequence, base length, composition and the like are known onto the plurality of absorptive regions of the biochemical analysis unit, thereby absorbing the specific binding substances in the plurality of absorptive regions, specifically binding the specific binding substances absorbed in the plurality of absorptive regions with a substance derived from a living organism and labeled with a labeling substance which generates chemiluminescence emission when it contacts a chemiluminescent substrate by means of hybridization or the like, thereby selective labeling the plurality of absorptive regions of the biochemical analysis unit with the labeling substance which generates chemiluminescence emission when it contacts a chemiluminescent substrate and recording chemiluminescence data therein, bringing the thus prepared biochemical analysis unit into contact with a chemiluminescent substrate, thereby causing the plurality of absorptive regions of the biochemical analysis unit to release chemiluminescence emission, superposing a stimulable phosphor sheet on the biochemical analysis unit releasing chemiluminescence emission, and exposing the plurality of stimulable phosphor layer regions formed in the support of the stimulable phosphor sheet to the chemiluminescence emission selectively released from the plurality of absorptive regions of the biochemical analysis unit, chemiluminescence emission released from the individual absorptive regions of the biochemical analysis unit can be effectively prevented from entering stimulable phosphor layer regions other than the stimulable phosphor layer region to be exposed to chemiluminescence emission released from the absorptive region. Therefore, it is possible to produce biochemical analysis data having an excellent quantitative characteristic with high resolution by scanning the plurality of thus exposed stimulable phosphor layer regions with a stimulating ray and photoelectrically detecting stimulated emission released from the plurality of stimulable phosphor layer regions.

In the present invention, the case where a plurality of absorptive regions are selectively labeled with a labeling substance which generates chemiluminescence emission when it contacts a chemiluminescent substrate as termed herein includes the case where a plurality of absorptive regions are selectively labeled with a labeling substance which generates chemiluminescence emission when it contacts a chemiluminescent substrate by selectively binding a substance derived from a living organism and labeled with a labeling substance which generates chemiluminescence emission when it contacts a chemiluminescent substrate with specific binding substances contained in the plurality of absorptive regions and the case where a plurality of absorptive regions are selectively labeled with a labeling substance which generates chemiluminescence emission when it contacts a chemiluminescent substrate by selectively binding a substance derived from a living organism and labeled with a hapten with specific binding substances contained in the plurality of absorptive, and binding an antibody for the hapten labeled with an enzyme which generates chemiluminescence emission when it contacts a chemiluminescent substrate with the hapten by an antigen-antibody reaction.

In the present invention, illustrative examples of the combination of hapten and antibody include digoxigenin and anti-digoxigenin antibody, theophylline and anti-theophylline antibody, fluorosein and anti-fluorosein antibody, and the like. Further, the combination of biotin and avidin, antigen and antibody may be utilized instead of the combination of hapten and antibody.

In a preferred aspect of the present invention, the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit and the method for exposing the stimulable phosphor sheet comprises the steps of superposing the biochemical analysis unit and the stimulable phosphor sheet so that at least two erect positioning pins formed on a substrate of an exposure device at positions corresponding to the at least two positioning through-holes formed in the substrate of the biochemical analysis unit and the at least two positioning through-holes formed in the support of the stimulable phosphor sheet are inserted into the at least two positioning through-holes formed in the substrate of the biochemical analysis unit and the at least two positioning through-holes formed in the support of the stimulable phosphor sheet, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

In a preferred aspect of the present invention, the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit and the method for exposing the stimulable phosphor sheet comprises the steps of superposing the biochemical analysis unit releasing chemiluminescence emission and the stimulable phosphor sheet so that at least two erect positioning pins formed on a substrate of an exposure device at positions corresponding to the at least two positioning through-holes formed in the substrate of the biochemical analysis unit and the at least two positioning through-holes formed in the support of the stimulable phosphor sheet are inserted into the at least two positioning through-holes formed in the substrate of the biochemical analysis unit and the at least two positioning through-holes formed in the support of the stimulable phosphor sheet, thereby positioning the biochemical analysis unit and the stimulable phosphor sheet, and exposing the plurality of stimulable phosphor layer regions of the stimulable phosphor sheet to chemiluminescence emission selectively released from the plurality of absorptive regions of the biochemical analysis unit.

In a preferred aspect of the present invention, the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit and the method for exposing the stimulable phosphor sheet comprises the steps of superposing the biochemical analysis unit and the stimulable phosphor sheet, positioning the biochemical analysis unit and the stimulable phosphor sheet by detecting light emitted from a light emitting means and transmitted through the at least two positioning through-holes formed in the substrate of the biochemical analysis unit and the at least two positioning through-holes formed in the support of the stimulable phosphor sheet with a light receiving means, and exposing the plurality of stimulable phosphor layer regions of the stimulable phosphor sheet to a radioactive labeling substrate selectively contained in the plurality of absorptive regions of the biochemical analysis unit.

In a preferred aspect of the present invention, the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit and the method for exposing the stimulable phosphor sheet comprises the steps of superposing the biochemical analysis unit releasing chemiluminescence emission and the stimulable phosphor sheet, positioning the biochemical analysis unit and the stimulable phosphor sheet by detecting light emitted from a light emitting means and transmitted through the at least two positioning through-holes formed in the substrate of the biochemical analysis unit and the at least two positioning through-holes formed in the support of the stimulable phosphor sheet with a light receiving means, and exposing the plurality of stimulable phosphor layer regions of the stimulable phosphor sheet to chemiluminescence emission selectively released from the plurality of absorptive regions of the biochemical analysis unit.

In a preferred aspect of the present invention, the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit and the method for exposing the stimulable phosphor sheet comprises the steps of setting one of the biochemical analysis unit and the stimulable phosphor sheet on a substrate of an exposure device, producing an image of the at least two positioning through-holes formed in the one of the biochemical analysis unit and the stimulable phosphor sheet using an imaging device, storing data corresponding to the image in a memory, superposing the other one of the biochemical analysis unit and the stimulable phosphor sheet on the surface of the one of the biochemical analysis unit and the stimulable phosphor sheet, producing an image of the at least two positioning through-holes formed in the other one of the biochemical analysis unit and the stimulable phosphor sheet using the imaging device, comparing the thus produced image with the image of the at least two positioning through-holes formed in the one of the biochemical analysis unit and the stimulable phosphor sheet based on the data stored in the memory, moving the other one of the biochemical analysis unit and the stimulable phosphor sheet so that the at least two positioning through-holes formed in the substrate of the biochemical analysis unit and the at least two positioning through-holes formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

In a preferred aspect of the present invention, the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit and the method for exposing the stimulable phosphor sheet comprises the steps of setting one of the biochemical analysis unit releasing chemiluminescence emission and the stimulable phosphor sheet on a substrate of an exposure device, producing an image of the at least two positioning through-holes formed in the one of the biochemical analysis unit and the stimulable phosphor sheet using an imaging device, storing data corresponding to the image in a memory, superposing the other one of the biochemical analysis unit releasing chemiluminescence emission and the stimulable phosphor sheet on the surface of the one of the biochemical analysis unit and the stimulable phosphor sheet, producing an image of the at least two positioning through-holes formed in the other one of the biochemical analysis unit and the stimulable phosphor sheet using the imaging device, comparing the thus produced image with the image of the at least two positioning through-holes formed in the one of the biochemical analysis unit and the stimulable phosphor sheet based on the data stored in the memory, moving the other one of the biochemical analysis unit and the stimulable phosphor sheet so that the at least two positioning through-holes formed in the substrate of the biochemical analysis unit and the at least two positioning through-holes formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to chemiluminescence emission selectively released from the plurality of absorptive regions of the biochemical analysis unit.

In a preferred aspect of the present invention, the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit and the method for exposing the stimulable phosphor sheet comprises the steps of superposing the biochemical analysis unit and the stimulable phosphor sheet so that at least two erect positioning pins formed on a substrate of an exposure device at positions corresponding to the at least two positioning notches formed in the substrate of the biochemical analysis unit and the at least two positioning notches formed in the support of the stimulable phosphor sheet are inserted into the at least two positioning notches formed in the substrate of the biochemical analysis unit and the at least two positioning notches formed in the support of the stimulable phosphor sheet, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

In a preferred aspect of the present invention, the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit and the method for exposing the stimulable phosphor sheet comprises the steps of superposing the biochemical analysis unit releasing chemiluminescence emission and the stimulable phosphor sheet so that at least two erect positioning pins formed on a substrate of an exposure device at positions corresponding to the at least two positioning notches formed in the substrate of the biochemical analysis unit and the at least two positioning notches formed in the support of the stimulable phosphor sheet are inserted into the at least two positioning notches formed in the substrate of the biochemical analysis unit and the at least two positioning notches formed in the support of the stimulable phosphor sheet, thereby positioning the biochemical analysis unit and the stimulable phosphor sheet, and exposing the plurality of stimulable phosphor layer regions of the stimulable phosphor sheet to chemiluminescence emission selectively released from the plurality of absorptive regions of the biochemical analysis unit.

In a preferred aspect of the present invention, the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit and the method for exposing the stimulable phosphor sheet comprises the steps of superposing the biochemical analysis unit and the stimulable phosphor sheet, positioning the biochemical analysis unit and the stimulable phosphor sheet by detecting light emitted from a light emitting means and transmitted through the at least two positioning notches formed in the substrate of the biochemical analysis unit and the at least two positioning notches formed in the support of the stimulable phosphor sheet with a light receiving means, and exposing the plurality of stimulable phosphor layer regions of the stimulable phosphor sheet to a radioactive labeling substrate selectively contained in the plurality of absorptive regions of the biochemical analysis unit.

In a preferred aspect of the present invention, the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit and the method for exposing the stimulable phosphor sheet comprises the steps of superposing the biochemical analysis unit releasing chemiluminescence emission and the stimulable phosphor sheet, positioning the biochemical analysis unit and the stimulable phosphor sheet by detecting light emitted from a light emitting means and transmitted through the at least two positioning notches formed in the substrate of the biochemical analysis unit and the at least two positioning notches formed in the support of the stimulable phosphor sheet with a light receiving means, and exposing the plurality of stimulable phosphor layer regions of the stimulable phosphor sheet to chemiluminescence emission selectively released from the plurality of absorptive regions of the biochemical analysis unit.

In a preferred aspect of the present invention, the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit and the method for exposing the stimulable phosphor sheet comprises the steps of setting one of the biochemical analysis unit and the stimulable phosphor sheet on a substrate of an exposure device, producing an image of the at least two positioning notches formed in the one of the biochemical analysis unit and the stimulable phosphor sheet using an imaging device, storing data corresponding to the image in a memory, superposing the other one of the biochemical analysis unit and the stimulable phosphor sheet on the surface of the one of the biochemical analysis unit and the stimulable phosphor sheet, producing an image of the at least two positioning notches formed in the other one of the biochemical analysis unit and the stimulable phosphor sheet using the imaging device, comparing the thus produced image with the image of the at least two positioning notches formed in the one of the biochemical analysis unit and the stimulable phosphor sheet based on the data stored in the memory, moving the other one of the biochemical analysis unit and the stimulable phosphor sheet so that the at least two positioning notches formed in the substrate of the biochemical analysis unit and the at least two positioning notches formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

In a preferred aspect of the present invention, the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit and the method for exposing the stimulable phosphor sheet comprises the steps of setting one of the biochemical analysis unit releasing chemiluminescence emission and the stimulable phosphor sheet on a substrate of an exposure device, producing an image of the at least two positioning notches formed in the one of the biochemical analysis unit and the stimulable phosphor sheet using an imaging device, storing data corresponding to the image in a memory, superposing the other one of the biochemical analysis unit releasing chemiluminescence emission and the stimulable phosphor sheet on the surface of the one of the biochemical analysis unit and the stimulable phosphor sheet, producing an image of the at least two positioning notches formed in the other one of the biochemical analysis unit and the stimulable phosphor sheet using the imaging device, comparing the thus produced image with the image of the at least two positioning notches formed in the one of the biochemical analysis unit and the stimulable phosphor sheet based on the data stored in the memory, moving the other one of the biochemical analysis unit and the stimulable phosphor sheet so that the at least two positioning notches formed in the substrate of the biochemical analysis unit and the at least two positioning notches formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to chemiluminescence emission selectively released from the plurality of absorptive regions of the biochemical analysis unit.

In a preferred aspect of the present invention, at least one positioning convex portion on the surface of the substrate of the biochemical analysis unit and at least one positioning recess having a complementary shape to that of the at least one positioning convex portion at a position corresponding to that of the at least one positioning convex portion on the surface of the support of the stimulable phosphor sheet and the method for exposing the stimulable phosphor sheet comprises the steps of fitting the at least one positioning convex portion formed on the surface of the substrate of the biochemical analysis unit into the at least one positioning recess formed on the surface of the support of the stimulable phosphor sheet, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

In a preferred aspect of the present invention, at least one positioning convex portion on the surface of the substrate of the biochemical analysis unit and at least one positioning recess having a complementary shape to that of the at least one positioning convex portion at a position corresponding to that of the at least one positioning convex portion on the surface of the support of the stimulable phosphor sheet and the method for exposing the stimulable phosphor sheet comprises the steps of fitting the at least one positioning convex portion formed on the surface of the substrate of the biochemical analysis unit into the at least one positioning recess formed on the surface of the support of the stimulable phosphor sheet, thereby positioning the biochemical analysis unit releasing chemiluminescence emission and the stimulable phosphor sheet, and exposing the plurality of stimulable phosphor layer regions of the stimulable phosphor sheet to chemiluminescence emission substrate selectively released from the plurality of absorptive regions of the biochemical analysis unit.

In a preferred aspect of the present invention, at least one positioning recess on the surface of the substrate of the biochemical analysis unit and at least one positioning convex portion having a complementary shape to that of the at least one positioning recess at a position corresponding to that of the at least one positioning recess on the surface of the support of the stimulable phosphor sheet and the method for exposing the stimulable phosphor sheet comprises the steps of fitting the at least one positioning convex portion formed on the surface of the support of the stimulable phosphor sheet into the at least one positioning recess formed on the surface of the substrate of the biochemical analysis unit, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

In a preferred aspect of the present invention, at least one positioning recess on the surface of the substrate of the biochemical analysis unit and at least one positioning convex portion having a complementary shape to that of the at least one positioning recess at a position corresponding to that of the at least one positioning recess on the surface of the support of the stimulable phosphor sheet and the method for exposing the stimulable phosphor sheet comprises the steps of fitting the at least one positioning convex portion formed on the surface of the support of the stimulable phosphor sheet into the at least one positioning recess formed on the surface of the substrate of the biochemical analysis unit, thereby positioning the biochemical analysis unit releasing chemiluminescence emission and the stimulable phosphor sheet, and exposing the plurality of stimulable phosphor layer regions of the stimulable phosphor sheet to chemiluminescence emission selectively released from the plurality of absorptive regions of the biochemical analysis unit.

In a preferred aspect of the present invention, the plurality of absorptive regions are formed in the substrate of the biochemical analysis unit in a predetermined positional relationship to a first reference edge of the substrate of the biochemical analysis unit and a second reference edge of the substrate of the biochemical analysis unit perpendicular to the first reference edge and the plurality of stimulable phosphor layer regions are formed in the support of the stimulable phosphor sheet in a predetermined positional relationship to a first reference edge of the support of the stimulable phosphor sheet corresponding to the first reference edge of the substrate of the biochemical analysis unit and a second reference edge of the support of the stimulable phosphor sheet corresponding to the substrate of the biochemical analysis unit and the method for exposing the stimulable phosphor sheet comprises the steps of superposing the biochemical analysis unit and the stimulable phosphor sheet so that the first reference edge of the substrate of the biochemical analysis unit and the first reference edge of the support of the stimulable phosphor sheet align with one of two erect reference pins formed on the support of an exposure device and that the second reference edge of the substrate of the biochemical analysis unit and the second reference edge of the support of the stimulable phosphor sheet align with the other one of the two erect reference pins formed on the support of an exposure device, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

In a preferred aspect of the present invention, the plurality of absorptive regions are formed in the substrate of the biochemical analysis unit in a predetermined positional relationship to a first reference edge of the substrate of the biochemical analysis unit and a second reference edge of the substrate of the biochemical analysis unit perpendicular to the first reference edge and the plurality of stimulable phosphor layer regions are formed in the support of the stimulable phosphor sheet in a predetermined positional relationship to a first reference edge of the support of the stimulable phosphor sheet corresponding to the first reference edge of the substrate of the biochemical analysis unit and a second reference edge of the support of the stimulable phosphor sheet corresponding to the substrate of the biochemical analysis unit and the method for exposing the stimulable phosphor sheet comprises the steps of superposing the biochemical analysis unit releasing chemiluminescence emission and the stimulable phosphor sheet so that the first reference edge of the substrate of the biochemical analysis unit and the first reference edge of the support of the stimulable phosphor sheet align with one of two erect reference pins formed on the support of an exposure device and that the second reference edge of the substrate of the biochemical analysis unit and the second reference edge of the support of the stimulable phosphor sheet align with the other one of the two erect reference pins formed on the support of an exposure device, thereby positioning the biochemical analysis unit and the stimulable phosphor sheet, and exposing the plurality of stimulable phosphor layer regions of the stimulable phosphor sheet to chemiluminescence emission selectively released from the plurality of absorptive regions of the biochemical analysis unit.

In a preferred aspect of the present invention, the plurality of absorptive regions of the biochemical analysis unit are formed by charging an absorptive material in a number of through-holes formed in the substrate and the plurality of stimulable phosphor layer regions of the stimulable phosphor sheet are formed by charging stimulable phosphor in a number of through-holes formed in the support so that the at least two positioning through-holes of the biochemical analysis unit are formed by through-holes in which no absorptive material is charged among the plurality of through-holes formed in the substrate of the biochemical analysis unit and that the at least two positioning through-holes of the stimulable phosphor sheet are formed by through-holes formed at positions corresponding to the at least two positioning through-holes of the biochemical analysis unit and in which no stimulable phosphor is charged among the plurality of through-holes formed in the support of the stimulable phosphor sheet, and the method for exposing the stimulable phosphor sheet comprises the steps of inserting at least two erect positioning pins formed on a substrate of an exposure device at positions corresponding to the at least two positioning through-holes formed in the substrate of the biochemical analysis unit and the at least two positioning through-holes formed in the support of the stimulable phosphor sheet into the at least two positioning through-holes formed in the substrate of the biochemical analysis unit and the at least two positioning through-holes formed in the support of the stimulable phosphor sheet, superposing the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

In a preferred aspect of the present invention, the plurality of absorptive regions of the biochemical analysis unit are formed by charging an absorptive material in a number of through-holes formed in the substrate and the plurality of stimulable phosphor layer regions of the stimulable phosphor sheet are formed by charging stimulable phosphor in a number of through-holes formed in the support so that the at least two positioning through-holes of the biochemical analysis unit are formed by through-holes in which no absorptive material is charged among the plurality of through-holes formed in the substrate of the biochemical analysis unit and that the at least two positioning through-holes of the stimulable phosphor sheet are formed by through-holes formed at positions corresponding to the at least two positioning through-holes of the biochemical analysis unit and in which no stimulable phosphor is charged among the plurality of through-holes formed in the support of the stimulable phosphor sheet, and the method for exposing the stimulable phosphor sheet comprises the steps of inserting at least two erect positioning pins formed on a substrate of an exposure device at positions corresponding to the at least two positioning through-holes formed in the substrate of the biochemical analysis unit and the at least two positioning through-holes formed in the support of the stimulable phosphor sheet into the at least two positioning through-holes formed in the substrate of the biochemical analysis unit and the at least two positioning through-holes formed in the support of the stimulable phosphor sheet, superposing the biochemical analysis unit releasing chemiluminescence emission and the stimulable phosphor sheet, and exposing the plurality of stimulable phosphor layer regions of the stimulable phosphor sheet to chemiluminescence emission selectively released from the plurality of absorptive regions of the biochemical analysis unit.

In a preferred aspect of the present invention, the plurality of absorptive regions of the biochemical analysis unit are formed by charging an absorptive material in a number of through-holes formed in the substrate and the plurality of stimulable phosphor layer regions of the stimulable phosphor sheet are formed by charging stimulable phosphor in a number of through-holes formed in the support so that the at least two positioning through-holes of the biochemical analysis unit are formed by through-holes in which no absorptive material is charged among the plurality of through-holes formed in the substrate of the biochemical analysis unit and that the at least two positioning through-holes of the stimulable phosphor sheet are formed by through-holes formed at positions corresponding to the at least two positioning through-holes of the biochemical analysis unit and in which no stimulable phosphor is charged among the plurality of through-holes formed in the support of the stimulable phosphor sheet, and the method for exposing the stimulable phosphor sheet comprises the steps of superposing the biochemical analysis unit and the stimulable phosphor sheet, positioning the biochemical analysis unit and the stimulable phosphor sheet by detecting light emitted from a light emitting means and transmitted through the at least two positioning through-holes formed in the substrate of the biochemical analysis unit and the at least two positioning through-holes formed in the support of the stimulable phosphor sheet with a light receiving means, and exposing the plurality of stimulable phosphor layer regions of the stimulable phosphor sheet to a radioactive labeling substrate selectively contained in the plurality of absorptive regions of the biochemical analysis unit.

In a preferred aspect of the present invention, the plurality of absorptive regions of the biochemical analysis unit are formed by charging an absorptive material in a number of through-holes formed in the substrate and the plurality of stimulable phosphor layer regions of the stimulable phosphor sheet are formed by charging stimulable phosphor in a number of through-holes formed in the support so that the at least two positioning through-holes of the biochemical analysis unit are formed by through-holes in which no absorptive material is charged among the plurality of through-holes formed in the substrate of the biochemical analysis unit and that the at least two positioning through-holes of the stimulable phosphor sheet are formed by through-holes formed at positions corresponding to the at least two positioning through-holes of the biochemical analysis unit and in which no stimulable phosphor is charged among the plurality of through-holes formed in the support of the stimulable phosphor sheet, and the method for exposing the stimulable phosphor sheet comprises the steps of superposing the biochemical analysis unit releasing chemiluminescence emission and the stimulable phosphor sheet, positioning the biochemical analysis unit and the stimulable phosphor sheet by detecting light emitted from a light emitting means and transmitted through the at least two positioning through-holes formed in the substrate of the biochemical analysis unit and the at least two positioning through-holes formed in the support of the stimulable phosphor sheet with a light receiving means, and exposing the plurality of stimulable phosphor layer regions of the stimulable phosphor sheet to chemiluminescence emission selectively released from the plurality of absorptive regions of the biochemical analysis unit.

In a preferred aspect of the present invention, the plurality of absorptive regions of the biochemical analysis unit are formed by charging an absorptive material in a number of through-holes formed in the substrate and the plurality of stimulable phosphor layer regions of the stimulable phosphor sheet are formed by charging stimulable phosphor in a number of through-holes formed in the support so that the at least two positioning through-holes of the biochemical analysis unit are formed by through-holes in which no absorptive material is charged among the plurality of through-holes formed in the substrate of the biochemical analysis unit and that the at least two positioning through-holes of the stimulable phosphor sheet are formed by through-holes formed at positions corresponding to the at least two positioning through-holes of the biochemical analysis unit and in which no stimulable phosphor is charged among the plurality of through-holes formed in the support of the stimulable phosphor sheet, and the method for exposing the stimulable phosphor sheet comprises the steps of setting one of the biochemical analysis unit and the stimulable phosphor sheet on a substrate of an exposure device, producing an image of the at least two positioning through-holes formed in the one of the biochemical analysis unit and the stimulable phosphor sheet using an imaging device, storing data corresponding to the image in a memory, superposing the other one of the biochemical analysis unit and the stimulable phosphor sheet on the surface of the one of the biochemical analysis unit and the stimulable phosphor sheet, producing an image of the at least two positioning through-holes formed in the other one of the biochemical analysis unit and the stimulable phosphor sheet using the imaging device, comparing the thus produced image with the image of the at least two positioning through-holes formed in the one of the biochemical analysis unit and the stimulable phosphor sheet based on the data stored in the memory, moving the other one of the biochemical analysis unit and the stimulable phosphor sheet so that the at least two positioning through-holes formed in the substrate of the biochemical analysis unit and the at least two positioning through-holes formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

In a preferred aspect of the present invention, the plurality of absorptive regions of the biochemical analysis unit are formed by charging an absorptive material in a number of through-holes formed in the substrate and the plurality of stimulable phosphor layer regions of the stimulable phosphor sheet are formed by charging stimulable phosphor in a number of through-holes formed in the support so that the at least two positioning through-holes of the biochemical analysis unit are formed by through-holes in which no absorptive material is charged among the plurality of through-holes formed in the substrate of the biochemical analysis unit and that the at least two positioning through-holes of the stimulable phosphor sheet are formed by through-holes formed at positions corresponding to the at least two positioning through-holes of the biochemical analysis unit and in which no stimulable phosphor is charged among the plurality of through-holes formed in the support of the stimulable phosphor sheet, and the method for exposing the stimulable phosphor sheet comprises the steps of setting one of the biochemical analysis unit releasing chemiluminescence emission and the stimulable phosphor sheet on a substrate of an exposure device, producing an image of the at least two positioning through-holes formed in the one of the biochemical analysis unit and the stimulable phosphor sheet using an imaging device, storing data corresponding to the image in a memory, superposing the other one of the biochemical analysis unit releasing chemiluminescence emission and the stimulable phosphor sheet on the surface of the one of the biochemical analysis unit and the stimulable phosphor sheet, producing an image of the at least two positioning through-holes formed in the other one of the biochemical analysis unit and the stimulable phosphor sheet using the imaging device, comparing the thus produced image with the image of the at least two positioning through-holes formed in the one of the biochemical analysis unit and the stimulable phosphor sheet based on the data stored in the memory, moving the other one of the biochemical analysis unit and the stimulable phosphor sheet so that the at least two positioning through-holes formed in the substrate of the biochemical analysis unit and the at least two positioning through-holes formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to chemiluminescence emission selectively released from the plurality of absorptive regions of the biochemical analysis unit.

In a preferred aspect of the present invention, the method for exposing the stimulable phosphor sheet comprises the steps of setting one of the biochemical analysis unit and the stimulable phosphor sheet on a substrate of an exposure device, producing an image of the one of the biochemical analysis unit and the stimulable phosphor sheet using an imaging device, storing data corresponding to the image in a memory, superposing the other one of the biochemical analysis unit and the stimulable phosphor sheet on the surface of the one of the biochemical analysis unit and the stimulable phosphor sheet, producing an image of the other one of the biochemical analysis unit and the stimulable phosphor sheet, comparing the thus produced image with the image of the at least two positioning through-holes formed in the one of the biochemical analysis unit and the stimulable phosphor sheet based on the data stored in the memory, moving the other one of the biochemical analysis unit and the stimulable phosphor sheet so that the plurality of absorptive regions formed in the substrate of the biochemical analysis unit and the plurality of stimulable phosphor layer regions formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

In a preferred aspect of the present invention, the method for exposing the stimulable phosphor sheet comprises the steps of setting one of the biochemical analysis unit releasing chemiluminescence emission and the stimulable phosphor sheet on a substrate of an exposure device, producing an image of the one of the biochemical analysis unit and the stimulable phosphor sheet using an imaging device, storing data corresponding to the image in a memory, superposing the other one of the biochemical analysis unit releasing chemiluminescence emission and the stimulable phosphor sheet on the surface of the one of the biochemical analysis unit and the stimulable phosphor sheet, producing an image of the other one of the biochemical analysis unit and the stimulable phosphor sheet, comparing the thus produced image with the image of the at least two positioning through-holes formed in the one of the biochemical analysis unit and the stimulable phosphor sheet based on the data stored in the memory, moving the other one of the biochemical analysis unit and the stimulable phosphor sheet so that the plurality of absorptive regions formed in the substrate of the biochemical analysis unit and the plurality of stimulable phosphor layer regions formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to chemiluminescence emission selectively released from the plurality of absorptive regions of the biochemical analysis unit.

In a preferred aspect of the present invention, each of the absorptive regions is formed substantially circular in the substrate of the biochemical analysis unit.

In a preferred aspect of the present invention, each of the stimulable phosphor layer regions is formed substantially circular in the support of the stimulable phosphor sheet.

In a preferred aspect of the present invention, each of the absorptive regions is formed substantially rectangular in the substrate of the biochemical analysis unit.

In a preferred aspect of the present invention, each of the stimulable phosphor layer regions is formed substantially rectangular in the support of the stimulable phosphor sheet.

In a preferred aspect of the present invention, the substrate of the biochemical analysis unit is formed with 10 or more absorptive regions.

In a further preferred aspect of the present invention, the substrate of the biochemical analysis unit is formed with 50 or more absorptive regions.

In a further preferred aspect of the present invention, the substrate of the biochemical analysis unit is formed with 100 or more absorptive regions.

In a further preferred aspect of the present invention, the substrate of the biochemical analysis unit is formed with 500 or more absorptive regions.

In a further preferred aspect of the present invention, the substrate of the biochemical analysis unit is formed with 1,000 or more absorptive regions.

In a further preferred aspect of the present invention, the substrate of the biochemical analysis unit is formed with 5,000 or more absorptive regions.

In a further preferred aspect of the present invention, the substrate of the biochemical analysis unit is formed with 10,000 or more absorptive regions.

In a further preferred aspect of the present invention, the substrate of the biochemical analysis unit is formed with 50,000 or more absorptive regions.

In a further preferred aspect of the present invention, the substrate of the biochemical analysis unit is formed with 100,000 or more absorptive regions.

In a preferred aspect of the present invention, each of the plurality of absorptive regions formed in the substrate of the biochemical analysis unit has a size of less than 5 mm².

In a further preferred aspect of the present invention, each of the plurality of absorptive regions formed in the substrate of the biochemical analysis unit has a size of less than 1 mm².

In a further preferred aspect of the present invention, each of the plurality of absorptive regions formed in the substrate of the biochemical analysis unit has a size of less than 0.5 mm².

In a further preferred aspect of the present invention, each of the plurality of absorptive regions formed in the substrate of the biochemical analysis unit has a size of less than 0.1 mm².

In a further preferred aspect of the present invention, each of the plurality of absorptive regions formed in the substrate of the biochemical analysis unit has a size of less than 0.05 mm².

In a further preferred aspect of the present invention, each of the plurality of absorptive regions formed in the substrate of the biochemical analysis unit has a size of less than 0.01 mm².

In the present invention, the density of the absorptive regions formed in the substrate of the biochemical analysis unit is determined depending upon the material for forming the substrate, the kind of electron beam released from a radioactive substance or the like.

In a preferred aspect of the present invention, the plurality of absorptive regions are formed in the substrate of the biochemical analysis unit at a density of 10 or more per cm².

In a further preferred aspect of the present invention, the plurality of absorptive regions are formed in the substrate of the biochemical analysis unit at a density of 50 or more per cm².

In a further preferred aspect of the present invention, the plurality of absorptive regions are formed in the substrate of the biochemical analysis unit at a density of 100 or more per cm².

In a further preferred aspect of the present invention, the plurality of absorptive regions are formed in the substrate of the biochemical analysis unit at a density of 500 or more per cm².

In a further preferred aspect of the present invention, the plurality of absorptive regions are formed in the substrate of the biochemical analysis unit at a density of 1,000 or more per cm².

In a further preferred aspect of the present invention, the plurality of absorptive regions are formed in the substrate of the biochemical analysis unit at a density of 5,000 or more per cm².

In a further preferred aspect of the present invention, the plurality of absorptive regions are formed in the substrate of the biochemical analysis unit at a density of 10,000 or more per cm².

In a further preferred aspect of the present invention, the plurality of absorptive regions are formed in the substrate of the biochemical analysis unit at a density of 50,000 or more per cm².

In a further preferred aspect of the present invention, the plurality of absorptive regions are formed in the substrate of the biochemical analysis unit at a density of 100,000 or more per cm².

In a preferred aspect of the present invention, the substrate of the biochemical analysis unit has a property of attenuating radiation energy.

According to this preferred aspect of the present invention, even in the case of forming the plurality of absorptive regions in the substrate of the biochemical analysis unit at a high density, spotting a solution containing specific binding substances whose sequence, base length, composition and the like are known onto the plurality of absorptive regions of the biochemical analysis unit, thereby absorbing the specific binding substances in the plurality of absorptive regions, specifically binding the specific binding substances absorbed in the plurality of absorptive regions with a substance derived from a living organism and labeled with a radioactive labeling substance by means of hybridization or the like, thereby selective labeling the plurality of absorptive regions of the biochemical analysis unit with the radioactive labeling substance and recording radiation data therein, superposing a stimulable phosphor sheet formed with the plurality of stimulable phosphor layer regions on the thus prepared biochemical analysis unit, and exposing the plurality of stimulable phosphor layer regions formed in the support of the stimulable phosphor sheet to the radioactive labeling substance selectively contained in the plurality of absorptive regions of the biochemical analysis unit, since electron beams (β rays) released from the radioactive labeling substance contained in the individual absorptive regions can be effectively prevented from scattering in the substrate of the biochemical analysis unit, it is possible to selectively impinge electron beams (β rays) released from the radioactive labeling substance contained in a particular absorptive region onto the corresponding stimulable phosphor layer region, thereby exposing only the corresponding stimulable phosphor layer region. Therefore, it is possible to produce biochemical analysis data having an excellent quantitative characteristic with high resolution by scanning the plurality of stimulable phosphor layer regions exposed to the radioactive labeling substance with a stimulating ray and photoelectrically detecting stimulated emission released from the plurality of stimulable phosphor layer regions.

In a preferred aspect of the present invention, the substrate of the biochemical analysis unit has a property of reducing the energy of radiation to 1/5 or less when the radiation travels in the substrate by a distance equal to that between neighboring absorptive regions.

In a further preferred aspect of the present invention, the substrate of the biochemical analysis unit has a property of reducing the energy of radiation to 1/10 or less when the radiation travels in the substrate by a distance equal to that between neighboring absorptive regions.

In a further preferred aspect of the present invention, the substrate of the biochemical analysis unit has a property of reducing the energy of radiation to 1/50 or less when the radiation travels in the substrate by a distance equal to that between neighboring absorptive regions.

In a further preferred aspect of the present invention, the substrate of the biochemical analysis unit has a property of reducing the energy of radiation to 1/100 or less when the radiation travels in the substrate by a distance equal to that between neighboring absorptive regions.

In a further preferred aspect of the present invention, the substrate of the biochemical analysis unit has a property of reducing the energy of radiation to 1/500 or less when the radiation travels in the substrate by a distance equal to that between neighboring absorptive regions.

In a further preferred aspect of the present invention, the substrate of the biochemical analysis unit has a property of reducing the energy of radiation to 1/1,000 or less when the radiation travels in the substrate by a distance equal to that between neighboring absorptive regions.

In a preferred aspect of the present invention, the substrate of the biochemical analysis unit has a property of attenuating radiation energy.

According to this preferred aspect of the present invention, even in the case of forming the plurality of absorptive regions in the substrate of the biochemical analysis unit at a high density, spotting a solution containing specific binding substances whose sequence, base length, composition and the like are known onto the plurality of absorptive regions of the biochemical analysis unit, thereby absorbing the specific binding substances in the plurality of absorptive regions, specifically binding the specific binding substances absorbed in the plurality of absorptive regions with a substance derived from a living organism and labeled with a labeling substance which generates chemiluminescence emission when it contacts a chemiluminescent substrate by means of hybridization or the like, thereby selective labeling the plurality of absorptive regions of the biochemical analysis unit with the labeling substance which generates chemiluminescence emission when it contacts a chemiluminescent substrate and recording chemiluminescence data therein, bringing the thus prepared biochemical analysis unit into contact with a chemiluminescent substrate, thereby causing the plurality of absorptive regions to selectively release chemiluminescence emission, superposing a stimulable phosphor sheet formed with the plurality of stimulable phosphor layer regions on the biochemical analysis unit releasing chemiluminescence emission, and exposing the plurality of stimulable phosphor layer regions formed in the support of the stimulable phosphor sheet to the chemiluminescence emission selectively released from the plurality of absorptive regions of the biochemical analysis unit, since chemiluminescence emission released from the individual absorptive regions can be effectively prevented from scattering in the substrate of the biochemical analysis unit, it is possible to selectively impinge chemiluminescence emission released from a particular absorptive region onto the corresponding stimulable phosphor layer region, thereby exposing only the corresponding stimulable phosphor layer region. Therefore, it is possible to produce biochemical analysis data having an excellent quantitative characteristic with high resolution by scanning the plurality of stimulable phosphor layer regions exposed to the chemiluminescence emission with a stimulating ray and photoelectrically detecting stimulated emission released from the plurality of stimulable phosphor layer regions.

In a preferred aspect of the present invention, the substrate of the biochemical analysis unit has a property of reducing the energy of light to 1/5 or less when the light travels in the substrate by a distance equal to that between neighboring absorptive regions.

In a further preferred aspect of the present invention, the substrate of the biochemical analysis unit has a property of reducing the energy of light to 1/10 or less when the light travels in the substrate by a distance equal to that between neighboring absorptive regions.

In a further preferred aspect of the present invention, the substrate of the biochemical analysis unit has a property of reducing the energy of light to 1/50 or less when the light travels in the substrate by a distance equal to that between neighboring absorptive regions.

In a further preferred aspect of the present invention, the substrate of the biochemical analysis unit has a property of reducing the energy of light to 1/100 or less when the light travels in the substrate by a distance equal to that between neighboring absorptive regions.

In a further preferred aspect of the present invention, the substrate of the biochemical analysis unit has a property of reducing the energy of light to 1/500 or less when the light travels in the substrate by a distance equal to that between neighboring absorptive regions.

In a further preferred aspect of the present invention, the substrate of the biochemical analysis unit has a property of reducing the energy of light to 1/1,000 or less when the light travels in the substrate by a distance equal to that between neighboring absorptive regions.

In the present invention, the material for forming the substrate of the biochemical analysis unit is preferably capable of attenuating radiation energy and/or light energy but is not particularly limited. The material for forming the substrate of the biochemical analysis unit may be any type of inorganic compound material or organic compound material and the substrate of the biochemical analysis unit can preferably be formed of a metal material, a ceramic material or a plastic material.

Illustrative examples of inorganic compound materials preferably usable for forming the substrate of the biochemical analysis unit in the present invention include metals such as gold, silver, copper, zinc, aluminum, titanium, tantalum, chromium, iron, nickel, cobalt, lead, tin, selenium and the like; alloys such as brass, stainless steel, bronze and the like; silicon materials such as silicon, amorphous silicon, glass, quartz, silicon carbide, silicon nitride and the like; metal oxides such as aluminum oxide, magnesium oxide, zirconium oxide and the like; and inorganic salts such as tungsten carbide, calcium carbide, calcium sulfate, hydroxy apatite, gallium arsenide and the like. These may have either a monocrystal structure or a polycrystal sintered structure such as amorphous, ceramic or the like.

In the present invention, a high molecular compound can preferably be used as an organic compound material preferably usable for forming the substrate of the biochemical analysis unit. Illustrative examples of high molecular compounds preferably usable for forming the substrate of the biochemical analysis unit in the present invention include polyolefins such as polyethylene, polypropylene and the like; acrylic resins such as polymethyl methacrylate, polybutylacrylate/polymethyl methacrylate copolymer and the like; polyacrylonitrile; polyvinyl chloride; polyvinylidene chloride; polyvinylidene fluoride; polytetrafluoroethylene; polychlorotrifuluoroethylene; polycarbonate; polyesters such as polyethylene naphthalate, polyethylene terephthalate and the like; nylons such as nylon-6, nylon-6,6, nylon-4,10 and the like; polyimide; polysulfone; polyphenylene sulfide; silicon resins such as polydiphenyl siloxane and the like; phenol resins such as novolac and the like; epoxy resin; polyurethane; polystyrene, butadiene-styrene copolymer; polysaccharides such as cellulose, acetyl cellulose, nitrocellulose, starch, calcium alginate, hydroxypropyl methyl cellulose and the like; chitin; chitosan; urushi (Japanese lacquer); polyamides such as gelatin, collagen, keratin and the like; and copolymers of these high molecular materials. These may be a composite compound, and metal oxide particles, glass fiber or the like may be added thereto as occasion demands. Further, an organic compound material may be blended therewith.

Since the capability of attenuating radiation energy generally increases as specific gravity increases, the substrate of the biochemical analysis unit is preferably formed of a compound material or a composite material having specific gravity of 1.0 g/cm³ or more and more preferably formed of a compound material or a composite material having specific gravity of 1.5 g/cm³ to 23 g/cm³.

Since the capability of attenuating light energy generally increases as scattering and/or absorption of light increases, the substrate of the biochemical analysis unit preferably has absorbance of 0.3 per cm (thickness) or more and more preferably has absorbance of 1 per cm (thickness) or more. The absorbance can be determined by placing an integrating sphere immediately behind a plate-like member having a thickness of T cm, measuring an amount A of transmitted light at a wavelength of probe light or emission light used for measurement by a spectrophotometer, and calculating A/T. In the present invention, a light scattering substance or a light absorbing substance may be added to the substrate of the biochemical analysis unit in order to improve the capability of attenuating light energy. Particles of a material different from a material forming the substrate of the biochemical analysis unit may be preferably used as a light scattering substance and a pigment or dye may be preferably used as a light absorbing substance.

In the present invention, a porous material or a fiber material may be preferably used as the absorptive material for forming the absorptive regions of the biochemical analysis unit. The absorptive regions may be formed by combining a porous material and a fiber material.

In the present invention, a porous material for forming the absorptive regions of the biochemical analysis unit may be any type of an organic material or an inorganic material and may be an organic/inorganic composite material.

In the present invention, an organic porous material used for forming the absorptive regions of the biochemical analysis unit is not particularly limited but a carbon porous material such as an activated carbon or a porous material capable of forming a membrane filter is preferably used. Illustrative examples of porous materials capable of forming a membrane filter include nylons such as nylon-6, nylon-6,6, nylon-4,10; cellulose derivatives such as nitrocellulose, acetyl cellulose, butyric-acetyl cellulose; collagen; alginic acids such as alginic acid, calcium alginate, alginic acid/poly-L-lysine polyionic complex; polyolefins such as polyethylene, polypropylene; polyvinyl chloride; polyvinylidene chloride; polyfluoride such as polyvinylidene fluoride, polytetrafluoride; and copolymers or composite materials thereof.

In the present invention, an inorganic porous material used for forming the absorptive regions of the biochemical analysis unit is not particularly limited. Illustrative examples of inorganic porous materials preferably usable in the present invention include metals such as platinum, gold, iron, silver, nickel, aluminum and the like; metal oxides such as alumina, silica, titania, zeolite and the like; metal salts such as hydroxy apatite, calcium sulfate and the like; and composite materials thereof.

In the present invention, a fiber material used for forming the absorptive regions of the biochemical analysis unit is not particularly limited. Illustrative examples of fiber materials preferably usable in the present invention include nylons such as nylon-6, nylon-6,6, nylon-4,10; and cellulose derivatives such as nitrocellulose, acetyl cellulose, butyric-acetyl cellulose.

In a preferred aspect of the present invention, specific binding substances whose sequence, base length, composition and the like are known.

In a preferred aspect of the present invention, the support of the stimulable phosphor sheet is formed with 10 or more stimulable phosphor layer regions.

In a further preferred aspect of the present invention, the support of the stimulable phosphor sheet is formed with 50 or more stimulable phosphor layer regions.

In a further preferred aspect of the present invention, the support of the stimulable phosphor sheet is formed with 100 or more stimulable phosphor layer regions.

In a further preferred aspect of the present invention, the support of the stimulable phosphor sheet is formed with 500 or more stimulable phosphor layer regions.

In a further preferred aspect of the present invention, the support of the stimulable phosphor sheet is formed with 1,000 or more stimulable phosphor layer regions.

In a further preferred aspect of the present invention, the support of the stimulable phosphor sheet is formed with 5,000 or more stimulable phosphor layer regions.

In a further preferred aspect of the present invention, the support of the stimulable phosphor sheet is formed with 10,000 or more stimulable phosphor layer regions.

In a further preferred aspect of the present invention, the support of the stimulable phosphor sheet is formed with 50,000 or more stimulable phosphor layer regions.

In a further preferred aspect of the present invention, the support of the stimulable phosphor sheet is formed with 100,000 or more stimulable phosphor layer regions.

In a preferred aspect of the present invention, each of the plurality of stimulable phosphor layer regions is formed in the stimulable phosphor sheet to have a size of less than 5 mm².

In a further preferred aspect of the present invention, each of the plurality of stimulable phosphor layer regions is formed in the stimulable phosphor sheet to have a size of less than 1 mm².

In a further preferred aspect of the present invention, each of the plurality of stimulable phosphor layer regions is formed in the stimulable phosphor sheet to have a size of less than 0.5 mm².

In a further preferred aspect of the present invention, each of the plurality of stimulable phosphor layer regions is formed in the stimulable phosphor sheet to have a size of less than 0.1 mm².

In a further preferred aspect of the present invention, each of the plurality of stimulable phosphor layer regions is formed in the stimulable phosphor sheet to have a size of less than 0.05 mm².

In a further preferred aspect of the present invention, each of the plurality of stimulable phosphor layer regions is formed in the stimulable phosphor sheet to have a size of less than 0.01 mm².

In the present invention, the density of the stimulable phosphor layer regions formed in the support of the stimulable phosphor sheet can be determined based upon the material of the support, the kind of electron beam released from the radioactive labeling substance and the like.

In a preferred aspect of the present invention, the plurality of stimulable phosphor layer regions are formed in the support of the stimulable phosphor sheet at a density of 10 or more per cm².

In a further preferred aspect of the present invention, the plurality of stimulable phosphor layer regions are formed in the support of the stimulable phosphor sheet at a density of 50 or more per cm².

In a further preferred aspect of the present invention, the plurality of stimulable phosphor layer regions are formed in the support of the stimulable phosphor sheet at a density of 100 or more per cm².

In a further preferred aspect of the present invention, the plurality of stimulable phosphor layer regions are formed in the support of the stimulable phosphor sheet at a density of 500 or more per cm².

In a further preferred aspect of the present invention, the plurality of stimulable phosphor layer regions are formed in the support of the stimulable phosphor sheet at a density of 1,000 or more per cm².

In a further preferred aspect of the present invention, the plurality of stimulable phosphor layer regions are formed in the support of the stimulable phosphor sheet at a density of 5,000 or more per cm².

In a further preferred aspect of the present invention, the plurality of stimulable phosphor layer regions are formed in the support of the stimulable phosphor sheet at a density of 10,000 or more per cm².

In a further preferred aspect of the present invention, the plurality of stimulable phosphor layer regions are formed in the support of the stimulable phosphor sheet at a density of 50,000 or more per cm².

In a further preferred aspect of the present invention, the plurality of stimulable phosphor layer regions are formed in the support of the stimulable phosphor sheet at a density of 100,000 or more per cm².

In a preferred aspect of the present invention, the support of the stimulable phosphor sheet has a property of attenuating radiation energy.

According to this preferred aspect of the present invention, even in the case of forming the plurality of absorptive regions in the substrate of the biochemical analysis unit at a high density, spotting a solution containing specific binding substances whose sequence, base length, composition and the like are known onto the plurality of absorptive regions of the biochemical analysis unit, thereby absorbing the specific binding substances in the plurality of absorptive regions, specifically binding the specific binding substances absorbed in the plurality of absorptive regions with a substance derived from a living organism and labeled with a radioactive labeling substance by means of hybridization or the like, thereby selective labeling the plurality of absorptive regions of the biochemical analysis unit with the radioactive labeling substance and recording radiation data therein, superposing a stimulable phosphor sheet on the thus prepared biochemical analysis unit, and exposing the plurality of stimulable phosphor layer regions formed in the support of the stimulable phosphor sheet in substantially the same pattern as that of the plurality of absorptive regions formed in the biochemical analysis unit to the radioactive labeling substance selectively contained in the plurality of absorptive regions of the biochemical analysis unit, since the support of the stimulable phosphor sheet has a property of attenuating radiation energy, electron beams (β rays) released from the radioactive labeling substance contained in the individual absorptive regions can be effectively prevented from scattering in the support of the stimulable phosphor sheet. Therefore, since it is possible to effectively prevent electron beams (β rays) released from the radioactive labeling substance contained in a particular absorptive region from entering stimulable phosphor layer regions next to the corresponding stimulable phosphor layer region, it is possible to produce biochemical analysis data having an excellent quantitative characteristic with high resolution by scanning the plurality of thus exposed stimulable phosphor layer regions with a stimulating ray and photoelectrically detecting stimulated emission released from the plurality of stimulable phosphor layer regions.

In a preferred aspect of the present invention, the support of the stimulable phosphor sheet has a property of reducing the radiation energy to 1/5 or less when the radiation travels in the support by a distance equal to that between neighboring stimulable phosphor layer regions.

In a further preferred aspect of the present invention, the support of the stimulable phosphor sheet has a property of reducing the radiation energy to 1/10 or less when the radiation travels in the support by a distance equal to that between neighboring stimulable phosphor layer regions.

In a further preferred aspect of the present invention, the support of the stimulable phosphor sheet has a property of reducing the radiation energy to 1/50 or less when the radiation travels in the support by a distance equal to that between neighboring stimulable phosphor layer regions.

In a further preferred aspect of the present invention, the support of the stimulable phosphor sheet has a property of reducing the radiation energy to 1/100 or less when the radiation travels in the support by a distance equal to that between neighboring stimulable phosphor layer regions.

In a further preferred aspect of the present invention, the support of the stimulable phosphor sheet has a property of reducing the radiation energy to 1/500 or less when the radiation travels in the support by a distance equal to that between neighboring stimulable phosphor layer regions.

In a further preferred aspect of the present invention, the support of the stimulable phosphor sheet has a property of reducing the radiation energy to 1/1,000 or less when the radiation travels in the support by a distance equal to that between neighboring stimulable phosphor layer regions.

In a preferred aspect of the present invention, the support of the stimulable phosphor sheet has a property of attenuating light energy.

According to this preferred aspect of the present invention, even in the case of forming the plurality of absorptive regions in the substrate of the biochemical analysis unit at a high density, spotting a solution containing specific binding substances whose sequence, base length, composition and the like are known onto the plurality of absorptive regions of the biochemical analysis unit, thereby absorbing the specific binding substances in the plurality of absorptive regions, specifically binding the specific binding substances absorbed in the plurality of absorptive regions with a substance derived from a living organism and labeled with a labeling substance which generates chemiluminescence emission when it contacts a chemiluminescent substrate by means of hybridization or the like, thereby selective labeling the plurality of absorptive regions of the biochemical analysis unit with the labeling substance which generates chemiluminescence emission when it contacts a chemiluminescent substrate and recording chemiluminescence data therein, bringing the thus prepared biochemical analysis unit into contact with a chemiluminescent substrate, thereby causing the plurality of absorptive regions to selectively release chemiluminescence emission, superposing a stimulable phosphor sheet on the biochemical analysis unit releasing chemiluminescence emission, and exposing the plurality of stimulable phosphor layer regions formed in the support of the stimulable phosphor sheet in substantially the same pattern as that of the olurality of absorptive regions formed in the biochemical analysis unit to the chemiluminescence emission selectively released from the plurality of absorptive regions of the biochemical analysis unit, since the support of the stimulable phosphor sheet has a property of attenuating light energy, chemiluminescence emission released from individual absorptive regions can be effectively prevented from scattering in the support of the stimulable phosphor sheet. Therefore, since it is possible to effectively prevent chemiluminescence emission released from a particular absorptive region from entering stimulable phosphor layer regions next to the corresponding stimulable phosphor layer region, it is possible to produce biochemical analysis data having an excellent quantitative characteristic with high resolution by scanning the plurality of thus exposed stimulable phosphor layer regions with a stimulating ray and photoelectrically detecting stimulated emission released from the plurality of stimulable phosphor layer regions.

In a preferred aspect of the present invention, the support of the stimulable phosphor sheet has a property of reducing the energy of light to 1/5 or less when the light travels in the support by a distance equal to that between neighboring stimulable phosphor layer regions.

In a further preferred aspect of the present invention, the support of the stimulable phosphor sheet has a property of reducing the energy of light to 1/10 or less when the light travels in the support by a distance equal to that between neighboring stimulable phosphor layer regions.

In a further preferred aspect of the present invention, the support of the stimulable phosphor sheet has a property of reducing the energy of light to 1/50 or less when the light travels in the support by a distance equal to that between neighboring stimulable phosphor layer regions.

In a further preferred aspect of the present invention, the support of the stimulable phosphor sheet has a property of reducing the energy of light to 1/100 or less when the light travels in the support by a distance equal to that between neighboring stimulable phosphor layer regions.

In a further preferred aspect of the present invention, the support of the stimulable phosphor sheet has a property of reducing the energy of light to 1/500 or less when the light travels in the support by a distance equal to that between neighboring stimulable phosphor layer regions.

In a further preferred aspect of the present invention, the support of the stimulable phosphor sheet has a property of reducing the energy of light to 1/1,000 or less when the light travels in the support by a distance equal to that between neighboring stimulable phosphor layer regions.

In the present invention, the material for forming the support of the stimulable phosphor sheet is preferably capable of attenuating radiation energy and/or light energy but is not particularly limited. The material for forming the support of the stimulable phosphor sheet may be any type of inorganic compound material or organic compound material and the support of the stimulable phosphor sheet can preferably be formed of a metal material, a ceramic material or a plastic material.

Illustrative examples of inorganic compound materials preferably usable for forming the support of the stimulable phosphor sheet in the present invention include metals such as gold, silver, copper, zinc, aluminum, titanium, tantalum, chromium, iron, nickel, cobalt, lead, tin, selenium and the like; alloys such as brass, stainless steel, bronze and the like; silicon materials such as silicon, amorphous silicon, glass, quartz, silicon carbide, silicon nitride and the like; metal oxides such as aluminum oxide, magnesium oxide, zirconium oxide and the like; and inorganic salts such as tungsten carbide, calcium carbide, calcium sulfate, hydroxy apatite, gallium arsenide and the like. These may have either a monocrystal structure or a polycrystal sintered structure such as amorphous, ceramic or the like.

In the present invention, a high molecular compound can preferably be used as an organic compound material preferably usable for forming the support of the stimulable phosphor sheet. Illustrative examples of high molecular compounds preferably usable for forming the support of the stimulable phosphor sheet in the present invention include polyolefins such as polyethylene, polypropylene and the like; acrylic resins such as polymethyl methacrylate, polybutylacrylate/polymethyl methacrylate copolymer and the like; polyacrylonitrile; polyvinyl chloride; polyvinylidene chloride; polyvinylidene fluoride; polytetrafluoroethylene; polychlorotrifuluoroethylene; polycarbonate; polyesters such as polyethylene naphthalate, polyethylene terephthalate and the like; nylons such as nylon-6, nylon-6,6, nylon-4,10 and the like; polyimide; polysulfone; polyphenylene sulfide; silicon resins such as polydiphenyl siloxane and the like; phenol resins such as novolac and the like; epoxy resin; polyurethane; polystyrene, butadiene-styrene copolymer; polysaccharides such as cellulose, acetyl cellulose, nitrocellulose, starch, calcium alginate, hydroxypropyl methyl cellulose and the like; chitin; chitosan; urushi (Japanese lacquer); polyamides such as gelatin, collagen, keratin and the like; and copolymers of these high molecular materials. These may be a composite compound, and metal oxide particles, glass fiber or the like may be added thereto as occasion demands. Further, an organic compound material may be blended therewith.

Since the capability of attenuating radiation energy generally increases as specific gravity increases, the support of the stimulable phosphor sheet is preferably formed of a compound material or a composite material having specific gravity of 1.0 g/cm³ or more and more preferably formed of a compound material or a composite material having specific gravity of 1.5 g/cm³ to 23 g/cm³.

Since the capability of attenuating light energy generally increases as scattering and/or absorption of light increases, the support of the stimulable phosphor sheet preferably has absorbance of 0.3 per cm (thickness) or more and more preferably has absorbance of 1 per cm (thickness) or more. The absorbance can be determined by placing an integrating sphere immediately behind a plate-like member having a thickness of T cm, measuring an amount A of transmitted light at a wavelength of probe light or emission light used for measurement by a spectrophotometer, and calculating A/T. In the present invention, a light scattering substance or a light absorbing substance may be added to the support of the stimulable phosphor sheet in order to improve the capability of attenuating light energy. Particles of a material different from a material forming the support of the stimulable phosphor sheet may be preferably used as a light scattering substance and a pigment or dye may be preferably used as a light absorbing substance.

In the present invention, the stimulable phosphor usable for storing radiation energy may be of any type insofar as it can store radiation energy or electron beam energy and can be stimulated by an electromagnetic wave to release the radiation energy or the electron beam energy stored therein in the form of light. More specifically, preferably employed stimulable phosphors include alkaline earth metal fluorohalide phosphors (Ba₁₋ₓ, M²⁺ₓ)FX:yA (where M²⁺ is at least one alkaline earth metal selected from the group consisting of Mg, Ca, Sr, Zn and Cd; X is at least one element selected from the group consisting of Cl, Br and I, A is at least one element selected from the group consisting of Eu, Tb, Ce, Tm, Dy, Pr, Ho, Nd, Yb and Er; x is equal to or greater than 0 and equal to or less than 0.6 and y is equal to or greater than 0 and equal to or less than 0.2) disclosed in U.S. Patent No. 4,239,968, alkaline earth metal fluorohalide phosphors SrFX:Z (where X is at least one halogen selected from the group consisting of Cl, Br and I; Z is at least one of Eu and Ce) disclosed in Japanese Patent Application Laid Open No. 2-276997, europium activated complex halide phosphors BaFXxNaX':aEu²⁺ (where each of X or X' is at least one halogen selected from the group consisting of Cl, Br and I; x is greater than 0 and equal to or less than 2; and y is greater than 0 and equal to or less than 0.2) disclosed in Japanese Patent Application Laid Open No. 59-56479, cerium activated trivalent metal oxyhalide phosphors MOX:xCe (where M is at least one trivalent metal selected from the group consisting of Pr, Nd, Pm, Sm, Eu, Tb, Dy, Ho, Er, Tm, Yb and Bi; X is at least one halogen selected from the group consisting of Br and I; and x is greater than 0 and less than 0.1) disclosed in Japanese Patent Application laid Open No. 58-69281, cerium activated rare earth oxyhalide phosphors LnOX:xCe (where Ln is at least one rare earth element selected from the group consisting of Y, La, Gd and Lu; X is at least one halogen selected from the group consisting of Cl, Br and I; and x is greater than 0 and equal to or less than 0.1) disclosed in U.S. Patent No. 4,539,137, and europium activated complex halide phosphors M^{II}FXaM^{I}X'bM'^{II}X"₂cM^{III}X'''₃xA:yEu²⁺ (where M^{II} is at least one alkaline earth metal selected from the group consisting of Ba, Sr and Ca; M^{I} is at least one alkaline metal selected from the group consisting of Li, Na, K, Rb and Cs; M'^{II} is at least one divalent metal selected from the group consisting of Be and Mg; M^{III} is at least one trivalent metal selected from the group consisting of Al, Ga, In and Ti; A is at least one metal oxide; X is at least one halogen selected from the group consisting of Cl, Br and I; each of X', X" and X"' is at least one halogen selected from the group consisting of F, Cl, Br and I; a is equal to or greater than 0 and equal to or less than 2; b is equal to or greater than 0 and equal to or less than 10⁻²; c is equal to or greater than 0 and equal to or less than 10⁻²; a+b+c is equal to or greater than 10⁻²; x is greater than 0 and equal to or less than 0.5; and y is greater than 0 and equal to or less than 0.2) disclosed in U.S. Patent No. 4,962,047.

In the present invention, the stimulable phosphor usable for storing the energy of chemiluminescence emission may be of any type insofar as it can store the energy of light in the wavelength band of visible light and can be stimulated by an electromagnetic wave to release in the form of light the energy of light in the wavelength band of visible light stored therein. More specifically, preferably employed stimulable phosphors include at least one selected from the group consisting of metal halophosphates, rare-earth-activated sulfide-host phosphors, aluminate-host phosphors, silicate-host phosphors, fluoride-host phosphors and mixtures of two, three or more of these phosphors. Among them, rare-earth-activated sulfide-host phosphors are more preferable and, particularly, rare-earth-activated alkaline earth metal sulfide-host phosphors disclosed in U.S. Patent Nos. 5,029,253 and 4,983,834, zinc germanate such as Zn₂GeO₄:Mn, V; Zn₂GeO₄:Mn disclosed in Japanese Patent Application Laid Open No. 2001-131545, alkaline-earth aluminate such as Sr₄Al₁₄O₂₅:Ln (wherein Ln is a rare-earth element) disclosed in Japanese Patent Application Laid Open No. 2001-123162, Y_{0.8}Lu_{1.2}SiO₅:Ce, Zr; GdOCl:Ce disclosed in Japanese Patent Publication No. 6-31904 and the like are most preferable.

The above and other objects and features of the present invention will become apparent from the following description made with reference to the accompanying drawings.

### BRIEF DESCRIPTION OF THE DRAWINGS

Figure 1 is a schematic perspective view showing a biochemical analysis unit which is a preferred embodiment of the present invention.

Figure 2 is a schematic front view showing a spotting device.

Figure 3 is a schematic longitudinal cross sectional view showing a hybridization reaction vessel.

Figure 4 is a schematic perspective view showing a stimulable phosphor sheet which is a preferred embodiment of the present invention.

Figure 5 is a schematic perspective view showing an exposure device for exposing a number of the stimulable phosphor layer regions formed in the stimulable phosphor sheet to a radioactive labeling substance selectively contained in a number of the absorptive regions formed in the biochemical analysis unit.

Figure 6 is a schematic cross-sectional view showing a method for exposing a number of the stimulable phosphor layer regions formed in the stimulable phosphor sheet to a radioactive labeling substance contained in a number of the absorptive regions formed in the biochemical analysis unit.

Figure 7 is a schematic view showing a scanner for reading radiation data recorded in a number of the stimulable phosphor layer regions formed in the support of the stimulable phosphor sheet to produce biochemical analysis data.

Figure 8 is a schematic perspective view showing details in the vicinity of a photomultiplier of a scanner shown in Figure 7.

Figure 9 is a schematic cross-sectional view taken along a line A-A in Figure 8.

Figure 10 is a schematic cross-sectional view taken along a line B-B in Figure 8.

Figure 11 is a schematic cross-sectional view taken along a line C-C in Figure 8.

Figure 12 is a schematic cross-sectional view taken along a line D-D in Figure 8.

Figure 13 is a schematic plan view showing the scanning mechanism of an optical head.

Figure 14 is a block diagram of a control system, an input system, a drive system and a detection system of the scanner shown in Figure 7.

Figure 15 is a schematic perspective view showing a stimulable phosphor sheet onto which chemiluminescence data recorded in a number of absorptive regions formed in a substrate of a biochemical analysis unit are to be transferred, which is another preferred embodiment of the present invention.

Figure 16 is a schematic perspective view showing a scanner for reading chemiluminescence data recorded in a number of stimulable phosphor layer regions formed in a support 11 of a stimulable phosphor sheet shown in Figure 15 and producing biochemical analysis data,

Figure 17 is a schematic perspective view showing details in the vicinity of a photomultiplier of a scanner shown in Figure 16.

Figure 18 is a schematic cross-sectional view taken along a line E-E in Figure 17.

Figure 19 is a schematic perspective view showing an exposure device used for a method for exposing a stimulable phosphor sheet which a further preferred embodiment of the present invention.

Figure 20 is a block diagram of a control system, a detection system and a display system of the exposure device shown in Figure 19.

Figure 21 is a schematic perspective view showing a biochemical analysis unit which a further preferred embodiment of the present invention.

Figure 22 is a schematic perspective view showing a stimulable phosphor sheet onto which radiation data are to be transferred, which is a further preferred embodiment of the present invention.

Figure 23 is a schematic cross sectional view showing a method for exposing a number of stimulable phosphor layer regions formed in the stimulable phosphor sheet shown in Figure 22 to a radioactive labeling substance selectively contained in the corresponding absorptive region formed in the biochemical analysis unit shown in Figure 21.

Figure 24 is a schematic perspective view showing a stimulable phosphor sheet onto which chemiluminescence data are to be transferred, which is a further preferred embodiment of the present invention.

Figure 25 is a schematic perspective view showing a biochemical analysis unit which is a further preferred embodiment of the present invention.

Figure 26 is a schematic perspective view showing a stimulable phosphor sheet onto which radiation data are to be transferred, which is a further preferred embodiment of the present invention.

Figure 27 is a schematic view showing an internal structure of an exposure device used for a method for exposing a stimulable phosphor sheet which is a further preferred embodiment of the present invention.

Figure 28 is a block diagram of a control system, a detection system and a memory system of a CCD area sensor and a control system, a memory system and a display system of an exposure device shown in Figure 27.

Figure 29 is a schematic perspective view showing a stimulable phosphor sheet onto which chemiluminescence data are to be transferred, which is a further preferred embodiment of the present invention.

Figure 30 is a schematic perspective view showing a biochemical analysis unit which is a further preferred embodiment of the present invention.

Figure 31 is a schematic perspective view showing a stimulable phosphor sheet onto which radiation data are to be transferred, which is a further preferred embodiment of the present invention.

Figure 32 is a schematic cross sectional view showing an exposure device used for a method for exposing a stimulable phosphor sheet which is a further preferred embodiment of the present invention.

Figure 33 is a schematic perspective view showing a stimulable phosphor sheet onto which chemiluminescence data are to be transferred, which is a further preferred embodiment of the present invention.

Figure 34 is a schematic perspective view showing a biochemical analysis unit which is a further preferred embodiment of the present invention.

Figure 35 is a schematic perspective view showing a stimulable phosphor sheet onto which radiation data are to be transferred, which is a further preferred embodiment of the present invention.

Figure 36 is a schematic cross sectional view showing a method for exposing a number of stimulable phosphor layer regions formed in a support of the stimulable phosphor sheet shown in Figure 35 to a radioactive labeling substance selectively contained in a number of absorptive regions formed in a substrate of the biochemical analysis unit shown in Figure 34.

Figure 37 is a schematic perspective view showing a stimulable phosphor sheet onto which chemiluminescence data are to be transferred, which is a further preferred embodiment of the present invention.

### DESCRIPTION OF THE PREFERRED EMBODIMENTS

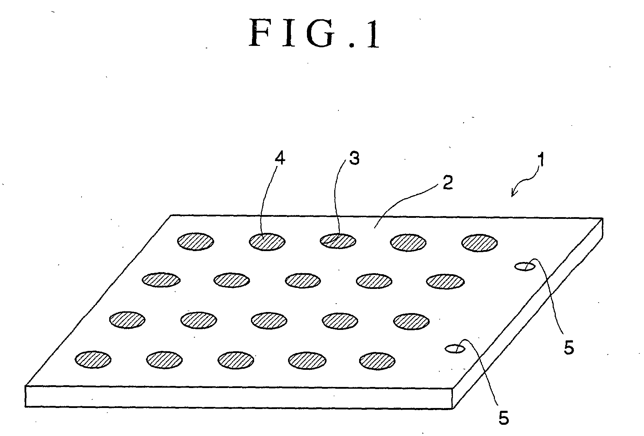

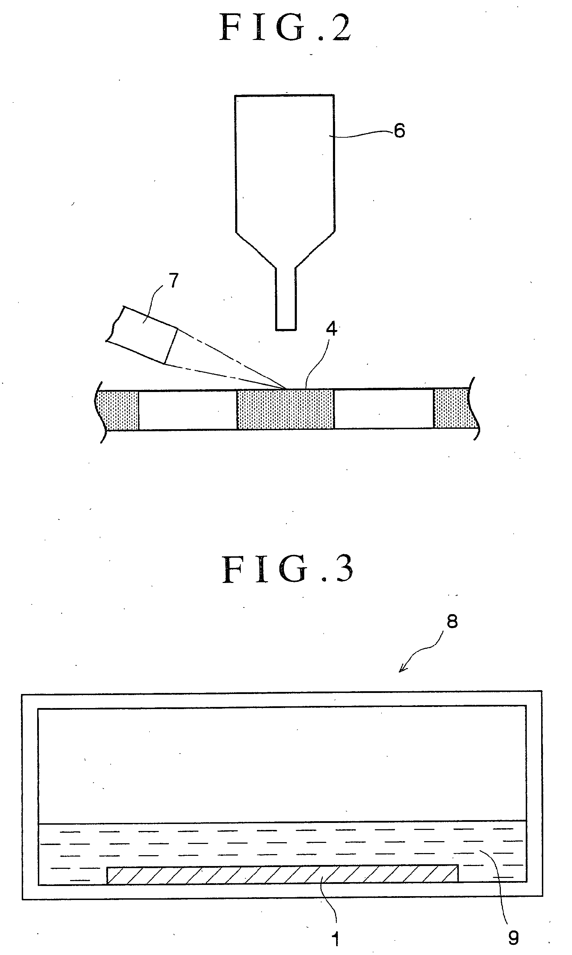

Figure 1 is a schematic perspective view showing a biochemical analysis unit which is a preferred embodiment of the present invention and Figure 2 is a schematic cross sectional view thereof.

As shown in Figure 1, a biochemical analysis unit 1 according to this embodiment includes a substrate 2 made of stainless steel and formed with a number of substantially circular through-holes 3 at a high density, and a number of absorptive regions 4 are dot-like formed by charging nylon-6 in the through-holes 3.

Although not accurately shown in Figure 1, in this embodiment, about 10,000 through-holes 3 having a size of about 0.01 mm² are regularly formed at a density of about 5,000 per cm² in the substrate 2.

A number of absorptive regions 4 are formed by charging nylon-6 in the through-holes 3 formed in the substrate in such a manner that the surfaces of the absorptive regions 4 are located at the same height level as that of the substrate.

As shown in Figure 1, the substrate 2 of the biochemical analysis unit 1 is formed with two circular positioning through-holes 5.

Figure 2 is a schematic front view showing a spotting device.

As shown in Figure 2, when biochemical analysis is performed, a solution containing specific binding substances such as a plurality of cDNAs whose sequences are known but differ from each other are spotted using a spotting device onto a number of the absorptive regions 4 of the biochemical analysis unit 1 and the specific binding substances are fixed therein.

As shown in Figure 2, the spotting device includes an injector 6 for ejecting a solution of specific binding substances toward the biochemical analysis unit 1 and a CCD camera 7 and is constituted so that the solution of specific binding substances such as cDNAs are spotted from the injector 6 when the tip end portion of the injector 6 and the center of the absorptive region 4 into which the solution containing specific binding substances is to be spotted are determined to coincide with each other as a result of viewing them using the CCD camera, thereby ensuring that the solution of specific binding substances can be accurately spotted into a number of the absorptive regions 4 of the biochemical analysis unit 1.

Figure 3 is a schematic longitudinal cross sectional view showing a hybridization reaction vessel.

As shown in Figure 3, a hybridization reaction vessel 8 is formed to have a substantially rectangular cross section and accommodates a hybridization solution 9 containing a substance derived from a living organism labeled with a labeling substance as a probe therein.

In the case where a specific binding substance such as cDNA is to be labeled with a radioactive labeling substance, a hybridization solution 9 containing a substance derived from a living organism and labeled with a radioactive labeling substance as a probe is prepared and is accommodated in the hybridization reaction vessel 8.

On the other hand, in the case where a specific binding substance such as cDNA is to be labeled with a labeling substance which generates chemiluminescence emission when it contacts a chemiluminescent substrate, a hybridization solution 9 containing a substance derived from a living organism and labeled with a labeling substance which generates chemiluminescence emission when it contacts a chemiluminescent substrate as a probe is prepared and is accommodated in the hybridization reaction vessel 8.

Further, in the case where a specific binding substance such as cDNA is to be labeled with a fluorescent substance such as a fluorescent dye, a hybridization solution 9 containing a substance derived from a living organism and labeled with a fluorescent substance such as a fluorescent dye as a probe is prepared and is accommodated in the hybridization reaction vessel 8.

It is possible to prepare a hybridization reaction solution 9 containing two or more substances derived from a living organism among a substance derived from a living organism and labeled with a radioactive labeling substance, a substance derived from a living organism and labeled with a labeling substance which generates chemiluminescence emission when it contacts a chemiluminescent substrate and a substance derived from a living organism and labeled with a fluorescent substance such as a fluorescent dye and accommodate it in the hybridization vessel 8. In this embodiment, a hybridization reaction solution 9 containing a substance derived from a living organism and labeled with a radioactive labeling substance, a substance derived from a living organism and labeled with a labeling substance which generates chemiluminescence emission when it contacts a chemiluminescent substrate and a substance derived from a living organism and labeled with a fluorescent substance such as a fluorescent dye is prepared and accommodated in the hybridization reaction vessel 8.

When hybridization is to be performed, the biochemical analysis unit 1 containing specific binding substances such as a plurality of cDNAs spotted into a number of absorptive regions 4 is accommodated in the hybridization reaction vessel 8.

As a result, specific binding substances spotted in a number of the absorptive regions 4 of the biochemical analysis unit 1 can be selectively hybridized with a substance derived from a living organism, labeled with a radioactive labeling substance and contained in the hybridization reaction solution 9, a substance derived from a living organism, labeled with a labeling substance which generates chemiluminescence emission when it contacts a chemiluminescent substrate and contained in the hybridization reaction solution 9 and a substance derived from a living organism, labeled with a fluorescent substance such as a fluorescent dye and contained in the hybridization reaction solution 9.

In this manner, radiation data of a radioactive labeling substance, chemiluminescence data of a labeling substance which generates chemiluminescence emission when it contacts a chemiluminescent substrate and fluorescence data of a fluorescent substance such as a fluorescent dye are recorded in a number of absorptive regions 4 formed in the biochemical analysis unit 1.

Fluorescence data recorded in a number of absorptive regions 4 formed in the biochemical analysis unit 1 are read by a scanner described later, thereby producing biochemical analysis data.

On the other hand, radiation data of the radioactive labeling substance recorded in a number of absorptive regions 4 formed in the biochemical analysis unit 1 are transferred onto a stimulable phosphor sheet and read by the scanner described later, thereby producing biochemical analysis data.

Further, chemiluminescence data recorded in a number of absorptive regions 4 formed in the biochemical analysis unit 1 are transferred onto a stimulable phosphor sheet described later and transferred chemiluminescence data are read by another scanner described later, thereby producing biochemical analysis data.

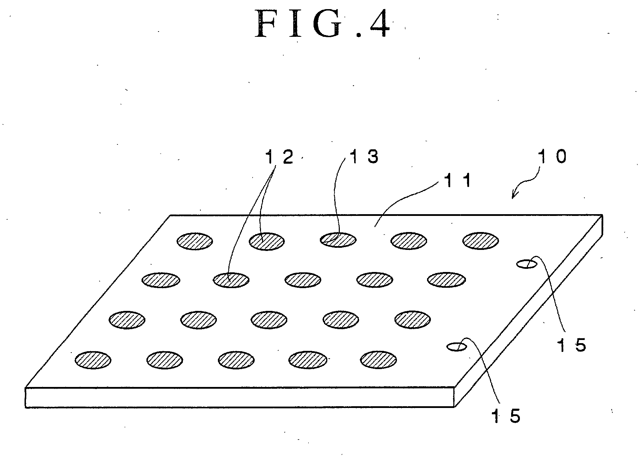

Figure 4 is a schematic perspective view showing a stimulable phosphor sheet which is a preferred embodiment of the present invention.

As shown in Figure 4, a stimulable phosphor sheet 10 according to this embodiment includes a support 11 made of stainless steel and regularly formed with a number of substantially circular through-holes 13 and a number of stimulable phosphor layer regions 12 are dot-like formed by charging BaFX system stimulable phosphor (where X is at least one halogen atom selected from the group consisting of Cl, Br and I) capable of absorbing and storing radiation energy in the through-holes 13.

A number of the through-holes 13 are formed in the support 11 in the same pattern as that of a number of the absorptive regions 4 formed in the substrate 2 of the biochemical analysis unit 1 and each of them has the same size as that of the absorptive region 4 formed in the substrate 2 of the biochemical analysis unit 1.

Therefore, although not accurately shown in Figure 4, in this embodiment, about 10,000 substantially circular stimulable phosphor layer regions 12 having a size of about 0.01 mm² are dot-like formed in a regular pattern at a density of about 5,000 per cm² in the support 11 of the stimulable phosphor sheet 10.

In this embodiment, stimulable phosphor is charged in a number of the through-holes 13 formed in the support 11 in such a manner that the surfaces of the stimulable phosphor layer regions 12 lie at the same height level of that of the surface of the support 11.

As shown in Figure 4, the support 11 of the stimulable phosphor sheet 10 is formed with two circular positioning through-holes 15 at positions corresponding to the two positioning through-holes 5 formed in the substrate 2 of the biochemical analysis unit 1 and each of the positioning through-holes 15 has the same size as that of the positioning through-hole 5 formed in the substrate 2 of the biochemical analysis unit 1.

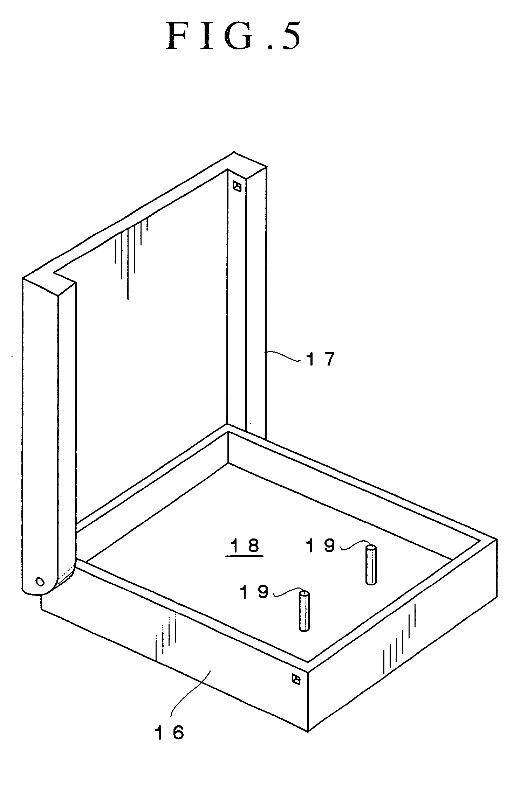

Figure 5 is a schematic perspective view showing an exposure device for exposing a number of the stimulable phosphor layer regions 12 formed in the stimulable phosphor sheet 10 to a radioactive labeling substance selectively contained in a number of the absorptive regions 4 formed in the biochemical analysis unit 1.

As shown in Figure 5, the exposure device includes a case 16 and a lid member 17 and a base plate 18 on which the biochemical analysis unit 1 and the stimulable phosphor sheet 10 are to be placed is provided in the case 16.

The case 16 and the lid member 17 are made of a material capable of attenuating radiation energy such as stainless steel and two erect positioning pins 19, 19 are formed on the base plate 18 at positions corresponding to the two positioning through-holes 5 formed in the substrate 2 of the biochemical analysis unit 1 and the two positioning through-holes 15 formed in the support 11 of the stimulable phosphor sheet 10.

Each of the two positioning pins 19, 19 has an outer diameter slightly smaller than the inner diameter of the two positioning through-holes 5 formed in the substrate 2 of the biochemical analysis unit 1 and that of the two positioning through-holes 15 formed in the support 11 of the stimulable phosphor sheet 10 so that there is no play when the two positioning pins 19, 19 are inserted into the two positioning through-holes 5 formed in the substrate 2 of the biochemical analysis unit 1 and the two positioning through-holes 15 formed in the support 11 of the stimulable phosphor sheet 10.

When a number of the stimulable phosphor layer regions 12 formed in the stimulable phosphor sheet 10 are to be exposed to a radioactive labeling substance selectively contained in a number of the absorptive regions 4 formed in the biochemical analysis unit 1, the biochemical analysis unit 1 is first set on the base plate 18 of the exposure device in such a manner that the two positioning pins 19, 19 formed on the base plate 18 are inserted into the two positioning through-holes 5.

The stimulable phosphor sheet 10 is then set on the biochemical analysis unit 1 set on the base plate 18 of the exposure device in such a manner that the two positioning pins 19, 19 formed on the base plate 18 are inserted into the two positioning through-holes 15.

In this manner, since the biochemical analysis unit 1 and the stimulable phosphor sheet 10 are set on the base plate 18 of the exposure device in such a manner that the two positioning pins 19, 19 formed on the base plate 18 of the exposure device are inserted into the two positioning through-holes 5 and the two positioning through-holes 15, the biochemical analysis unit 1 and the stimulable phosphor sheet 10 can be positioned and superposed so that each of a number of the absorptive regions 4 formed in the substrate 2 of the biochemical analysis unit 1 faces one of a number of the stimulable phosphor layer regions 12 formed in the support 11 of the stimulable phosphor sheet 10 in the same pattern as that of a number of the absorptive regions 4, thereby exposing a number of the stimulable phosphor layer regions 12 formed in the support 11 of the stimulable phosphor sheet 10 to a radioactive labeling substance selectively contained in a number of the absorptive regions 4 formed in the substrate 2 of the biochemical analysis unit 1.

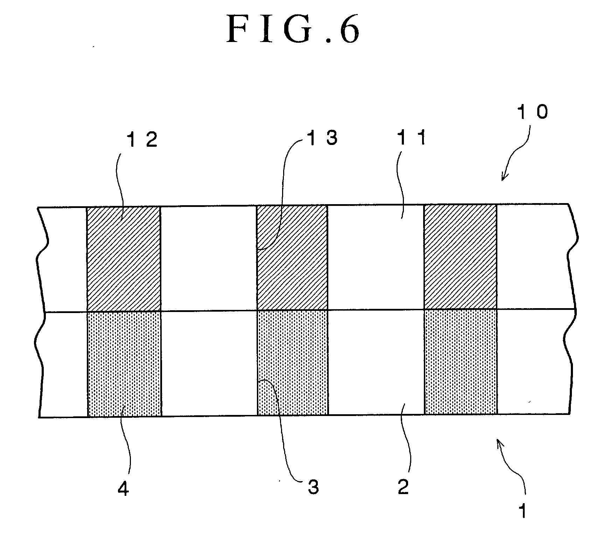

Figure 6 is a schematic cross-sectional view showing a method for exposing a number of the stimulable phosphor layer regions 12 formed in the stimulable phosphor sheet 10 by a radioactive labeling substance contained in a number of the absorptive regions 4 formed in the biochemical analysis unit 1.

In this embodiment, since the biochemical analysis unit 1 is formed by charging nylon-6 in a number of the through-holes 3 formed in the substrate 2 made of stainless steel, the biochemical analysis unit 1 does not substantially stretch or shrink when it is subjected to liquid processing such as hybridization and, therefore, it is possible to superpose the stimulable phosphor sheet 10 on the biochemical analysis unit 1 so that each of the stimulable phosphor layer regions 12 formed in the support 11 of the stimulable phosphor sheet 10 accurately faces the corresponding absorptive region 4 formed in the substrate 2 of the biochemical analysis unit 1 by setting the biochemical analysis unit 1 and the stimulable phosphor sheet 10 on the base plate 18 of the exposure device in such a manner that the two positioning pins 19, 19 formed on the base plate 18 of the exposure device are inserted into the two positioning through-holes 5 and the two positioning through-holes 15, thereby exposing the stimulable phosphor layer regions 12.

In this manner, each of the stimulable phosphor layer regions 12 formed in the support 11 of the stimulable phosphor sheet 10 is kept to face the corresponding absorptive region 4 formed in the substrate 2 of the biochemical analysis unit 1 for a predetermined time period, whereby a number of the stimulable phosphor layer regions 12 formed in the support 11 of the stimulable phosphor sheet 10 are exposed to the radioactive labeling substance contained in a number of the absorptive regions 4 formed in the substrate 2 of the biochemical analysis unit 1.

During the exposure operation, electron beams (β rays) are released from the radioactive labeling substance contained in the absorptive regions 4 of the biochemical analysis unit 1. However, since a number of the absorptive regions 4 of the biochemical analysis unit 1 are formed spaced apart from each other in the substrate 2 made of stainless steel and the substrate 2 made of stainless steel capable of attenuating radiation energy is present around each of the absorptive regions 4, electron beams (β rays) released from the radioactive labeling substance contained in the absorptive regions 4 of the biochemical analysis unit 1 can be efficiently prevented from scattering in the substrate 2 of the biochemical analysis unit 1. Further, since a number of the stimulable phosphor layer regions 12 of the stimulable phosphor sheet 10 are formed by charging stimulable phosphor in a number of the through-holes 13 formed in the support 11 made of stainless steel capable of attenuating radiation energy and the support 11 made of stainless steel is present around each of the stimulable phosphor layer regions 12, electron beams (β rays) released from the radioactive labeling substance contained in the absorptive regions 4 of the biochemical analysis unit 1 can be efficiently prevented from scattering in the support 11 of the stimulable phosphor sheet 10. Therefore, it is possible to cause all electron beams (β rays) released from the radioactive labeling substance contained in the absorptive region 4 to enter the stimulable phosphor layer region 12 the absorptive region 4 faces and to effectively prevent electron beams (β rays) released from the absorptive region 4 from entering stimulable phosphor layer regions 12 to be exposed to electron beams (β rays) released from neighboring absorptive regions 4.

In this manner, a number of the stimulable phosphor layer regions 12 formed in the support 11 of the stimulable phosphor sheet 10 are selectively exposed to a radioactive labeling substance contained in the corresponding absorptive region 4 of the biochemical analysis unit 1.

Thus, radiation data of a radioactive labeling substance are recorded in a number of the stimulable phosphor layer regions 12 formed in the support 11 of the stimulable phosphor sheet 10.

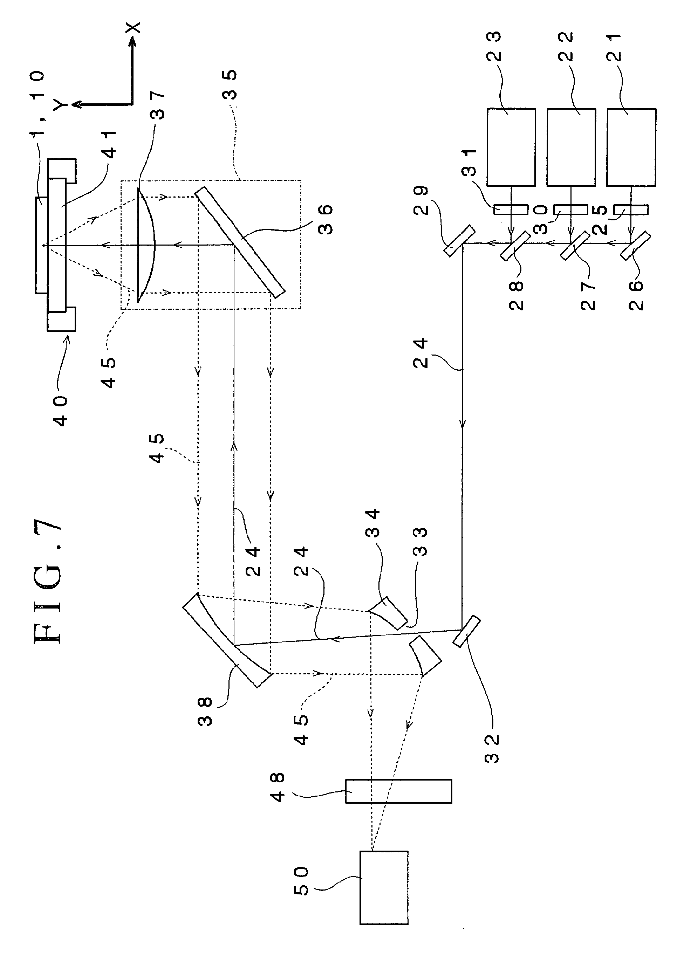

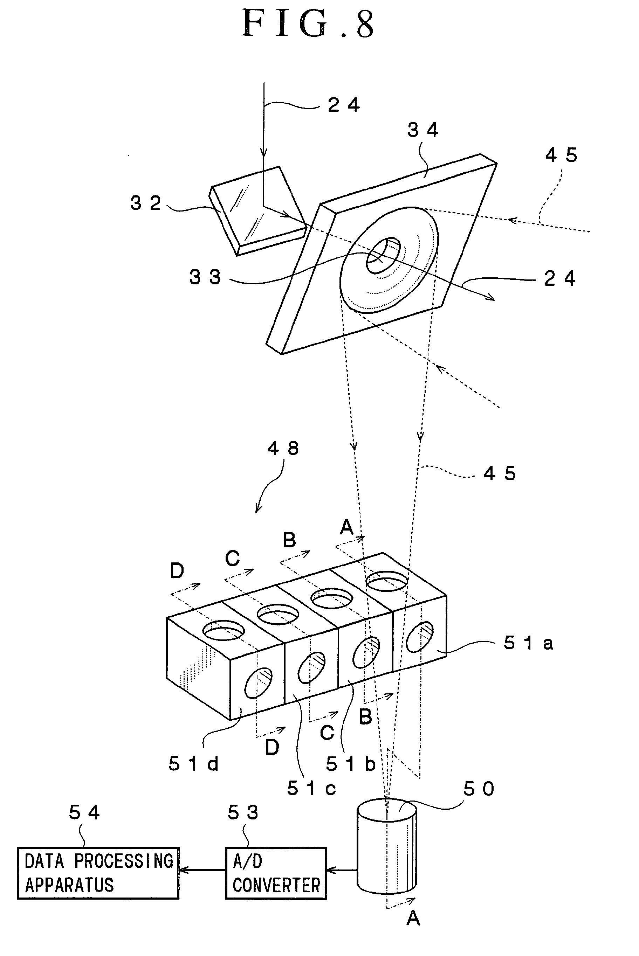

Figure 7 is a schematic view showing a scanner for reading radiation data recorded in a number of the stimulable phosphor layer regions 12 formed in the support 11 of the stimulable phosphor sheet 10 to produce biochemical analysis data and Figure 8 is a schematic perspective view showing details in the vicinity of a photomultiplier of a scanner shown in Figure 7.

The scanner shown in Figures 7 and 8 is constituted so as to read radiation data recorded in a number of the stimulable phosphor layer regions 123 formed in the support 11 of the stimulable phosphor sheet 10 and fluorescence data of a fluorescent substance such as a fluorescent dye recorded in a number of the absorptive regions 4 formed in the substrate 2 of the biochemical analysis unit 1 to produce biochemical analysis data.

As shown in Figure 7, the scanner includes a first laser stimulating ray source 21 for emitting a laser beam having a wavelength of 640 nm, a second laser stimulating ray source 22 for emitting a laser beam having a wavelength of 532 nm and a third laser stimulating ray source 23 for emitting a laser beam having a wavelength of 473 nm.

In this embodiment, the first laser stimulating ray source 21 is constituted by a semiconductor laser beam source and the second laser stimulating ray source 22 and the third laser stimulating ray source 23 are constituted by a second harmonic generation element.

A laser beam 24 emitted from the first laser stimulating source 21 passes through a collimator lens 25, thereby being made a parallel beam, and is reflected by a mirror 26. A first dichroic mirror 27 for transmitting light having a wavelength of 640 nm but reflecting light having a wavelength of 532 nm and a second dichroic mirror 28 for transmitting light having a wavelength equal to and longer than 532 nm but reflecting light having a wavelength of 473 nm are provided in the optical path of the laser beam 24 emitted from the first laser stimulating ray source 21. The laser beam 24 emitted from the first laser stimulating ray source 21 and reflected by the mirror 26 passes through the first dichroic mirror 27 and the second dichroic mirror 28 and advances to a mirror 29.

On the other hand, the laser beam 24 emitted from the second laser stimulating ray source 22 passes through a collimator lens 30, thereby being made a parallel beam, and is reflected by the first dichroic mirror 27, thereby changing its direction by 90 degrees. The laser beam 24 then passes through the second dichroic mirror 28 and advances to the mirror 29.

Further, the laser beam 24 emitted from the third laser stimulating ray source 23 passes through a collimator lens 31, thereby being made a parallel beam, and is reflected by the second dichroic mirror 28, thereby changing its direction by 90 degrees. The laser beam 24 then advances to the mirror 29.

The laser beam 24 advancing to the mirror 29 is reflected by the mirror 29 and advances to a mirror 32 to be reflected thereby.

A perforated mirror 34 formed with a hole 33 at the center portion thereof is provided in the optical path of the laser beam 24 reflected by the mirror 32. The laser beam 24 reflected by the mirror 32 passes through the hole 33 of the perforated mirror 34 and advances to a concave mirror 38.

The laser beam 24 advancing to the concave mirror 38 is reflected by the concave mirror 38 and enters an optical head 35.

The optical head 35 includes a mirror 36 and an aspherical lens 37. The laser beam 24 entering the optical head 35 is reflected by the mirror 36 and condensed by the aspherical lens 37 onto the stimulable phosphor sheet 10 or the biochemical analysis unit 1 placed on the glass plate 41 of a stage 40.

When the laser beam 24 impinges on one of the stimulable phosphor layer regions 12 formed in the support 11 of the stimulable phosphor sheet 10, stimulable phosphor contained in the stimulable phosphor layer region 12 is excited, thereby releasing stimulated emission 45. On the other hand, when the laser beam 24 impinges on one of the absorptive regions 4 formed in the substrate 2 of the biochemical analysis unit 1, a fluorescent dye or the like contained in the absorptive region 4 is excited, thereby releasing fluorescence emission 45.

The stimulated emission 45 released from the stimulable phosphor layer region 12 formed in the support 11 of the stimulable phosphor 10 or the fluorescence emission 45 released from the absorptive region 4 formed in the substrate 2 of the biochemical analysis unit 1 is condensed onto the mirror 36 by the aspherical lens 37 provided in the optical head 35 and reflected by the mirror 36 on the side of the optical path of the laser beam 24, thereby being made a parallel beam to advance to the concave mirror 38.

The stimulated emission 45 or the fluorescence emission 45 advancing to the concave mirror 38 is reflected by the concave mirror 38 and advances to the perforated mirror 34.

As shown in Figure 8, the stimulated emission 45 or the fluorescence emission 45 advancing to the perforated mirror 34 is reflected downward by the perforated mirror 34 formed as a concave mirror and advances to a filter unit 48, whereby light having a predetermined wavelength is cut. The stimulated emission 45 or the fluorescence emission 45 then impinges on a photomultiplier 50, thereby being photoelectrically detected.

As shown in Figure 8, the filter unit 48 is provided with four filter members 51a, 51b, 51c and 51d and is constituted to be laterally movable in Figure 8 by a motor (not shown).

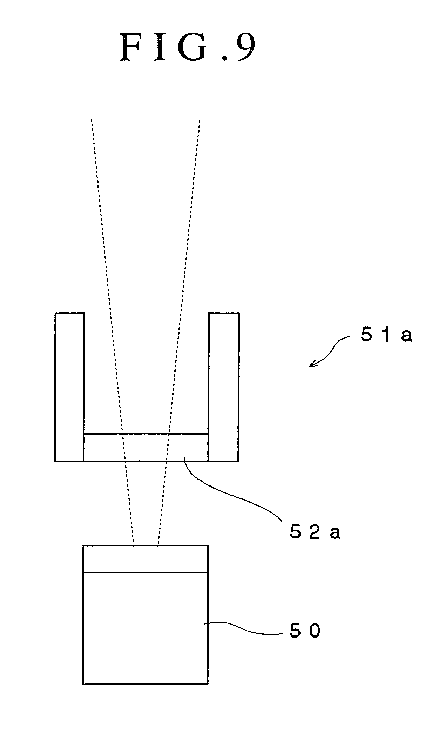

Figure 9 is a schematic cross-sectional view taken along a line A-A in Figure 8.

As shown in Figure 9, the filter member 51a includes a filter 52a and the filter 52a is used for reading fluorescence emission 45 by stimulating a fluorescent substance such as a fluorescent dye contained in a number of the absorptive regions 4 formed in the substrate 2 of the biochemical analysis unit 1 using the first laser stimulating ray source 21 and has a property of cutting off light having a wavelength of 640 nm but transmitting light having a wavelength longer than 640 nm.

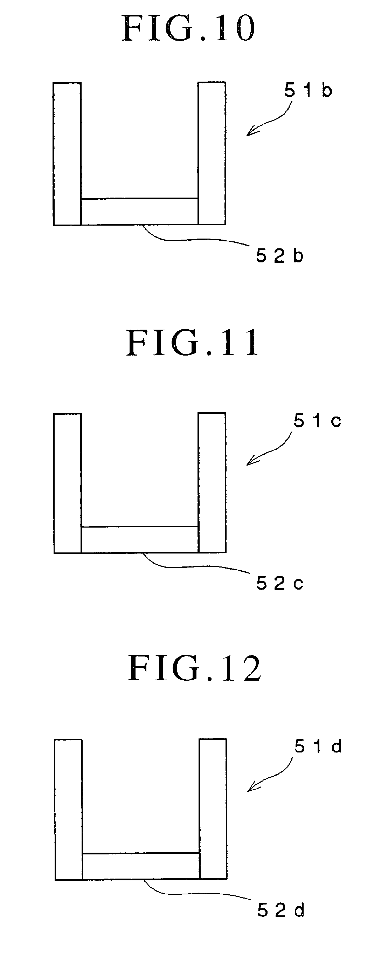

Figure 10 is a schematic cross-sectional view taken along a line B-B in Figure 8.

As shown in Figure 10, the filter member 51b includes a filter 52b and the filter 52b is used for reading fluorescence emission 45 by stimulating a fluorescent substance such as a fluorescent dye contained in a number of the absorptive regions 4 formed in the substrate 2 of the biochemical analysis unit 1 using the second laser stimulating ray source 22 and has a property of cutting off light having a wavelength of 532 nm but transmitting light having a wavelength longer than 532 nm.

Figure 11 is a schematic cross-sectional view taken along a line C-C in Figure 8.

As shown in Figure 11, the filter member 51c includes a filter 52c and the filter 52c is used for reading fluorescence emission 45 by stimulating a fluorescent substance such as a fluorescent dye contained in in a number of the absorptive regions 4 formed in the substrate 2 of the biochemical analysis unit 1 using the third laser stimulating ray source 23 and has a property of cutting off light having a wavelength of 473 nm but transmitting light having a wavelength longer than 473 nm.

Figure 12 is a schematic cross-sectional view taken along a line D-D in Figure 8.

As shown in Figure 12, the filter member 51d includes a filter 52d and the filter 52d is used for reading stimulated emission 45 released from stimulable phosphor contained in the stimulable phosphor layer 12 formed in the support 11 of the stimulable phosphor sheet 10 upon being stimulated using the first laser stimulating ray source 1 and has a property of transmitting only light having a wavelength corresponding to that of stimulated emission 45 emitted from stimulable phosphor and cutting off light having a wavelength of 640 nm.

Therefore, in accordance with the kind of a stimulating ray source to be used, one of these filter members 51a, 51b, 51c, 51d is selectively positioned in front of the photomultiplier 50, thereby enabling the photomultiplier 50 to photoelectrically detect only light to be detected.

The analog data produced by photoelectrically detecting stimulated emission 45 or fluorescence emission 45 with the photomultiplier 50 are converted by an A/D converter 53 into digital data and the digital data are fed to a data processing apparatus 54.

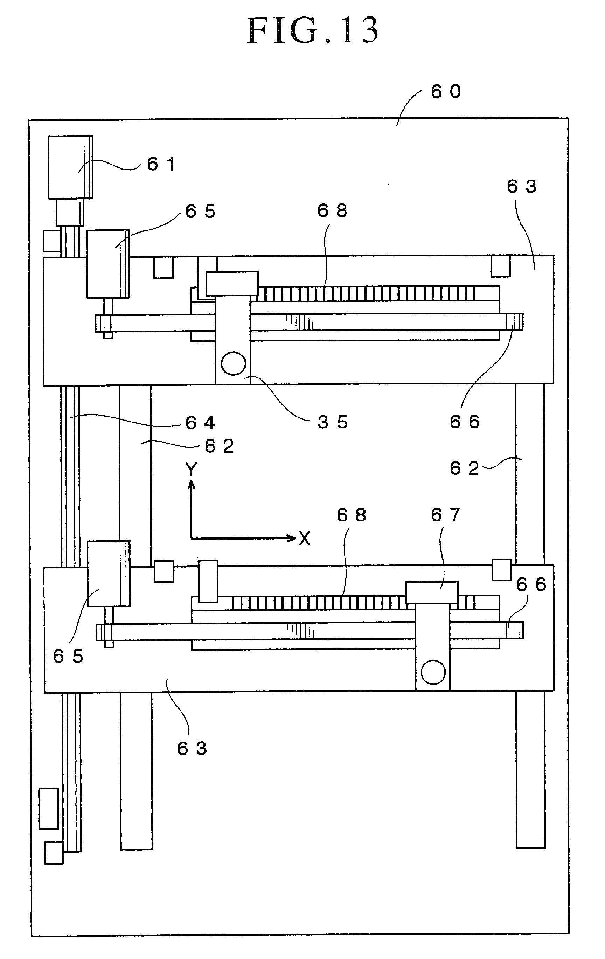

Figure 13 is a schematic plan view showing the scanning mechanism of the optical head 35.

In Figure 13, optical systems other than the optical head 35 and the paths of the laser beam 24 and stimulated emission 45 or fluorescence emission 45 are omitted for simplification.

As shown in Figure 13, the scanning mechanism of the optical head 35 includes a base plate 60, and a sub-scanning pulse motor 61 and a pair of rails 62, 62 are fixed on the base plate 60. A movable base plate 63 is further provided so as to be movable in the sub-scanning direction indicated by an arrow Y in Figure 13.

The movable base plate 63 is formed with a threaded hole (not shown) and a threaded rod 64 rotated by the sub-scanning pulse motor 61 is engaged with the inside of the hole.

A main scanning stepping motor 65 is provided on the movable base plate 63. The main scanning stepping motor 65 is adapted for intermittently driving an endless belt 66 at a pitch equal to the distance between neighboring absorptive regions 4 formed in the biochemical analysis unit 1, namely, the distance between neighboring stimulable phosphor layer regions 12 formed in the stimulable phosphor sheet 10.

The optical head 35 is fixed to the endless belt 66 and when the endless belt 66 is driven by the main scanning stepping motor 65, the optical head 35 is moved in the main scanning direction indicated by an arrow X in Figure 13.

In Figure 13, the reference numeral 67 designates a linear encoder for detecting the position of the optical head 35 in the main scanning direction and the reference numeral 68 designates slits of the linear encoder 67.

Therefore, the optical head 35 is moved in the main scanning direction indicated by the arrow X and the sub-scanning direction indicated by the arrow Y in Figure 13 by driving the endless belt 66 in the main scanning direction by the main scanning stepping motor 65 and intermittently moving the movable base plate 63 in the sub-scanning direction by the sub-scanning pulse motor 61, thereby scanning all of the stimulable phosphor layer regions 12 formed in the support 11 of the stimulable phosphor sheet 10 or all of the absorptive regions 4 formed in the substrate 2 of the biochemical analysis unit 1 with the laser beam 24.

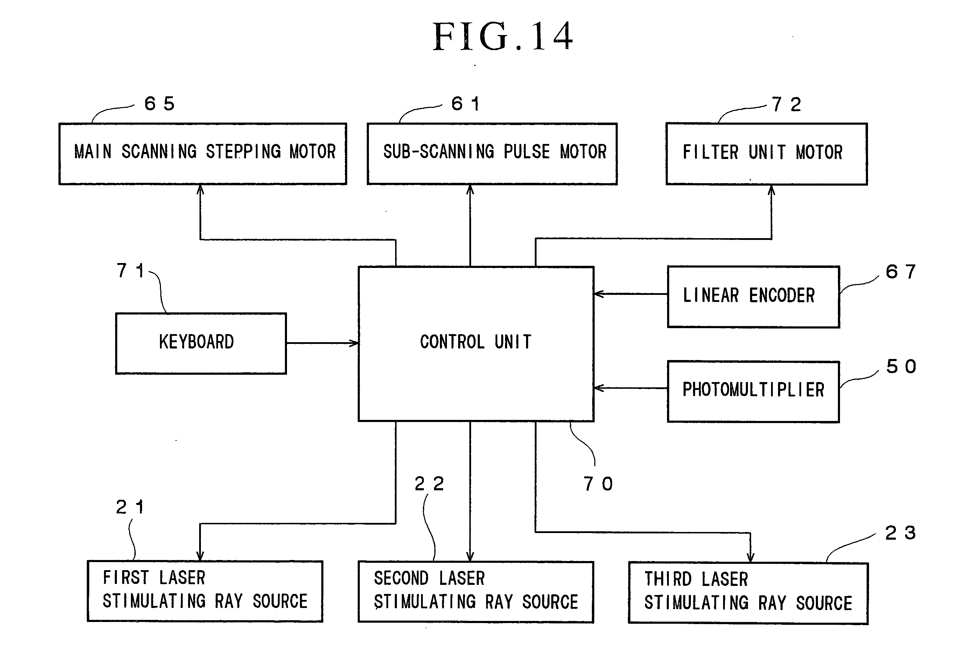

Figure 14 is a block diagram of a control system, an input system, a drive system and a detection system of the scanner shown in Figure 7.

As shown in Figure 14, the control system of the scanner includes a control unit 70 for controlling the overall operation of the scanner and the input system of the scanner includes a keyboard 71 which can be operated by a user and through which various instruction signals can be input.

As shown in Figure 14, the drive system of the scanner includes the main scanning stepping motor 65 for intermittently moving the optical head 35 in the main scanning direction, the sub-scanning pulse motor 61 for moving the optical head 35 in the sub-scanning direction and a filter unit motor 72 for moving the filter unit 48 provided with the four filter members 51a, 51b, 51c and 51d.

The control unit 70 is adapted for selectively outputting a drive signal to the first laser stimulating ray source 21, the second laser stimulating ray source 22 or the third laser stimulating ray source 23 and outputting a drive signal to the filter unit motor 72.

As shown in Figure 14, the detection system of the scanner includes the photomultiplier 50 and the linear encoder 67 for detecting the position of the optical head 35 in the main scanning direction.

In this embodiment, the control unit 70 is adapted to control the on and off operation of the first laser stimulating ray source 21, the second laser stimulating ray source 22 or the third laser stimulating ray source 23 in accordance with a detection signal indicating the position of the optical head 35 input from the linear encoder 67.

The thus constituted scanner reads radiation data recorded in a number of the stimulable phosphor layer regions 12 formed in the support 11 of the stimulable phosphor sheet 10 and produces biochemical analysis data in the following manner.

A stimulable phosphor sheet 10 is first set on the glass plate 41 of the stage 40 by a user.

An instruction signal indicating that radiation data recorded in the stimulable phosphor layer region 12 formed in the support 11 of the stimulable phosphor sheet 10 are to be read is then input through the keyboard 71.

The instruction signal input through the keyboard 71 is input to the control unit 70 and the control unit 70 outputs a drive signal to the filter unit motor 72 in accordance with the instruction signal, thereby moving the filter unit 48 so as to locate the filter member 51d provided with the filter 52d having a property of transmitting only light having a wavelength corresponding to that of stimulated emission emitted from stimulable phosphor but cutting off light having a wavelength of 640 nm in the optical path of stimulated emission 45.

The control unit 70 further outputs a drive signal to the main scanning stepping motor 65 to move the optical head 35 in the main scanning direction and when it determines based on a detection signal indicating the position of the optical head 35 input from the linear encoder 67 that the optical head 35 has reached a position where a laser beam 24 can be projected onto a first stimulable phosphor layer region 12 among a number of the stimulable phosphor layer regions 12 formed in the support 11 of the stimulable phosphor sheet 10, it outputs a drive stop signal to the main scanning stepping motor 65 and a drive signal to the first stimulating ray source 21, thereby actuating it to emit a laser beam 24 having a wavelength of 640 nm.

A laser beam 24 emitted from the first laser stimulating source 21 passes through the collimator lens 25, thereby being made a parallel beam, and is reflected by the mirror 26.

The laser beam 24 reflected by the mirror 26 passes through the first dichroic mirror 27 and the second dichroic mirror 28 and advances to the mirror 29.

The laser beam 24 advancing to the mirror 29 is reflected by the mirror 29 and advances to the mirror 32 to be reflected thereby.

The laser beam 24 reflected by the mirror 32 passes through the hole 33 of the perforated mirror 34 and advances to the concave mirror 38.

The laser beam 24 advancing to the concave mirror 38 is reflected by the concave mirror 38 and enters the optical head 35.

The laser beam 24 entering the optical head 35 is reflected by the mirror 36 and condensed by the aspherical lens 37 onto the first stimulable phosphor layer region 12 of the stimulable phosphor sheet 10 placed on the glass plate 41 of a stage 40.

In this embodiment, since the stimulable phosphor layer regions 12 are formed by charging stimulable phosphor in a number of the through-holes 13 formed in the support 11 made of stainless steel capable of attenuating light energy, it is possible to effectively prevent the laser beam 24 from scattering in each of the stimulable phosphor layer regions 12 and entering the neighboring stimulable phosphor layer regions 12 to excite stimulable phosphor contained in the neighboring stimulable phosphor layer regions 12.

When the laser beam 24 impinges onto the first stimulable phosphor layer region 12 formed in the support 11 of the stimulable phosphor sheet 10, stimulable phosphor contained in the first stimulable phosphor layer region 12 is excited by the laser beam 24, thereby releasing stimulated emission 45 from the first stimulable phosphor layer region 12.

The stimulated emission 45 released from the first stimulable phosphor layer region 12 is condensed onto the mirror 36 by the aspherical lens 37 provided in the optical head 35 and reflected by the mirror 36 on the side of the optical path of the laser beam 24, thereby being made a parallel beam to advance to the concave mirror 38.

The stimulated emission 45 advancing to the concave mirror 38 is reflected by the concave mirror 38 and advances to the perforated mirror 34.

As shown in Figure 8, the stimulated emission 45 advancing to the perforated mirror 34 is reflected downward by the perforated mirror 34 formed as a concave mirror and advances to the filter 52d of the filter unit 48.

Since the filter 52d has a property of transmitting only light having a wavelength corresponding to that of stimulated emission emitted from stimulable phosphor and cutting off light having a wavelength of 640 nm, light having a wavelength of 640 nm corresponding to that of the stimulating ray is cut off by the filter 52d and only light having a wavelength corresponding to that of stimulated emission passes through the filter 52d to be photoelectrically detected by the photomultiplier 50.

Analog data produced by photoelectrically detecting stimulated emission 45 with the photomultiplier 50 are converted by the A/D converter 53 into digital data and the digital data are fed to the data processing apparatus 54.

When a predetermined time, for example, several microseconds, has passed after the first stimulating ray source 21 was turned on, the control unit 70 outputs a drive stop signal to the first stimulating ray source 21, thereby turning it off and outputs a drive signal to the main scanning stepping motor 65, thereby moving the optical head 35 by one pitch equal to the distance between neighboring stimulable phosphor layer regions 12 of the stimulable phosphor sheet 10.

When the control unit 70 determines based on a detection signal indicating the position of the optical head 35 input from the linear encoder 67 that the optical head 35 has been moved by one pitch equal to the distance between neighboring stimulable phosphor layer regions 12 and has reached a position where a laser beam 24 can be projected onto a second stimulable phosphor layer region 12 next to the first stimulable phosphor layer region 12 formed in the stimulable phosphor sheet 10, it outputs a drive signal to the first stimulating ray source 21 to turn it on, thereby causing the laser beam 24 to excite stimulable phosphor contained in the second stimulable phosphor layer region 12 formed in the stimulable phosphor sheet 10 next to the first stimulable phosphor layer region 12.

Similarly to the above, the second stimulable phosphor layer region 12 formed in the support 11 of the stimulable phosphor sheet 10 is irradiated with the laser beam 24 emitted from the first laser stimulating ray source 21 for a predetermined time and when biochemical analysis data have been produced from radiation data recorded in the second stimulable phosphor layer region 12 by photoelectrically detecting stimulated emission 45 released from the second stimulable phosphor layer region 12 in response to the excitation of stimulable phosphor with the photomultiplier 50 to produce analog data and digitizing the analog data by the A/D converter 53, the control unit 70 outputs a drive stop signal to the first stimulating ray source 21, thereby turning it off and outputs a drive signal to the main scanning stepping motor 65, thereby moving the optical head 35 by one pitch equal to the distance between neighboring stimulable phosphor layer regions 12.

In this manner, the on and off operation of the first stimulating ray source 21 is repeated in synchronism with the intermittent movement of the optical head 35 and when the control unit 70 determines based on a detection signal indicating the position of the optical head 35 input from the linear encoder 67 that the optical head 35 has been moved by one scanning line in the main scanning direction and that the stimulable phosphor layer regions 12 included in a first line of the stimulable phosphor layer regions 12 formed in the support 11 of the stimulable phosphor sheet 10 have been scanned with the laser beam 24, it outputs a drive signal to the main scanning stepping motor 65, thereby returning the optical head 35 to its original position and outputs a drive signal to the sub-scanning pulse motor 61, thereby causing it to move the movable base plate 63 by one scanning line in the sub-scanning direction.

When the control unit 70 determines based on a detection signal indicating the position of the optical head 35 input from the linear encoder 67 that the optical head 35 has been returned to its original position and determines that the movable base plate 63 has been moved by one scanning line in the sub-scanning direction, similarly to the manner in which the stimulable phosphor layer regions 12 included in the first line of the stimulable phosphor layer regions 12 formed in the support 11 of the stimulable phosphor sheet 10 were sequentially irradiated with the laser beam 24 emitted from the first laser stimulating ray source 21, the stimulable phosphor layer regions 12 included in a second line of the stimulable phosphor layer regions 12 formed in the support 11 of the stimulable phosphor sheet 10 are sequentially irradiated with the laser beam 24 emitted from the first laser stimulating ray source 21, thereby exciting stimulable phosphor contained in the stimulable phosphor layer regions 12 included in the second line and stimulated emission 45 released from the stimulable phosphor layer regions 12 in the second line is sequentially and photoelectrically detected by the photomultiplier 50.

Analog data produced by photoelectrically detecting stimulated emission 45 with the photomultiplier 50 are converted by an A/D converter 53 into digital data, thereby producing biochemical analysis data from radiation data recorded in the stimulable phosphor layer regions 12 formed in the support 11 of the stimulable phosphor sheet 10.

When all of the stimulable phosphor layer regions 12 formed in the support 11 of the stimulable phosphor sheet 10 have been scanned with the laser beam 24 emitted from the first laser stimulating ray source 21 to excite stimulable phosphor contained in the stimulable phosphor layer regions 12 and biochemical analysis data produced from radiation data recorded in the stimulable phosphor layer regions 12 formed in the support 11 of the stimulable phosphor sheet 10 by photoelectrically detecting stimulated emission 45 released from the stimulable phosphor layer regions 12 with the photomultiplier 50 to produce analog data and digitizing the analog data by the A/D converter 53 have been forwarded to the data processing apparatus 54, the control unit 70 outputs a drive stop signal to the first laser stimulating ray source 21, thereby turning it off.

As described above, radiation data of the radioactive labeling substance recorded in a number of the stimulable phosphor layer regions 12 of the stimulable phosphor sheet 10 are read by the first scanner to produce biochemical analysis data.

On the other hand, when fluorescence data of a fluorescent substance recorded in a number of the absorptive regions 4 formed in the substrate 2 of the biochemical analysis unit 1 are to be read to produce biochemical analysis data, the biochemical analysis unit 1 is first set by the user on the glass plate 41 of the stage 40.

An instruction signal indicating that fluorescence data recorded in a number of the absorptive regions 4 formed in the substrate 2 of the biochemical analysis unit 1 are to be read is then input by the user through the keyboard 71 together with a labeling substance identifying signal for identifying the kind of a fluorescent substance such as a fluorescent dye labeling a substance derived from a living organism.

When the instruction signal and the labeling substance identifying signal are input by the user through the keyboard 71, the control unit 70 selects based on the instruction signal and the labeling substance identifying signal a laser stimulating ray source for emitting a laser beam 24 of a wavelength capable of efficiently stimulating the input fluorescent substance from among the first laser stimulating ray source 21, the second laser stimulating ray source 22 and the third laser stimulating ray source 23 and selects the filter member for cutting light having a wavelength of the laser beam 24 to be used for stimulating the input fluorescent substance and transmitting light having a longer wavelength than that of the laser beam to be used for stimulation from among the three filter members 51a, 51b and 51c.

Similarly to the case where radiation data recorded in a number of the stimulable phosphor layer regions 12 formed in the support 11 of the stimulable phosphor sheet 10 are read, all of the absorptive regions 4 formed in the substrate 2 of the biochemical analysis unit 1 are scanned by the laser beam 24, thereby stimulating a fluorescent substance contained in the absorptive regions 4, fluorescence emission 45 released from the fluorescent substance is photoelectrically detected by the photomultiplier 50 to produce analog data and the analog data are digitized by the A/D converter 53 to be forwarded to the data processing apparatus 54.

In this embodiment, since the absorptive regions 4 of the biochemical analysis unit 1 are formed by charging nylon-6 in the through-holes 3 formed in the substrate 2 made of stainless steel capable of attenuating light energy, it is possible to effectively prevent the laser beam 24 from scattering in each of the absorptive regions 4 and entering the neighboring absorptive regions 4 to excite a fluorescent substance contained in the neighboring absorptive regions 4.

In this manner, fluorescence data of the fluorescent substance are read to produce biochemical analysis data.

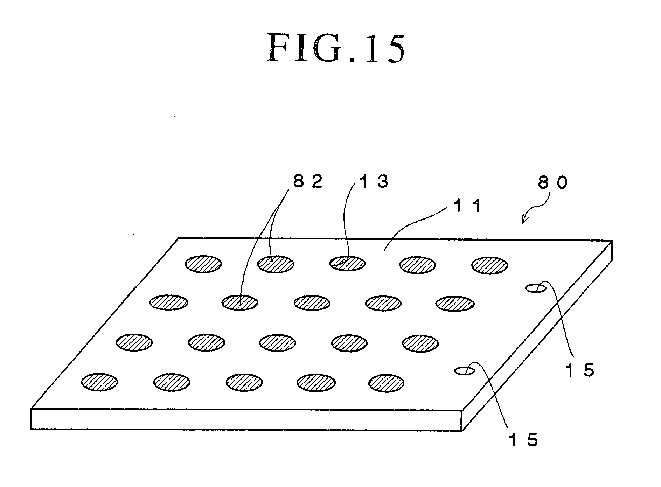

Figure 15 is a schematic perspective view showing a stimulable phosphor sheet onto which chemiluminescence data recorded in a number of the absorptive regions 4 formed in the substrate 2 of the biochemical analysis unit 1 are to be transferred, which is another preferred embodiment of the present invention.

A stimulable phosphor sheet 80 shown in Figure 15 has the same configuration as that of the stimulable phosphor sheet 10 shown in Figure 4 except that a number of stimulable phosphor layer regions 82 are formed by charging SrS system stimulable phosphor capable of absorbing and storing light energy in the through-holes 13 formed in the support 11 made of stainless steel.

As shown in Figure 15, similarly to the stimulable phosphor sheet 10 shown in Figure 4. the support 11 of the stimulable phosphor sheet 80 is formed with the two positioning through-holes 15.

Chemiluminescence data recorded in a number of the absorptive regions 4 formed in the substrate 2 of the biochemical analysis unit 1 are transferred onto a number of the stimulable phosphor layer regions 82 of the stimulable phosphor 80 shown in Figure 15.

When chemiluminescence data recorded in a number of the absorptive regions 4 formed in the substrate 2 of the biochemical analysis unit 1 are to be transferred onto a number of the stimulable phosphor layer regions 17 of the stimulable phosphor 15, a number of the absorptive regions 4 of the biochemical analysis unit 1 are first brought into contact with a chemiluminescent substrate.

As a result, chemiluminescence emission in a wavelength of visible light is selectively released from a number of the absorptive regions 4 of the biochemical analysis unit 1.

The biochemical analysis unit 1 releasing chemiluminescence emission is first set on the base plate 18 of the exposure device shown in Figure 5 in such a manner that the two positioning pins 19, 19 formed on the base plate 18 are inserted into the two positioning through-holes 5.

The stimulable phosphor sheet 80 is then set on the biochemical analysis unit 1 set on the base plate 18 of the exposure device in such a manner that the two positioning pins 19, 19 formed on the base plate 18 are inserted into the two positioning through-holes 15.

In this manner, since the biochemical analysis unit 1 and the stimulable phosphor sheet 80 are set on the base plate 18 of the exposure device in such a manner that the two positioning pins 19, 19 formed on the base plate 18 of the exposure device are inserted into the two positioning through-holes 5 and the two positioning through-holes 15, the biochemical analysis unit 1 and the stimulable phosphor sheet 80 can be positioned and superposed so that each of a number of the absorptive regions 4 formed in the substrate 2 of the biochemical analysis unit 1 faces one of a number of the stimulable phosphor layer regions 82 formed in the support 11 of the stimulable phosphor sheet 80 in the same pattern as that of a number of the absorptive regions 4, thereby exposing a number of the stimulable phosphor layer regions 82 formed in the support 11 of the stimulable phosphor sheet 80 to chemiluminescence emission selectively released from a number of the absorptive regions 4 formed in the substrate 2 of the biochemical analysis unit 1.

Thus, each of the stimulable phosphor layer regions 82 formed in the support 11 of the stimulable phosphor sheet 80 is kept to face the corresponding absorptive region 4 formed in the substrate 2 of the biochemical analysis unit 1 for a predetermined time period, whereby a number of the stimulable phosphor layer regions 82 formed in the support 11 of the stimulable phosphor sheet 80 are exposed to chemiluminescence emission selectively released from a number of the absorptive regions 4 formed in the substrate 2 of the biochemical analysis unit 1.

In this embodiment, since the biochemical analysis unit 1 is formed by charging nylon-6 in a number of the through-holes 3 formed in the substrate 2 made of stainless steel, the biochemical analysis unit 1 does not substantially stretch or shrink when it is subjected to liquid processing such as hybridization and, therefore, it is possible to superpose the stimulable phosphor sheet 80 on the biochemical analysis unit 1 so that each of the stimulable phosphor layer regions 82 formed in the support 11 of the stimulable phosphor sheet 80 accurately faces the corresponding absorptive region 4 formed in the substrate 2 of the biochemical analysis unit 1 by setting the biochemical analysis unit 1 and the stimulable phosphor sheet 80 on the base plate 18 of the exposure device in such a manner that the two positioning pins 19, 19 formed on the base plate 18 of the exposure device are inserted into the two positioning through-holes 5 and the two positioning through-holes 15, thereby exposing the stimulable phosphor layer regions 82.

During the exposure operation, since a number of the absorptive regions 4 of the biochemical analysis unit 1 are formed spaced apart from each other in the substrate 2 made of stainless steel and the substrate 2 made of stainless steel capable of attenuating light energy is present around each of the absorptive regions 4, chemiluminescence emission released from the absorptive regions 4 of the biochemical analysis unit 1 can be efficiently prevented from scattering in the substrate 2 of the biochemical analysis unit 1. Further, since a number of the stimulable phosphor layer regions 82 of the stimulable phosphor sheet 80 are formed by charging stimulable phosphor in a number of the through-holes 13 formed in the support 11 made of stainless steel capable of attenuating light energy and the support 11 made of stainless steel is present around each of the stimulable phosphor layer regions 82, chemiluminescence emission released from the absorptive regions 4 of the biochemical analysis unit 1 can be efficiently prevented from scattering in the support 11 of the stimulable phosphor sheet 80. Therefore, it is possible to cause all chemiluminescence emission released from the absorptive region 4 to enter the stimulable phosphor layer region 82 the absorptive region 4 faces and to effectively prevent chemiluminescence emission released from the absorptive region 4 from entering stimulable phosphor layer regions 82 to be exposed to chemiluminescence emission released from neighboring absorptive regions 4.

In this manner, a number of the stimulable phosphor layer regions 82 formed in the support 11 of the stimulable phosphor sheet 80 are selectively exposed to chemiluminescence emission released from the corresponding absorptive region 4 of the biochemical analysis unit 1.

In this manner, chemiluminescence data recorded in a number of the absorptive regions 4 of the biochemical analysis unit 1 are transferred onto and recorded in a number of the stimulable phosphor layer regions 82 formed in the support 11 of the stimulable phosphor sheet 80.

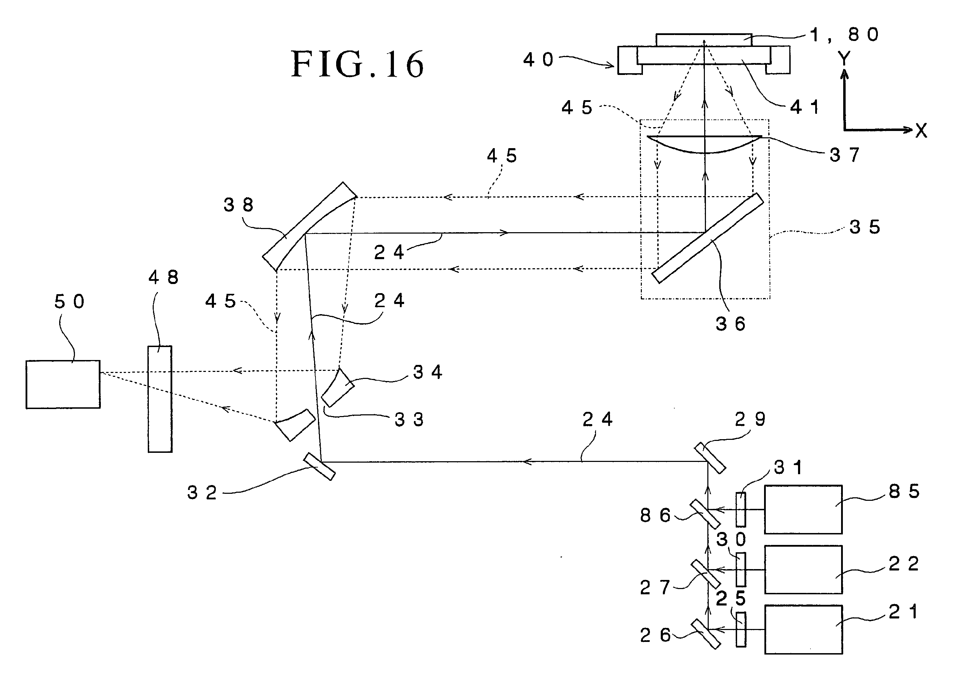

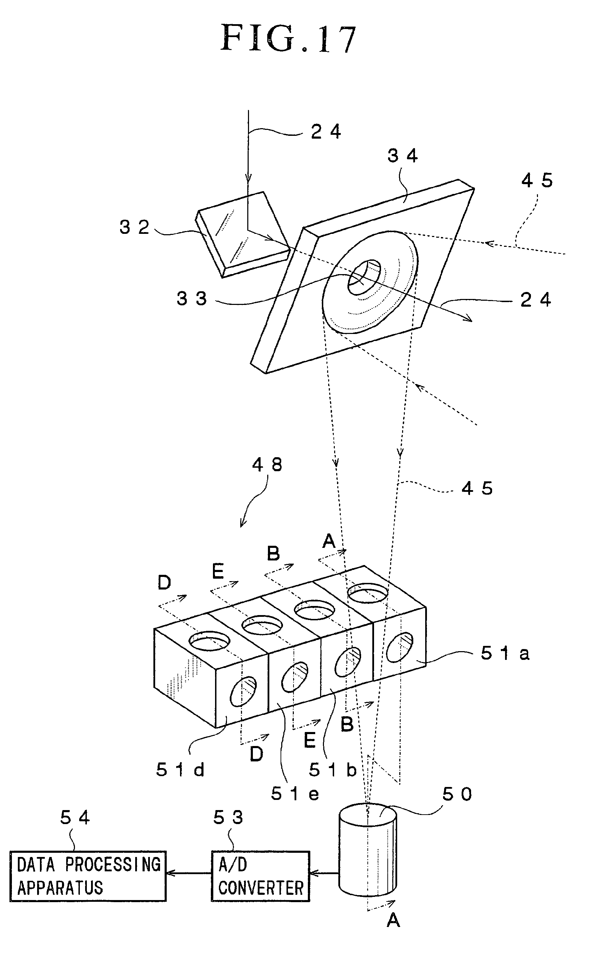

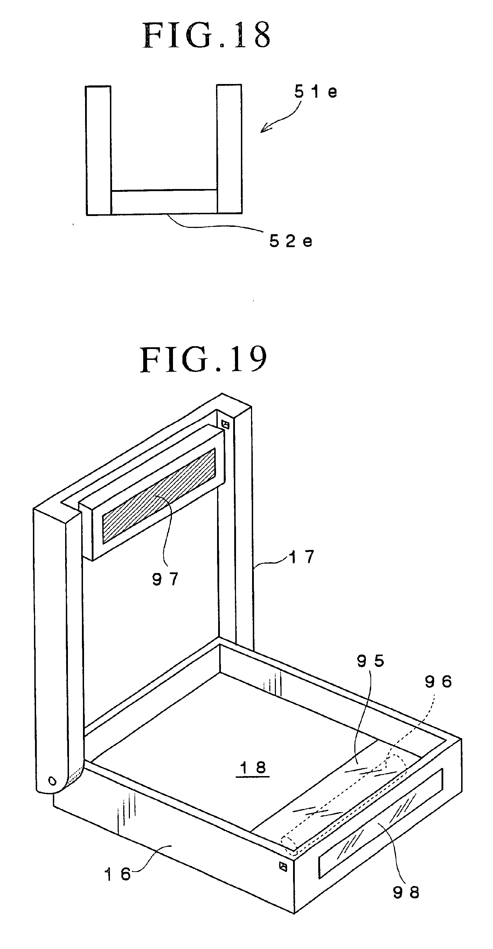

Figure 16 is a schematic perspective view showing a scanner for reading chemiluminescence data recorded in a number of the stimulable phosphor layer regions 82 formed in the support 11 of the stimulable phosphor sheet 80 and producing biochemical analysis data, Figure 17 is a schematic perspective view showing details in the vicinity of a photomultiplier of a scanner shown in Figure 16 and Figure 18 is a schematic cross-sectional view taken along a line E-E in Figure 17.

The scanner shown in Figures 16 to 18 has the same configuration as that of the scanner shown in Figures 7 to 14 except that it includes a fourth laser stimulating ray source 85 for emitting a laser beam 24 having a wavelength of 980 nm which can effectively stimulate SrS system stimulable phosphor instead of the third laser stimulating ray source 23 for emitting a laser beam 24 having a wavelength of 473 nm, includes a filter member 51e provided with a filter having a property of transmitting only light having a wavelength corresponding to that of stimulated emission emitted from stimulable phosphor and cutting off light having a wavelength of 980 nm, and includes a third dichroic mirror 86 for transmitting light having a wavelength equal to and shorter than 640 nm but reflecting light having a wavelength of 980 nm instead of the second dichroic mirror 28 for transmitting light having a wavelength equal to and longer than 532 nm but reflecting light having a wavelength of 473 nm.

The thus constituted scanner reads chemiluminescence data recorded in a number of the stimulable phosphor layer regions 82 of the stimulable phosphor sheet 80 and produces biochemical analysis data in the following manner.

A stimulable phosphor sheet 80 is first set on the glass plate 41 of the stage 40 by a user.

An instruction signal indicating that chemiluminescence data recorded in the stimulable phosphor layer regions 82 formed in the stimulable phosphor sheet 80 are to be read is then input through the keyboard 71.

The instruction signal input through the keyboard 71 is input to the control unit 70 and the control unit 70 outputs a drive signal to the filter unit motor 72 in accordance with the instruction signal, thereby moving the filter unit 48 so as to locate the filter member 51e provided with a filter 52e having a property of transmitting only light having a wavelength corresponding to that of stimulated emission emitted from the stimulable phosphor layer regions 82 and cutting off light having a wavelength of 980 nm in the optical path of stimulated emission 45.

The control unit 70 further outputs a drive signal to the main scanning stepping motor 65 to move the optical head 35 in the main scanning direction and when it determines based on a detection signal indicating the position of the optical head 35 input from the linear encoder 67 that the optical head 35 has reached a position where a laser beam 24 can be projected onto a first stimulable phosphor layer region 82 among a number of the stimulable phosphor layer regions 82 formed in the stimulable phosphor sheet 80, it outputs a drive stop signal to the main scanning stepping motor 65 and a drive signal to the fourth stimulating ray source 85, thereby actuating it to emit a laser beam 24 having a wavelength of 980 nm.

A laser beam 24 emitted from the fourth laser stimulating ray source 85 passes through a collimator lens 31, thereby being made a parallel beam, and is reflected by the third dichroic mirror 86, thereby changing its direction by 90 degrees. The laser beam 24 then advances to the mirror 29.

The laser beam 24 advancing to the mirror 29 is reflected by the mirror 29 and advances to the mirror 32 to be reflected thereby.

The laser beam 24 reflected by the mirror 32 passes through the hole 33 of the perforated mirror 34 and advances to the concave mirror 38.

The laser beam 24 advancing to the concave mirror 38 is reflected by the concave mirror 38 and enters the optical head 35.

The laser beam 24 entering the optical head 35 is reflected by the mirror 36 and condensed by the aspherical lens 37 onto the first stimulable phosphor layer region 82 of the stimulable phosphor sheet 80 placed on the glass plate 41 of a stage 40.

In this embodiment, since the stimulable phosphor layer regions 82 are formed by charging stimulable phosphor in a number of the through-holes 13 formed in the support 11 made of stainless steel, it is possible to effectively prevent the laser beam 24 from scattering in each of the stimulable phosphor layer regions 82 and entering the neighboring stimulable phosphor layer regions 82 to excite stimulable phosphor contained in the neighboring stimulable phosphor layer regions 82.

When the laser beam 24 impinges onto the first stimulable phosphor layer region 82 formed in the stimulable phosphor sheet 80, stimulable phosphor contained in the first stimulable phosphor layer region 82 formed in the stimulable phosphor sheet 80 is excited by the laser beam 24, thereby releasing stimulated emission 45 from the first stimulable phosphor layer region 82.

The stimulated emission 45 released from the first stimulable phosphor layer region 82 of the stimulable phosphor sheet 80 is condensed onto the mirror 36 by the aspherical lens 37 provided in the optical head 35 and reflected by the mirror 36 on the side of the optical path of the laser beam 24, thereby being made a parallel beam to advance to the concave mirror 38.

The stimulated emission 45 advancing to the concave mirror 38 is reflected by the concave mirror 38 and advances to the perforated mirror 34.

As shown in Figure 17, the stimulated emission 45 advancing to the perforated mirror 34 is reflected downward by the perforated mirror 34 formed as a concave mirror and advances to the filter 52e of the filter unit 48.

Since the filter 52e has a property of transmitting only light having a wavelength corresponding to that of stimulated emission emitted from stimulable phosphor and cutting off light having a wavelength of 980 nm, light having a wavelength of 980 nm corresponding to that of the stimulating ray is cut off by the filter 52e and only light having a wavelength corresponding to that of stimulated emission passes through the filter 52e to be photoelectrically detected by the photomultiplier 50.

Analog data produced by photoelectrically detecting stimulated emission 45 with the photomultiplier 50 are converted by an A/D converter 53 into digital data and the digital data are fed to a data processing apparatus 54.

When a predetermined time has passed after the fourth stimulating ray source 85 was turned on, the control unit 70 outputs a drive stop signal to the fourth stimulating ray source 85, thereby turning it off and outputs a drive signal to the main scanning stepping motor 65, thereby moving the optical head 35 by one pitch equal to the distance between neighboring stimulable phosphor layer regions 82 of the stimulable phosphor sheet 80.

When the control unit 70 determines based on a detection signal indicating the position of the optical head 35 input from the linear encoder 67 that the optical head 35 has been moved by one pitch equal to the distance between neighboring stimulable phosphor layer regions 82, it outputs a drive signal to the fourth stimulating ray source 85 to turn it on, thereby causing the laser beam 24 to excite stimulable phosphor contained in a second stimulable phosphor layer region 82 formed in the support 11 of the stimulable phosphor sheet 80 next to the first stimulable phosphor layer region 82.

Similarly to the above, the second stimulable phosphor layer region 82 formed in the stimulable phosphor sheet 80 is irradiated with the laser beam 24 for a predetermined time and when stimulated emission 45 released from the second stimulable phosphor layer region 82 is photoelectrically detected by the photomultiplier 50, thereby producing biochemical analysis data of the second stimulable phosphor layer region 82, the control unit 70 outputs a drive stop signal to the fourth stimulating ray source 85, thereby turning it off and outputs a drive signal to the main scanning stepping motor 65, thereby moving the optical head 35 by one pitch equal to the distance between neighboring stimulable phosphor layer regions 17.

In this manner, the on and off operation of the fourth stimulating ray source 85 is repeated in synchronism with the intermittent movement of the optical head 35 and when the control unit 70 determines based on a detection signal indicating the position of the optical head 35 input from the linear encoder 67 that the optical head 35 has been moved by one scanning line in the main scanning direction and that the stimulable phosphor layer regions 82 included in a first line of the stimulable phosphor layer regions 82 formed in the stimulable phosphor sheet 80 have been scanned with the laser beam 24, it outputs a drive signal to the main scanning stepping motor 65, thereby returning the optical head 35 to its original position and outputs a drive signal to the sub-scanning pulse motor 61, thereby causing it to move the movable base plate 63 by one scanning line in the sub-scanning direction.

When the control unit 70 determines based on a detection signal indicating the position of the optical head 35 input from the linear encoder 67 that the optical head 35 has been returned to its original position and determines that the movable base plate 63 has been moved by one scanning line in the sub-scanning direction, similarly to the manner in which the stimulable phosphor layer regions 82 included in the first line of the stimulable phosphor layer regions 82 formed in the stimulable phosphor sheet 80 were sequentially irradiated with the laser beam 24 emitted from the fourth laser stimulating ray source 85, the stimulable phosphor layer regions 82 included in a second line of the stimulable phosphor layer regions 82 formed in the stimulable phosphor sheet 80 are sequentially irradiated with the laser beam 24 emitted from the fourth laser stimulating ray source 85, thereby exciting stimulable phosphor contained in the stimulable phosphor layer regions 82 included in the second line and stimulated emission 45 released from the stimulable phosphor layer regions 82 is sequentially and photoelectrically detected by the photomultiplier 50.

Analog data produced by photoelectrically detecting stimulated emission 45 with the photomultiplier 50 are converted by the A/D converter 53 into digital data and the digital data are fed to the data processing apparatus 54.

When all of the stimulable phosphor layer regions 82 formed in the support 11 of the stimulable phosphor sheet 80 have been scanned with the laser beam 24 released from the fourth laser stimulating ray source 85 to excite stimulable phosphor contained in the stimulable phosphor layer regions 82 and biochemical analysis data produced from chemiluminescence data recorded in the stimulable phosphor layer regions 82 formed in the support 11 of the stimulable phosphor sheet 80 by photoelectrically detecting stimulated emission 45 released from the stimulable phosphor layer regions 82 with the photomultiplier 50 to produce analog data and digitizing the analog data by the A/D converter 53 have been forwarded to the data processing apparatus 54, the control unit 70 outputs a drive stop signal to the fourth laser stimulating ray source 85, thereby turning it off.

As described above, chemiluminescence data recorded in a number of the stimulable phosphor layer regions 82 of the stimulable phosphor sheet 80 are read by the scanner to produce biochemical analysis data.

According to the embodiments shown in Figures 1 to 18, when a number of the stimulable phosphor layer regions 12 formed in the support 11 of the stimulable phosphor sheet 10 are exposed to a radioactive labeling substance selectively contained in a number of the absorptive regions 4 formed in the substrate 2 of the biochemical analysis unit 1, since a number of the absorptive regions 4 of the biochemical analysis unit 1 are formed by charging nylon-6 in a number of the through-holes 3 formed in the substrate 2 made of stainless steel and the substrate 2 made of stainless steel capable of attenuating radiation energy is present around each of the absorptive regions 4, electron beams (β rays) released from the radioactive labeling substance contained in a particular absorptive regions 4 can be efficiently prevented from scattering in the substrate 2 of the biochemical analysis unit 1 and entering stimulable phosphor layer regions 12 next to the corresponding stimulable phosphor layer region 12 of the stimulable phosphor sheet 10. Further, since a number of the stimulable phosphor layer regions 12 of the stimulable phosphor sheet 10 are formed by charging stimulable phosphor in a number of the through-holes 13 formed in the support 11 made of stainless steel and the support 11 made of stainless steel capable of attenuating radiation energy is present around each of the stimulable phosphor layer region 12, electron beams (β rays) released from the radioactive labeling substance contained in a particular absorptive regions 4 can be efficiently prevented from scattering in the support 11 of the stimulable phosphor sheet 10 and entering stimulable phosphor layer regions 12 next to the corresponding stimulable phosphor layer region 12 of the stimulable phosphor sheet 10. Therefore, it is possible to effectively prevent noise caused by the exposure of the stimulable phosphor layer region 12 to be exposed to electron beams (β rays) released from the radioactive labeling substance contained in a particular absorptive regions 4 to electron beams (β rays) released from the radioactive labeling substance contained in the neighboring absorptive regions 4 from being generated in biochemical analysis data and to produce biochemical analysis data having an excellent quantitative characteristic with high resolution.

Furthermore, according to the embodiments shown in Figures 1 to 18, when a number of the stimulable phosphor layer regions 12 formed in the support 11 of the stimulable phosphor sheet 10 are to be exposed to a radioactive labeling substance selectively contained in a number of the absorptive regions 4 formed in the substrate 2 of the biochemical analysis unit 1, since the biochemical analysis unit 1 and the stimulable phosphor sheet 10 are set on the base plate 18 of the exposure device in such a manner that the two positioning pins 19, 19 formed on the base plate 18 of the exposure device are inserted into the two positioning through-holes 5 of the biochemical analysis unit 1 and the two positioning through-holes 15 of the stimulable phosphor sheet 10, the biochemical analysis unit 1 and the stimulable phosphor sheet 10 can be positioned and superposed so that each of a number of the absorptive regions 4 formed in the substrate 2 of the biochemical analysis unit 1 accurately faces the corresponding stimulable phosphor layer region 12 among a number of the stimulable phosphor layer regions 12 formed in the support 11 of the stimulable phosphor sheet 10 in the same pattern as that of a number of the absorptive regions 4 formed in the substrate 2 of the biochemical analysis unit 1, thereby exposing a number of the stimulable phosphor layer regions 12 formed in the support 11 of the stimulable phosphor sheet 10 to a radioactive labeling substance selectively contained in a number of the absorptive regions 4 formed in the substrate 2 of the biochemical analysis unit 1 in a desired manner and, therefore, it is possible to produce biochemical analysis data having an excellent quantitative characteristic with high resolution by scanning a number of the stimulable phosphor layer regions 12 exposed to a radioactive labeling substance with the laser beam 24 and photoelectrically detecting stimulated emission 45 released from a number of the stimulable phosphor layer regions 12.

Moreover, according to the embodiments shown in Figures 1 to 18, when a number of the stimulable phosphor layer regions 82 formed in the support 11 of the stimulable phosphor sheet 80 are exposed to chemiluminescence emission selectively released from a number of the absorptive regions 4 formed in the substrate 2 of the biochemical analysis unit 1, since a number of the absorptive regions 4 of the biochemical analysis unit 1 are formed by charging nylon-6 in a number of the through-holes 3 formed in the substrate 2 made of stainless steel and the substrate 2 made of stainless steel capable of attenuating light energy is present around each of the absorptive regions 4, chemiluminescence emission selectively released from a number of the absorptive regions 4 formed in the substrate 2 of the biochemical analysis unit 1 can be efficiently prevented from scattering in the substrate 2 of the biochemical analysis unit 1 and entering stimulable phosphor layer regions 82 next to the corresponding stimulable phosphor layer region 82 of the stimulable phosphor sheet 80. Further, since a number of the stimulable phosphor layer regions 82 of the stimulable phosphor sheet 80 are formed by charging stimulable phosphor in a number of the through-holes 13 formed in the support 11 made of stainless steel and the support 11 made of stainless steel capable of attenuating radiation energy is present around each of the stimulable phosphor layer region 82, chemiluminescence emission selectively released from a number of the absorptive regions 4 formed in the substrate 2 of the biochemical analysis unit 1 can be efficiently prevented from scattering in the support 11 of the stimulable phosphor sheet 80 and entering stimulable phosphor layer regions 82 next to the corresponding stimulable phosphor layer region 82 of the stimulable phosphor sheet 80. Therefore, it is possible to effectively prevent noise caused by the exposure of the stimulable phosphor layer region 82 to be exposed to chemiluminescence emission released from a particular absorptive regions 4 to chemiluminescence emission released from neighboring absorptive regions 4 from being generated in biochemical analysis data and to produce biochemical analysis data having an excellent quantitative characteristic with high resolution.

Furthermore, according to the embodiments shown in Figures 1 to 18, when a number of the stimulable phosphor layer regions 82 formed in the support 11 of the stimulable phosphor sheet 80 are to be exposed to chemiluminescence emission selectively released from a number of the absorptive regions 4 formed in the substrate 2 of the biochemical analysis unit 1, since the biochemical analysis unit 1 and the stimulable phosphor sheet 80 are set on the base plate 18 of the exposure device in such a manner that the two positioning pins 19, 19 formed on the base plate 18 of the exposure device are inserted into the two positioning through-holes 5 of the biochemical analysis unit 1 and the two positioning through-holes 15 of the stimulable phosphor sheet 80, the biochemical analysis unit 1 and the stimulable phosphor sheet 80 can be positioned and superposed so that each of a number of the absorptive regions 4 formed in the substrate 2 of the biochemical analysis unit 1 accurately faces the corresponding stimulable phosphor layer region 82 among a number of the stimulable phosphor layer regions 82 formed in the support 11 of the stimulable phosphor sheet 80 in the same pattern as that of a number of the absorptive regions 4 formed in the substrate 2 of the biochemical analysis unit 1, thereby exposing a number of the stimulable phosphor layer regions 82 formed in the support 11 of the stimulable phosphor sheet 80 to chemiluminescence emission selectively released from a number of the absorptive regions 4 formed in the substrate 2 of the biochemical analysis unit 1 in a desired manner and, therefore, it is possible to produce biochemical analysis data having an excellent quantitative characteristic with high resolution by scanning a number of the stimulable phosphor layer regions 82 exposed to chemiluminescence emission with the laser beam 24 and photoelectrically detecting stimulated emission 45 released from a number of the stimulable phosphor layer regions 82.

Figure 19 is a schematic perspective view showing an exposure device used for a method for exposing a stimulable phosphor sheet which a further preferred embodiment of the present invention.

As shown in Figure 19, in an exposure device for exposing a stimulable phosphor sheet according to this embodiment, a part of the base plate 18 is formed of a transparent glass plate 95 and a fluorescent lamp 96 is provided in the case 16 below the transparent glass plate 95.

On the other hand, the lid member 17 is formed with a photo-sensor 97 so as to face the fluorescent lamp 96 when the lid member 17 is closed and a display panel 98 is formed on the case 16.

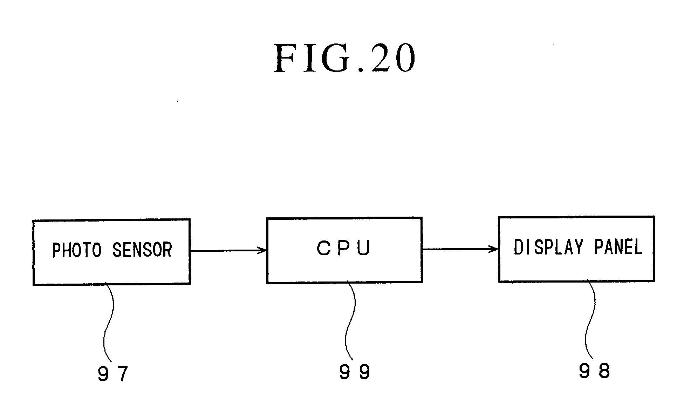

Figure 20 is a block diagram of a control system, a detection system and a display system of the exposure device shown in Figure 19.

As shown in Figure 20, the control system, the detection system and the display system of the exposure device for exposing a stimulable phosphor sheet according to this embodiment includes a CPU 99 for controlling the overall operation of the exposure device, the photo-sensor 97 and the display panel 98.

When a number of the stimulable phosphor layer regions 12 formed in the stimulable phosphor sheet 10 are to be exposed to a radioactive labeling substance selectively contained in a number of the absorptive regions 4 formed in the biochemical analysis unit 1 to transfer radiation data recorded in a number of the biochemical analysis unit 1 onto a number of the stimulable phosphor layer regions 12 of the stimulable phosphor sheet 10, one of the biochemical analysis unit 1 and the stimulable phosphor sheet 10 is first set on the base plate 18 of the exposure device in such a manner that the two positioning through-holes 5 of the biochemical analysis unit 1 or the two positioning through-holes 15 of the stimulable phosphor sheet 10 are located on the transparent glass plate 95.

The other one of the stimulable phosphor sheet 10 and the biochemical analysis unit 1 is then superposed on the one of the biochemical analysis unit 1 and the stimulable phosphor sheet 10 so that the two positioning through-holes 15 of the stimulable phosphor sheet 10 or the two positioning through-holes 5 of the biochemical analysis unit 1 substantially align with the two positioning through-holes 5 of the biochemical analysis unit 1 or the two positioning through-holes 15 of the stimulable phosphor sheet 10. The lid member 17 is then closed and the fluorescent lamp 96 is turned on.

When the two positioning through-holes 5 of the biochemical analysis unit 1 and the two positioning through-holes 15 of the stimulable phosphor sheet 10 do not overlap with each other at all, since light emitted from the fluorescent lamp 96 is blocked by the biochemical analysis unit 1 and the stimulable phosphor sheet 10, the photo-sensor 97 does not detect light emitted from the fluorescent lamp 96 and, therefore, the photo-sensor 97 does not output any detection signal.

When the CPU 99 does not receive any detection signal input from the photo-sensor 97 even though the lid member 17 has been closed, it causes the display panel 98 to display a message reporting that the biochemical analysis unit 1 and the stimulable phosphor sheet 10 are not accurately superposed.

To the contrary, when even a part of the two positioning through-holes 5 of the biochemical analysis unit 1 and a part the two positioning through-holes 15 of the stimulable phosphor sheet 10 overlap each other, light emitted from the fluorescent lamp 96 is transmitted through the two positioning through-holes 5 of the biochemical analysis unit 1 and the two positioning through-holes 15 of the stimulable phosphor sheet 10 and detected by the photo-sensor 97 and a detection signal is output from the photo-sensor 97 to the CPU 99.

Since the two positioning through-holes 5 are formed in the substrate 2 of the biochemical analysis unit 1 and the two positioning through-holes 15 are formed in the support 11 of the stimulable phosphor sheet 10 in such a manner that when the biochemical analysis unit 1 and the stimulable phosphor sheet 10 are superposed so that each of a number of the absorptive regions 4 formed in the substrate 2 of the biochemical analysis unit 1 accurately faces one of a number of the stimulable phosphor layer regions 12 formed in the support 11 of the stimulable phosphor sheet 10 in the same pattern as that of a number of the absorptive regions 4, the two positioning through-holes 5 of the biochemical analysis unit 1 and the two positioning through-holes 15 of the stimulable phosphor sheet 10 completely align with each other and the amount of light to be detected by the photo-sensor 97 becomes maximum, the CPU 99 compares the signal intensity of the detection signal input from the photo-sensor 97 with a threshold value determined in advance and stored in a memory (not shown).

As a result, when the CPU 99 judges that the signal intensity of the detection signal input from the photo-sensor 97 is lower than the threshold value, it causes the display panel 98 to display a message reporting that the biochemical analysis unit 1 and the stimulable phosphor sheet 10 are not accurately superposed.

To the contrary, when the CPU 99 judges that the signal intensity of the detection signal input from the photo-sensor 97 is equal to or higher than the threshold value, it causes the display panel 98 to display a message reporting that the biochemical analysis unit 1 and the stimulable phosphor sheet 10 are accurately superposed.

In this manner, the position of the other one of the stimulable phosphor sheet 10 and the biochemical analysis unit 1 is adjusted by the user so that the signal intensity of the detection signal input from the photo-sensor 97 to the CPU 99 is equal to or higher than the threshold value and, then, each of the stimulable phosphor layer regions 12 formed in the support 11 of the stimulable phosphor sheet 10 is kept to face the corresponding absorptive region 4 formed in the substrate 2 of the biochemical analysis unit 1 for a predetermined time period, whereby a number of the stimulable phosphor layer regions 12 formed in the support 11 of the stimulable phosphor sheet 10 are exposed to the radioactive labeling substance contained in a number of the absorptive regions 4 formed in the substrate 2 of the biochemical analysis unit 1.

In this manner, radiation data recorded in a number of the absorptive regions 4 of the biochemical analysis unit 1 are transferred onto and recorded in a number of the stimulable phosphor layer regions 12 of the stimulable phosphor sheet 10.

On the other hand, when chemiluminescence data recorded in a number of the absorptive regions 4 of the biochemical analysis unit 1 are to be transferred onto a number of the stimulable phosphor layer regions 82 of the stimulable phosphor sheet 80, a number of the absorptive regions 4 of the biochemical analysis unit 1 are first brought into contact with a chemiluminescent substrate.

As a result, chemiluminescence emission in a wavelength of visible light is selectively released from a number of the absorptive regions 4 of the biochemical analysis unit 1.

Then, one of the biochemical analysis unit 1 releasing chemiluminescence emission and the stimulable phosphor sheet 80 is set on the base plate 18 of the exposure device in such a manner that the two positioning through-holes 5 of the biochemical analysis unit 1 or the two positioning through-holes 15 of the stimulable phosphor sheet 10 are located on the transparent glass plate 95.

The other one of the stimulable phosphor sheet 80 and the biochemical analysis unit 1 is further superposed on the one of the biochemical analysis unit 1 and the stimulable phosphor sheet 80 so that the two positioning through-holes 15 of the stimulable phosphor sheet 80 or the two positioning through-holes 5 of the biochemical analysis unit 1 substantially align with the two positioning through-holes 5 of the biochemical analysis unit 1 or the two positioning through-holes 15 of the stimulable phosphor sheet 80. The lid member 17 is then closed and the fluorescent lamp 96 is turned on.

Similarly to the above, the position of the other one of the stimulable phosphor sheet 80 and the biochemical analysis unit 1 is adjusted by the user so that the signal intensity of the detection signal output from the photo-sensor 97 to the CPU 99 is equal to or higher than the threshold value and, then, each of the stimulable phosphor layer regions 82 formed in the support 11 of the stimulable phosphor sheet 80 is kept to face the corresponding absorptive region 4 formed in the substrate 2 of the biochemical analysis unit 1 for a predetermined time period, whereby a number of the stimulable phosphor layer regions 82 formed in the support 11 of the stimulable phosphor sheet 80 are exposed to chemiluminescence emission selectively released from a number of the absorptive regions 4 formed in the substrate 2 of the biochemical analysis unit 1.

According to this embodiment, since the CPU 99 of exposure device is constituted so as to automatically judge based on the amount of light emitted from the fluorescent lamp 96 and detected by the photo-sensor 97 that the positioning through-holes 5 of the biochemical analysis unit 1 and the positioning through-holes 15 of the stimulable phosphor sheet 10 completely have aligned with each other and the biochemical analysis unit 1 and the stimulable phosphor sheet 10 have been superposed so that each of a number of the absorptive regions 4 formed in the substrate 2 of the biochemical analysis unit 1 accurately faces one of a number of the stimulable phosphor layer regions 12 formed in the support 11 of the stimulable phosphor sheet 10 in the same pattern as that of a number of the absorptive regions 4 and display a message indicating this fact on the display panel 98, it is possible to position and superpose the biochemical analysis unit 1 and the stimulable phosphor sheet 10 so that each of a number of the absorptive regions 4 formed in the substrate 2 of the biochemical analysis unit 1 accurately faces one of a number of the stimulable phosphor layer regions 12 formed in the support 11 of the stimulable phosphor sheet 10 in the same pattern as that of a number of the absorptive regions 4 and to expose a number of the stimulable phosphor layer regions 12 formed in the support 11 of the stimulable phosphor sheet 10 to a radioactive labeling substance selectively contained in a number of the absorptive regions 4 formed in the substrate 2 of the biochemical analysis unit 1 in a desired manner. Therefore, it is possible to produce biochemical analysis data having an excellent quantitative characteristic with high resolution by scanning a number of the stimulable phosphor layer regions 12 exposed to a radioactive labeling substance with the laser beam 24 and photoelectrically detecting stimulated emission 45 released from a number of the stimulable phosphor layer regions 12.

Furthermore, according to this embodiment, since the CPU 99 of exposure device is constituted so as to automatically judge based on the amount of light emitted from the fluorescent lamp 96 and detected by the photo-sensor 97 that the positioning through-holes 5 of the biochemical analysis unit 1 and the positioning through-holes 15 of the stimulable phosphor sheet 80 completely have aligned with each other and the biochemical analysis unit 1 and the stimulable phosphor sheet 80 have been superposed so that each of a number of the absorptive regions 4 formed in the substrate 2 of the biochemical analysis unit 1 accurately faces one of a number of the stimulable phosphor layer regions 82 formed in the support 11 of the stimulable phosphor sheet 80 in the same pattern as that of a number of the absorptive regions 4 and display a message indicating this fact on the display panel 98, it is possible to position and superpose the biochemical analysis unit 1 and the stimulable phosphor sheet 80 so that each of a number of the absorptive regions 4 formed in the substrate 2 of the biochemical analysis unit 1 accurately faces one of a number of the stimulable phosphor layer regions 82 formed in the support 11 of the stimulable phosphor sheet 80 in the same pattern as that of a number of the absorptive regions 4 and to expose a number of the stimulable phosphor layer regions 82 formed in the support 11 of the stimulable phosphor sheet 80 to chemiluminescence emission selectively released from a number of the absorptive regions 4 formed in the substrate 2 of the biochemical analysis unit 1 in a desired manner. Therefore, it is possible to produce biochemical analysis data having an excellent quantitative characteristic with high resolution by scanning a number of the stimulable phosphor layer regions 82 exposed to chemiluminescence emission with the laser beam 24 and photoelectrically detecting stimulated emission 45 released from a number of the stimulable phosphor layer regions 82.

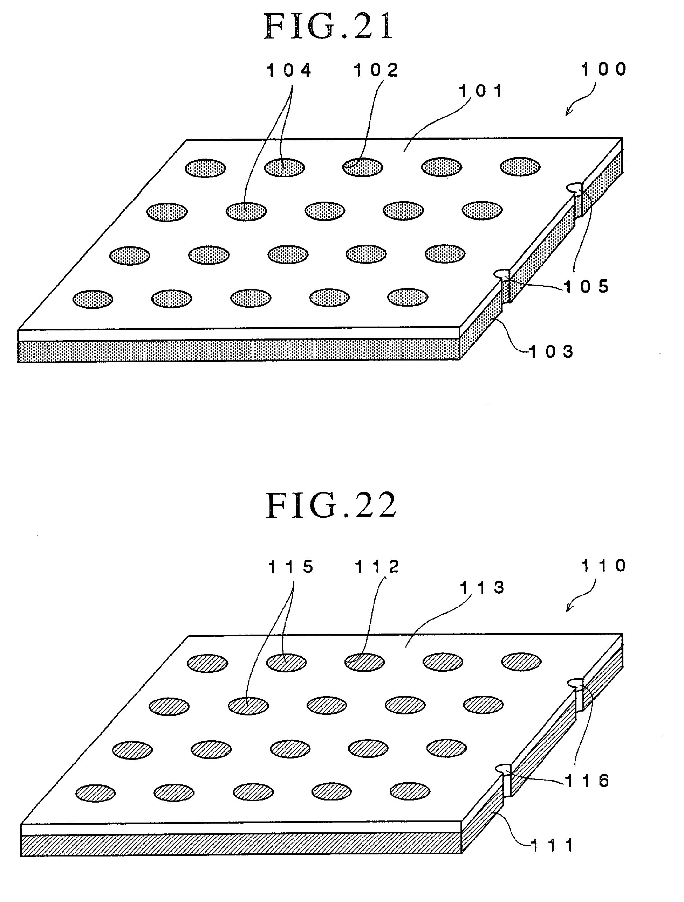

Figure 21 is a schematic perspective view showing a biochemical analysis unit which a further preferred embodiment of the present invention.

As shown in Figure 21, a biochemical analysis unit 100 according to this embodiment includes a substrate 101 made of stainless steel and regularly formed with a number of substantially circular through-holes 102 and a number of absorptive regions 104 are regularly formed by pressing an absorptive membrane 103 formed on nylon-6 into a number of the through-holes 102 formed in the substrate 101 using a calender processing apparatus (not shown).

Although not accurately shown in Figure 21, in this embodiment, about 10,000 substantially circular absorptive regions 104 having a size of about 0.01 mm² are regularly formed at a density of about 5,000 per cm² in the biochemical analysis unit 100.

In this embodiment, the biochemical analysis unit 100 is formed by pressing the absorptive membrane 103 into a number of the through-holes 102 formed in the substrate 101 in such a manner that the surfaces of the absorptive regions 104 and the surface of the substrate 101 lie at the same height level.

As shown in Figure 21, in this embodiment, one side portion of the substrate 101 of the biochemical analysis unit 100 is formed with two semi-circular positioning notches 105.

In this embodiment, similarly to the biochemical analysis unit 1 shown in Figure 1, a solution containing specific binding substances such as cDNAs is spotted using the spotting device shown in Figure 2 onto a number of the absorptive regions 104 formed in the biochemical analysis unit 100 and the specific binding substances are absorbed in a number of the absorptive regions 104.

Since a number of the absorptive regions 104 of the biochemical analysis unit 100 shown in Figure 21 are formed by pressing the absorptive membrane 103 into a number of the through-holes 102 formed in the substrate 101, cavities in the absorptive membrane 103 have been eliminated by the pressing operation in regions between neighboring absorptive regions 104. Therefore, a solution of specific binding substances spotted in the absorptive regions 104 can be effectively prevented from permeating the absorptive membrane 103 and the specific binding substances are absorbed only in the absorptive regions 104.

Further, as shown in Figure 3, the biochemical analysis unit 100 is set in the hybridization reaction vessel 8 accommodating a hybridization reaction solution 9 containing a substance derived from a living organism and labeled with a radioactive labeling substance, a substance derived from a living organism and labeled with a labeling substance which generates chemiluminescence emission when it contacts a chemiluminescent substrate and a substance derived from a living organism and labeled with a fluorescent substance such as a fluorescent dye and specific binding substances absorbed in a number of the absorptive regions 104 of the biochemical analysis unit 100 are selectively hybridized with a substance derived from a living organism, labeled with a radioactive labeling substance and contained in the hybridization reaction solution 9, a substance derived from a living organism, labeled with a labeling substance which generates chemiluminescence emission when it contacts a chemiluminescent substrate and contained in the hybridization reaction solution 9 and a substance derived from a living organism, labeled with a fluorescent substance such as a fluorescent dye and contained in the hybridization reaction solution 9.

Thus, radiation data, chemiluminescence data and fluorescence data are recorded in a number of the absorptive regions 104 formed in the biochemical analysis unit 100.

Similarly to the previous embodiments, fluorescence data recorded in a number of the absorptive regions 104 of the biochemical analysis unit 100 are read by the scanner shown in Figures 7 to 14 to produce biochemical analysis data.

On the other hand, radiation data recorded in a number of the absorptive regions 104 of the biochemical analysis unit 100 are transferred onto a stimulable phosphor sheet and chemiluminescence data recorded in a number of the absorptive regions 104 of the biochemical analysis unit 100 are transferred onto another stimulable phosphor sheet.

Figure 22 is a schematic perspective view showing a stimulable phosphor sheet onto which radiation data are to be transferred, which is a further preferred embodiment of the present invention.

As shown in Figure 22, a stimulable phosphor sheet 110 according to this embodiment includes a stimulable phosphor membrane 111 containing BaFX system stimulable phosphor (where X is at least one halogen atom selected from the group consisting of Cl, Br and I) capable of absorbing and storing radiation energy and a binder and a support 113 made of stainless steel and regularly formed with a number of through-holes 112, and the stimulable phosphor membrane 111 is pressed into a number of the through-holes 112 formed in the support 113 using a calender processing apparatus (not shown), thereby dot-like forming a number of stimulable phosphor layer regions 115 at positions corresponding to those of a number of the through-holes 112 of the support 113.

A number of the through-holes 112 are formed in the support 113 in the same pattern as that of a number of the absorptive regions 104 formed in the biochemical analysis unit 100.

Therefore, although not accurately shown in Figure 22, in this embodiment, about 10,000 substantially circular stimulable phosphor layer regions 115 having a size of about 0.01 mm² are dot-like formed in a regular pattern at a density of about 5,000 per cm² in the support 113 of the stimulable phosphor sheet 110 in the same pattern as that of a number of the absorptive regions 104 formed in the biochemical analysis unit 100.

As shown in Figure 22, one side portion of the support 113 of the stimulable phosphor sheet 110 corresponding to the one end portion of the substrate 101 of the biochemical analysis unit 100 formed with the positioning notches 105, 105 is formed with two semi-circular positioning notches 116, 116 at positions corresponding to those of the positioning notches 105, 105 formed at the one side portion of the substrate 101 of the biochemical analysis unit 100. Each of the semi-circular positioning notches 116, 116 has the same size as that of the positioning notch 105, 105 formed in the substrate 101 of the biochemical analysis unit 100.

In this embodiment, the stimulable phosphor sheet 110 is formed by pressing the stimulable phosphor membrane 111 into a number of the through-holes 112 formed in the support 113 in such a manner that the surface of the support 113 and the surfaces of a number of the stimulable phosphor layer regions 115 lie at the same height level.

In this embodiment, a number of the stimulable phosphor layer regions 115 formed in the stimulable phosphor sheet 110 are exposed to a radioactive labeling substance selectively contained in a number of the absorptive regions 104 formed in the biochemical analysis unit 100 using the exposure device shown in Figure 5, thereby transferring radiation data recorded in a number of the absorptive regions 104 formed in the biochemical analysis unit 100 onto a number of the stimulable phosphor layer regions 115 formed in the stimulable phosphor sheet 110.

Each of the two positioning notches 105, 105 of the biochemical analysis unit 100 and each of the two positioning notches 116, 116 of the stimulable phosphor sheet 110 have inner diameters slightly larger than the outer diameter of each of the two erect positioning pins 19, 19 formed on the base plate 18 of the exposure device. Therefore, it is possible to accurately position the biochemical analysis unit 100 and the stimulable phosphor sheet 110 so that each of a number of the stimulable phosphor layer regions 115 formed in the stimulable phosphor sheet 110 faces the corresponding absorptive region 104 formed in the biochemical analysis unit 100 by superposing the biochemical analysis unit 100 and the stimulable phosphor sheet 110 in such a manner that the two erect positioning pins 19, 19 formed on the base plate 18 of the exposure device are fitted into the two positioning notches 105, 105 of the biochemical analysis unit 100 and the two positioning notches 116, 116 of the stimulable phosphor sheet 110.

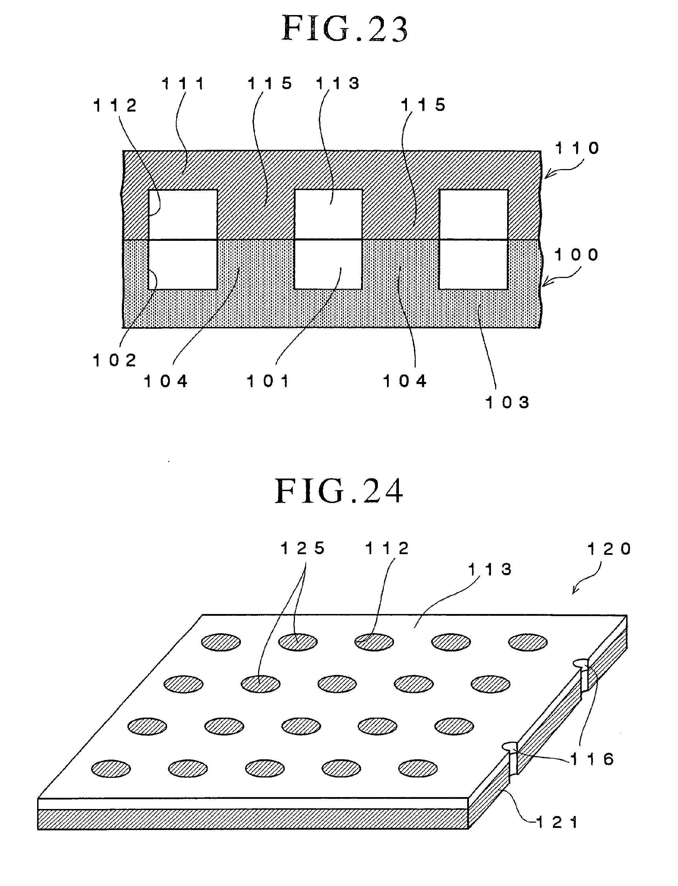

Figure 23 is a schematic cross sectional view showing a method for exposing a number of the stimulable phosphor layer regions 115 formed in the stimulable phosphor sheet 110 to a radioactive labeling substance selectively contained in the corresponding absorptive region 104 formed in the biochemical analysis unit 100.

In this embodiment, since the biochemical analysis unit 100 is formed by pressing the absorptive membrane 103 formed of nylon-6 into a number of the through-holes 102 formed in the substrate 101 made of stainless steel, the biochemical analysis unit 100 does not substantially stretch or shrink when it is subjected to liquid processing such as hybridization and, therefore, it is possible to superpose the stimulable phosphor sheet 110 on the biochemical analysis unit 100 so that each of the stimulable phosphor layer regions 115 formed in stimulable phosphor sheet 110 accurately faces the corresponding absorptive region 104 formed in the biochemical analysis unit 100 by setting the biochemical analysis unit 100 and the stimulable phosphor sheet 110 on the base plate 18 of the exposure device in such a manner that the two positioning pins 19, 19 formed on the base plate 18 of the exposure device are fitted into the two positioning through-holes 105, 105 and the two positioning notches 116, 116, thereby exposing a number of the stimulable phosphor layer regions 115 of the stimulable phosphor sheet 110 to a radioactive labeling substance selectively contained in a number of the absorptive regions 104 of the biochemical analysis unit 100.

When a number of the stimulable phosphor layer regions 115 of the stimulable phosphor sheet 110 are exposed to a radioactive labeling substance selectively contained in a number of the absorptive regions 104 of the biochemical analysis unit 100, electron beams (β rays) are released from the radioactive labeling substance contained in the absorptive regions 104 of the biochemical analysis unit 100. However, since a number of absorptive regions 104 of the biochemical analysis unit 100 are formed by pressing the absorptive membrane 103 into a number of the through-holes 102 formed in the substrate 101 made of stainless steel and the substrate 101 made of stainless steel capable of attenuating radiation energy is present around each of the absorptive regions 104, electron beams (β rays) released from the radioactive labeling substance contained in the absorptive regions 104 of the biochemical analysis unit 100 can be efficiently prevented from scattering in the biochemical analysis unit 100. Further, since a number of the stimulable phosphor layer regions 115 of the stimulable phosphor sheet 110 are formed by pressing the stimulable phosphor membrane 111 into a number of the through-holes 112 formed in the support 113 made of stainless steel and the support 113 made of stainless steel capable of attenuating radiation energy is present around each of the stimulable phosphor layer regions 115, electron beams (β rays) released from the radioactive labeling substance contained in the absorptive regions 104 of the biochemical analysis unit 100 can be efficiently prevented from scattering in the stimulable phosphor sheet 110. Therefore, it is possible to effectively prevent electron beams (β rays) released from the radioactive labeling substance contained in a particular absorptive region 104 from entering stimulable phosphor layer regions 115 next to the corresponding stimulable phosphor layer region 115 and to impinge all of them onto the corresponding stimulable phosphor layer region 115, thereby exposing it to electron beams (β rays).

In this manner, radiation data recorded in a number of the absorptive regions 104 of the biochemical analysis unit 100 are transferred onto and recorded in a number of the stimulable phosphor layer regions 115 of the stimulable phosphor sheet 110.

Similarly to the previous embodiments, the radiation data recorded in a number of the stimulable phosphor layer regions 115 of the stimulable phosphor sheet 110 are read by the scanner shown in Figures 7 to 14 to produce biochemical analysis data.

On the other hand, when chemiluminescence data recorded in a number of the absorptive regions 104 of the biochemical analysis unit 100 are to be transferred onto a stimulable phosphor sheet, a number of the absorptive regions 104 of the biochemical analysis unit 100 are first brought into contact with a chemiluminescent substrate.

As a result, chemiluminescence emission in a wavelength of visible light is selectively released from a number of the absorptive regions 104 of the biochemical analysis unit 100.

Figure 24 is a schematic perspective view showing a stimulable phosphor sheet onto which chemiluminescence data are to be transferred, which is a further preferred embodiment of the present invention.

As shown in Figure 24, a stimulable phosphor sheet 120 according to this embodiment has the same configuration as that of the stimulable phosphor sheet 110 shown in Figure 22 except that a number of stimulable phosphor layer regions 125 are formed by pressing a stimulable phosphor membrane 121 containing SrS system stimulable phosphor capable of absorbing and storing light energy into a number of the through-holes 112 formed in the support 113 made of stainless steel.

As shown in Figure 24, similarly to the stimulable phosphor sheet 110 shown in Figure 22, one side portion of the support 113 of the stimulable phosphor sheet 120 corresponding to the one end portion of the substrate 101 of the biochemical analysis unit 100 formed with the positioning notches 105, 105 is formed with two semi-circular positioning notches 116, 116 at positions corresponding to those of the positioning notches 105, 105 formed at the one side portion of the substrate 101 of the biochemical analysis unit 100.

In this embodiment, a number of the stimulable phosphor layer regions 125 formed in the stimulable phosphor sheet 120 are exposed to chemiluminescence emission selectively released from a number of the absorptive regions 104 formed in the biochemical analysis unit 100 using the exposure device shown in Figure 5, thereby transferring chemiluminescence data recorded in a number of the absorptive regions 104 formed in the biochemical analysis unit 100 onto a number of the stimulable phosphor layer regions 125 formed in the stimulable phosphor sheet 120.

When a number of the stimulable phosphor layer regions 125 formed in the stimulable phosphor sheet 120 are to be exposed to chemiluminescence emission selectively released from a number of the absorptive regions 104 formed in the biochemical analysis unit 100, the biochemical analysis unit 100 releasing chemiluminescence emission and the stimulable phosphor sheet 120 are superposed on the base plate 18 of the exposure device in such a manner that the two positioning pins 19, 19 formed on the base plate 18 of the exposure device are fitted into the two positioning through-holes 105, 105 and the two positioning notches 116, 116.

As a result, the biochemical analysis unit 100 and the stimulable phosphor sheet 120 are accurately positioned on the base plate 18 of the exposure device so that each of a number of the stimulable phosphor layer regions 125 formed in the stimulable phosphor sheet 120 faces the corresponding absorptive region 104 formed in the biochemical analysis unit 100.

Thus, each of the stimulable phosphor layer regions 125 formed in the stimulable phosphor sheet 120 is kept to face the corresponding absorptive region 104 formed in the biochemical analysis unit 100 for a predetermined time period, whereby a number of the stimulable phosphor layer regions 125 formed in the stimulable phosphor sheet 120 are exposed to chemiluminescence emission selectively released from a number of the absorptive regions 104 formed in the biochemical analysis unit 100.

In this embodiment, since a number of absorptive regions 104 of the biochemical analysis unit 100 are formed by pressing the absorptive membrane 103 into a number of the through-holes 102 formed in the substrate 101 made of stainless steel and the substrate 101 made of stainless steel capable of attenuating light energy is present around each of the absorptive regions 104, chemiluminescence emission released from the absorptive regions 104 of the biochemical analysis unit 100 can be efficiently prevented from scattering in the biochemical analysis unit 100. Further, since a number of the stimulable phosphor layer regions 125 of the stimulable phosphor sheet 120 are formed by pressing the stimulable phosphor membrane 121 into a number of the through-holes 112 formed in the support 113 made of stainless steel and the support 113 made of stainless steel capable of attenuating light energy is present around each of the stimulable phosphor layer regions 125, chemiluminescence emission released from the absorptive regions 104 of the biochemical analysis unit 100 can be efficiently prevented from scattering in the stimulable phosphor sheet 120. Therefore, it is possible to effectively prevent chemiluminescence emission released from a particular absorptive region 104 from entering stimulable phosphor layer regions 125 next to the corresponding stimulable phosphor layer region 125 and to impinge all of them onto the corresponding stimulable phosphor layer region 125, thereby exposing it to chemiluminescence emission.

In this manner, chemiluminescence data recorded in a number of the absorptive regions 104 of the biochemical analysis unit 100 are transferred onto and recorded in a number of the stimulable phosphor layer regions 125 formed in the support 113 of the stimulable phosphor sheet 120.

Similarly to the previous embodiments, the chemiluminescence data recorded in a number of the stimulable phosphor layer regions 125 of the stimulable phosphor sheet 120 are read by the scanner shown in Figures 16 to 18 to produce biochemical analysis data.

According to the embodiments shown in Figures 21 to 24, since it is possible to accurately position and superpose the biochemical analysis unit 100 and the stimulable phosphor sheet 110 so that each of a number of the absorptive regions 104 formed in the biochemical analysis unit 100 faces the corresponding stimulable phosphor layer region 115 among a number of the stimulable phosphor layer regions 115 formed in the stimulable phosphor sheet 110 in the same pattern as that of a number of the absorptive regions 104 by setting the biochemical analysis unit 100 and the stimulable phosphor sheet 110 in such a manner that the two erect positioning pins 19, 19 formed on the base plate 18 of the exposure device are fitted into the two positioning notches 105, 105 of the biochemical analysis unit 100 and the two positioning notches 116, 116 of the stimulable phosphor sheet 110, a number of the stimulable phosphor layer regions 115 formed in the stimulable phosphor sheet 110 can be exposed to a radioactive labeling substance selectively contained in a number of the absorptive regions 104 formed in the biochemical analysis unit 100 in a desired manner. Therefore, it is possible to produce biochemical analysis data having an excellent quantitative characteristic with high resolution by scanning a number of the stimulable phosphor layer regions 115 exposed to a radioactive labeling substance with the laser beam 24 and photoelectrically detecting stimulated emission 45 released from a number of the stimulable phosphor layer regions 115.

Furthermore, according to the embodiments shown in Figures 21 to 24, since it is possible to accurately position and superpose the biochemical analysis unit 100 and the stimulable phosphor sheet 120 so that each of a number of the absorptive regions 104 formed in the biochemical analysis unit 100 faces the corresponding stimulable phosphor layer region 125 among a number of the stimulable phosphor layer regions 125 formed in the stimulable phosphor sheet 120 in the same pattern as that of a number of the absorptive regions 104 by setting the biochemical analysis unit 100 and the stimulable phosphor sheet 120 in such a manner that the two erect positioning pins 19, 19 formed on the base plate 18 of the exposure device are fitted into the two positioning notches 105, 105 of the biochemical analysis unit 100 and the two positioning notches 116, 116 of the stimulable phosphor sheet 120, a number of the stimulable phosphor layer regions 125 formed in the stimulable phosphor sheet 120 can be exposed to chemiluminescence emission selectively released from a number of the absorptive regions 104 formed in the biochemical analysis unit 100 in a desired manner. Therefore, it is possible to produce biochemical analysis data having an excellent quantitative characteristic with high resolution by scanning a number of the stimulable phosphor layer regions 125 exposed to chemiluminescence emission with the laser beam 24 and photoelectrically detecting stimulated emission 45 released from a number of the stimulable phosphor layer regions 125.

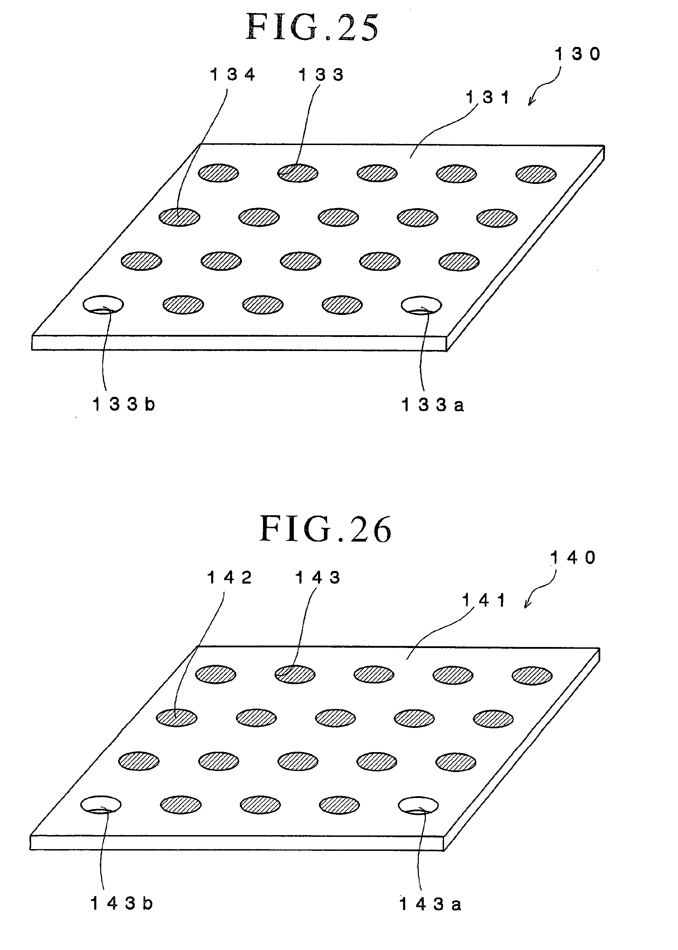

Figure 25 is a schematic perspective view showing a biochemical analysis unit which is a further preferred embodiment of the present invention.

As shown in Figure 25, a biochemical analysis unit 130 according to this embodiment includes a substrate 131 made of stainless steel and regularly formed with a number of through-holes 133 and a number of absorptive regions 134 are formed by charging nylon-6 in a number of the through-holes 133 except two through-holes 133a and 133b.

Although not accurately shown in Figure 25, in this embodiment, about 10,000 substantially circular through-holes 133 having a size of about 0.01 mm² are formed in a regular pattern at a density of about 5,000 per cm² in the substrate 131.

A number of the absorptive regions 134 are formed by charging nylon-6 in a number of the through-holes 133 in such a manner that the surfaces thereof lie at the same height level as that of the surface of the substrate 131.

In this embodiment, similarly to the biochemical analysis unit 1 shown in Figure 1, a solution containing specific binding substances such as cDNAs is spotted using the spotting device shown in Figure 2 onto a number of the absorptive regions 134 formed in the biochemical analysis unit 130 and the specific binding substances are absorbed in a number of the absorptive regions 134.

Further, as shown in Figure 3, the biochemical analysis unit 130 is set in the hybridization reaction vessel 8 accommodating a hybridization reaction solution 9 containing a substance derived from a living organism and labeled with a radioactive labeling substance, a substance derived from a living organism and labeled with a labeling substance which generates chemiluminescence emission when it contacts a chemiluminescent substrate and a substance derived from a living organism and labeled with a fluorescent substance such as a fluorescent dye and specific binding substances absorbed in a number of the absorptive regions 134 of the biochemical analysis unit 130 are selectively hybridized with a substance derived from a living organism, labeled with a radioactive labeling substance and contained in the hybridization reaction solution 9, a substance derived from a living organism, labeled with a labeling substance which generates chemiluminescence emission when it contacts a chemiluminescent substrate and contained in the hybridization reaction solution 9 and a substance derived from a living organism, labeled with a fluorescent substance such as a fluorescent dye and contained in the hybridization reaction solution 9.

Thus, radiation data, chemiluminescence data and fluorescence data are recorded in a number of the absorptive regions 134 formed in the biochemical analysis unit 130.

Similarly to the previous embodiments, fluorescence data recorded in a number of the absorptive regions 134 of the biochemical analysis unit 130 are read by the scanner shown in Figures 7 to 14 to produce biochemical analysis data.

On the other hand, radiation data recorded in a number of the absorptive regions 134 of the biochemical analysis unit 130 are transferred onto a stimulable phosphor sheet and chemiluminescence data recorded in a number of the absorptive regions 134 of the biochemical analysis unit 130 are transferred onto another stimulable phosphor sheet.

Figure 26 is a schematic perspective view showing a stimulable phosphor sheet onto which radiation data are to be transferred, which is a further preferred embodiment of the present invention.

As shown in Figure 26, a stimulable phosphor sheet 140 according to this embodiment includes a support 141 made of stainless steel and regularly formed with a number of substantially circular through-holes 143 and a number of stimulable phosphor layer regions 142 are formed by charging BaFX system stimulable phosphor (where X is at least one halogen atom selected from the group consisting of Cl, Br and I) capable of absorbing and storing radiation energy in a number of the through-holes 143 except two through-holes 143a and 143b.

A number of the through-holes 143 are formed in the support 141 in the same pattern as that of a number of the through-holes 133 formed in the substrate 131 of the biochemical analysis unit 130 so that each of them has the same size as that of each of the through-holes 133 formed in the substrate 131 of the biochemical analysis unit 130.

Therefore, although not accurately shown in Figure 26, in this embodiment, about 10,000 substantially circular stimulable phosphor layer regions 142 having a size of about 0.01 mm² are dot-like formed in a regular pattern at a density of about 5,000 per cm² in the support 141 of the stimulable phosphor sheet 140 in the same pattern as that of a number of the absorptive regions 134 formed in the substrate 131 of the biochemical analysis unit 130.

The through-holes 143a and 143b in which no stimulable phosphor is charged are formed in the support 141 of the stimulable phosphor sheet 140 at positions corresponding to those of the two through-holes 133a and 133b in which no nylon-6 is charged formed in the substrate 131 of the biochemical analysis unit 130.

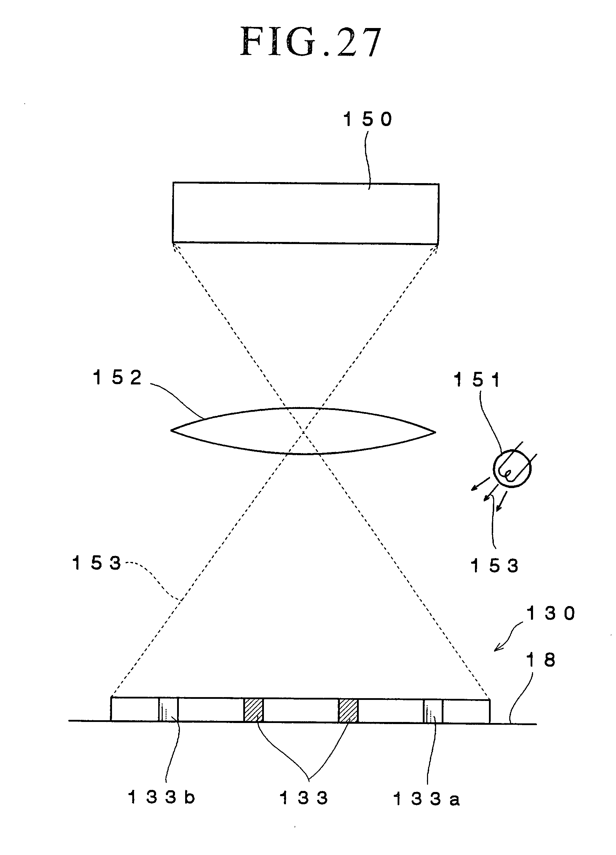

Figure 27 is a schematic perspective view showing the internal structure of an exposure device used for a method for exposing a stimulable phosphor sheet, which is a further preferred embodiment of the present invention.

As shown in Figure 27, the exposure device according to this embodiment includes a CCD area sensor 150, a light source 151 and a condenser lens 152. In this embodiment, an ultraviolet ray source for emitting light which cannot excite stimulable phosphor and whose energy cannot be stored in stimulable phosphor is selected for the light source 151.

When a number of the stimulable phosphor layer regions 142 formed in the stimulable phosphor sheet 140 are to be exposed to a radioactive labeling substance selectively contained in a number of the absorptive regions 134 formed in the biochemical analysis unit 130 to transfer radiation data recorded in a number of the absorptive regions 134 of the biochemical analysis unit 130 onto a number of the stimulable phosphor layer regions 142 of the stimulable phosphor sheet 140, the biochemical analysis unit 130 is first placed on the base plate 18 of the exposure device and the biochemical analysis unit 130 is irradiated with light emitted from the light source 151.

Light 153 emitted from the light source 151 and reflected by the biochemical analysis unit 130 is condensed onto the light detecting surface of the CCD area sensor 150 by the condenser lens 152.

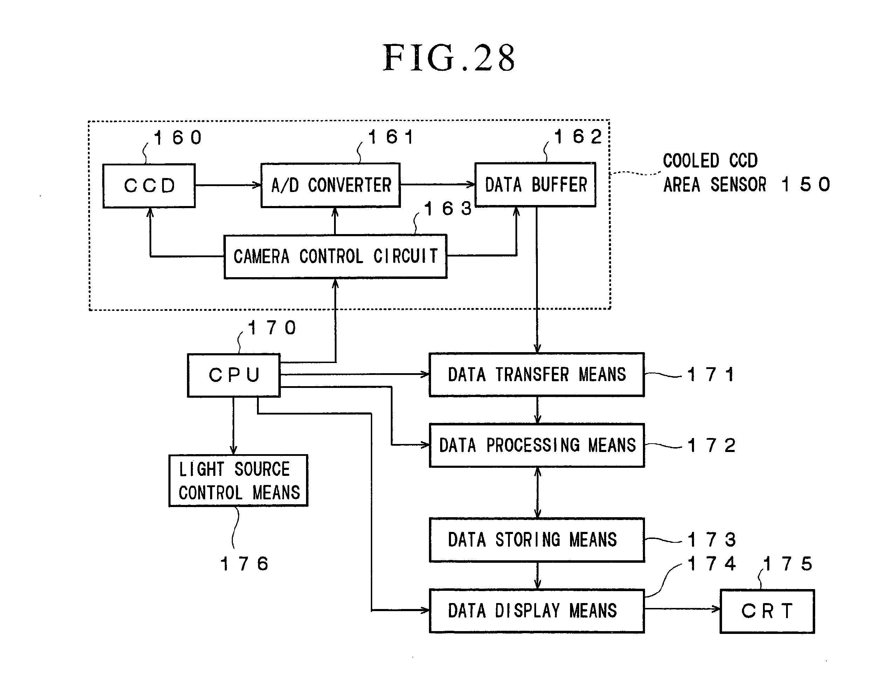

Figure 28 is a block diagram of a control system, a detection system and a memory system of the CCD area sensor 150 and a control system, a memory system and a display system of the exposure device shown in Figure 27.

As shown in Figure 28, the CCD area sensor 150 includes a CCD 160, an A/D converter 161 for digitizing analog data produced by accumulating electric charge with the CCD 160 to produce image data, a data buffer 162 for temporarily storing image data produced by digitizing analog data by the A/D converter 161 and a camera control circuit 163 for controlling the overall operation of the CCD area sensor 150.

As shown in Figure 28, the exposure device according to this embodiment includes a CPU 170 for controlling the overall operation of the exposure device, a data transfer means 171 for reading image data produced by the CCD area sensor 150 from the data buffer 162, a data processing means 172 for effecting data processing on image data read by the data transfer means 171, a data storing means 173 for storing image data subjected to data processing by the data processing means 172, a data display means 174 for displaying a visible image on the screen of a CRT 175 based on image data stored in the data storing means 173 and a light source control means 176 for controlling the light source 151. The CPU 170 is constituted so as to control the light source control means 176 and output various signals to the camera control circuit 163 of the CCD area sensor 150.

Light 153 emitted from the light source 151 and reflected by the biochemical analysis unit 130 is condensed onto the light detecting surface of the CCD 160 by the condenser lens 152, thereby forming an image. The CCD 160 receives light of the image formed on the light detecting surface thereof and accumulates it in the form of electric charge.

When a predetermined time has passed, the CPU 170 outputs an exposure completion signal to the camera control circuit 163 of the CCD area sensor 150 and the light source control means 176.

When the light source control means 176 receives the exposure completion signal from the CPU 170, it turns off the light source 151.

On the other hand, when the camera control circuit 163 receives the exposure completion signal from the CPU 170, it transfers analog data accumulated in the CCD 160 in the form of electric charge to the A/D converter 161 to cause the A/D converter 161 to digitize the data, thereby producing image data and to temporarily store the thus produced image data in the data buffer 162.

At the same time, the CPU 170 outputs a data transfer signal to the data transferring means 171 to cause it to read out the image data from the data buffer 162 of the CCD area sensor 150 and to input them to the data processing means 172.

The data processing means 172 effects necessary data processing on the image data and stores the image data in the data storing means 173.

Then, the CPU 170 outputs a data display signal to the data display means 174, thereby causing it to display a visible image on the screen of the CRT 175 based on the image data stored in the data storing means 173.

Image data of the biochemical analysis unit 130 including the two through-holes 133a and 133b in which no nylon-6 is charged are produced and stored in the data storing means 173 in this manner and a visible image of the biochemical analysis unit 130 including the two through-holes 133a and 133b in which no nylon-6 is charged is displayed on the screen of the CRT 175.

Then, the stimulable phosphor sheet 140 is placed on the biochemical analysis unit 130 set on the base plate 18 of the exposure device and the light source 151 is again activated, thereby irradiating the stimulable phosphor sheet 140 with light emitted from the light source 151.

Similarly to the production of the image data of the biochemical analysis unit 130, image data of the stimulable phosphor sheet 140 including the two through-holes 143a and 143b in which no stimulable phosphor is charged are produced by the CCD area sensor 150 and stored in the data storing means 173 and a visible image of the stimulable phosphor sheet 140 including the two through-holes 143a and 143b in which no stimulable phosphor is charged is displayed on the screen of the CRT 175 together with the visible image of the biochemical analysis unit 130 including the two through-holes 133a and 133b in which no nylon-6 is charged.

While viewing the visible images displayed on the screen of the CRT 175, the user adjusts the position of the stimulable phosphor sheet 140 so that the two through-holes 143a and 143b in which no stimulable phosphor is charged of the stimulable phosphor sheet 140 overlap with the two through-holes 133a and 133b in which no nylon-6 is charged of the biochemical analysis unit 130, whereby it is possible to accurately position and superpose the biochemical analysis unit 130 and the stimulable phosphor sheet 140 so that each of a number of the absorptive regions 134 formed in the substrate 131 of the biochemical analysis unit 130 faces the corresponding stimulable phosphor layer regions 142 among a number of the stimulable phosphor layer regions 142 formed in the support 141 of the stimulable phosphor sheet 140 in the same pattern as that of a number of the absorptive regions 134.

As a result, electron beams (β rays) released from the individual absorptive regions 134 of the biochemical analysis unit 130 are selectively impinged onto the corresponding stimulable phosphor layer regions 142 of the stimulable phosphor sheet 140 and the individual stimulable phosphor layer regions 142 of the stimulable phosphor sheet 140 are exposed to electron beams (β rays) released from the corresponding absorptive regions 134 of the biochemical analysis unit 130.

In this manner, radiation data recorded in a number of the absorptive regions 134 of the biochemical analysis unit 130 are transferred onto a number of the stimulable phosphor layer regions 142 of the stimulable phosphor sheet 140 and similarly to the previous embodiments, the radiation data transferred onto a number of the stimulable phosphor layer regions 142 of the stimulable phosphor sheet 140 are read by the scanner shown in Figures 7 to 14, thereby producing biochemical analysis data.

On the other hand, chemiluminescence data recorded in a number of the absorptive regions 134 are transferred onto another stimulable phosphor sheet and biochemical analysis data are produced by reading chemiluminescence data transferred onto the stimulable phosphor sheet.

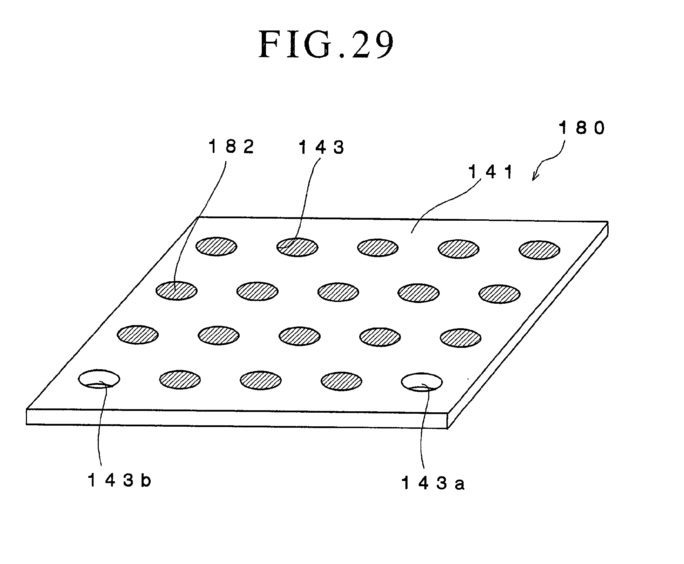

Figure 29 is a schematic perspective view showing a stimulable phosphor sheet onto which chemiluminescence data are to be transferred, which is a further preferred embodiment of the present invention.

As shown in Figure 29, a stimulable phosphor sheet 180 according to this embodiment has the same configuration as that of the stimulable phosphor sheet 140 shown in Figure 26 except that a number of stimulable phosphor layer regions 182 are formed by charging SrS system stimulable phosphor capable of absorbing and storing light energy in a number of the through-holes 143 formed in the support 141 made of stainless steel except the two through-holes 143a and 143b.

When chemiluminescence data recorded in a number of the absorptive regions 134 of the biochemical analysis unit 130 are to be transferred onto a number of the stimulable phosphor layer regions 182 of the stimulable phosphor sheet 180, a number of the absorptive regions 134 of the biochemical analysis unit 130 are first brought into contact with a chemiluminescent substrate.

As a result, chemiluminescence emission in a wavelength of visible light is selectively released from a number of the absorptive regions 134 of the biochemical analysis unit 130.

Similarly to the operation for exposing a number of the stimulable phosphor layer regions 142 of the stimulable phosphor sheet 140 to a radioactive labeling substance selectively contained in a number of the absorptive regions 134 of the biochemical analysis unit 130, image data of the biochemical analysis unit 130 including the two through-holes 133a and 133b in which no nylon-6 is charged and placed on the base plate 18 of the exposure device are produced by the CCD area sensor 150 and a visible image of the biochemical analysis unit 130 including the two through-holes 133a and 133b in which no nylon-6 is charged is displayed on the screen of the CRT 175.

The stimulable phosphor sheet 180 including the two through-holes 143a and 143b in which no stimulable phosphor is charged is placed on the biochemical analysis unit 130 placed on the base plate 18 of the exposure device and similarly to the operation for exposing a number of the stimulable phosphor layer regions 142 of the stimulable phosphor sheet 140 to a radioactive labeling substance selectively contained in a number of the absorptive regions 134 of the biochemical analysis unit 130, image data of the stimulable phosphor sheet 180 including the two through-holes 143a and 143b in which no stimulable phosphor is charged are produced by the CCD area sensor 150 and a visible image of the stimulable phosphor sheet 180 including the two through-holes 143a and 143b in which no stimulable phosphor is charged is displayed on the screen of the CRT 175 together with the visible image of the biochemical analysis unit 130 including the two through-holes 133a and 133b in which no nylon-6 is charged.

While viewing the visible images displayed on the screen of the CRT 175, the user adjusts the position of the stimulable phosphor sheet 180 so that the two through-holes 143a and 143b in which no stimulable phosphor is charged of the stimulable phosphor sheet 180 overlap with the two through-holes 133a and 133b in which no nylon-6 is charged of the biochemical analysis unit 130, whereby it is possible to accurately position and superpose the biochemical analysis unit 130 and the stimulable phosphor sheet 180 so that each of a number of the absorptive regions 134 formed in the substrate 131 of the biochemical analysis unit 130 faces the corresponding stimulable phosphor layer regions 182 among a number of the stimulable phosphor layer regions 182 formed in the support 141 of the stimulable phosphor sheet 180 in the same pattern as that of a number of the absorptive regions 134.

As a result, chemiluminescence emission released from the individual absorptive regions 134 of the biochemical analysis unit 130 are selectively impinged onto the corresponding stimulable phosphor layer regions 182 of the stimulable phosphor sheet 180 and the individual stimulable phosphor layer regions 182 of the stimulable phosphor sheet 180 are exposed to chemiluminescence emission released from the corresponding absorptive regions 134 of the biochemical analysis unit 130.

In this manner, chemiluminescence data recorded in a number of the absorptive regions 134 of the biochemical analysis unit 130 are transferred onto a number of the stimulable phosphor layer regions 182 of the stimulable phosphor sheet 180 and similarly to the previous embodiments, the chemiluminescence data transferred onto a number of the stimulable phosphor layer regions 182 of the stimulable phosphor sheet 180 are read by the scanner shown in Figures 16 to 18, thereby producing biochemical analysis data.

According to the embodiments shown in Figure 25 to 29, since it is possible to accurately position the stimulable phosphor sheet 140 on the biochemical analysis unit 130 so that each of a number of the absorptive regions 134 formed in substrate 131 of the biochemical analysis unit 130 faces the corresponding stimulable phosphor layer region 142 among a number of the stimulable phosphor layer regions 142 formed in the support 141 of the stimulable phosphor sheet 140 in the same pattern as that of a number of the absorptive regions 134 by placing the biochemical analysis unit 130 including the two through-holes 133a and 133b in which no nylon-6 is charged on the base plate 18 of the exposure device, producing image data of the biochemical analysis unit 130 including the two through-holes in which no nylon-6 is charged using the CCD area sensor 150 to display the visible image of the biochemical analysis unit 130 including the two through-holes in which no nylon-6 is charged on the screen of the CRT 175 based thereon, placing the stimulable phosphor sheet 140 including the two through-holes 143a and 143b in which no stimulable phosphor is charged at positions corresponding to those of the two through-holes 133a and 133b of the biochemical analysis unit 130 in which no nylon-6 is charged on the biochemical analysis unit 130 placed on the base plate 18 of the exposure device, producing image data of the stimulable phosphor sheet 140 including the two through-holes 143a and 143b in which no stimulable phosphor is charged to display the visible image of the stimulable phosphor sheet 140 including the two through-holes 143a and 143b in which no stimulable phosphor is charged on the screen of the CRT 175 based thereon together with the visible image of the biochemical analysis unit 130 including the two through-holes in which no nylon-6 is charged and moving the stimulable phosphor sheet 140 on the biochemical analysis unit 130 so that the images of two through-holes 143a and 143b in which no stimulable phosphor is charged overlap with the images of the two through-holes 133a and 133b in which no nylon-6 is charged, electron beams (β rays) released from the individual absorptive regions 134 of the biochemical analysis unit 130 can be selectively impinged onto the corresponding stimulable phosphor layer regions 142 of the stimulable phosphor sheet 140 and the individual stimulable phosphor layer regions 142 of the stimulable phosphor sheet 140 can be exposed to electron beams (β rays) released from the corresponding absorptive regions 134 of the biochemical analysis unit 130. Therefore, it is possible to produce biochemical analysis data having an excellent quantitative characteristic with high resolution by scanning a number of the stimulable phosphor layer regions 142 exposed to a radioactive labeling substance with the laser beam 24 and photoelectrically detecting stimulated emission 45 released from a number of the stimulable phosphor layer regions 142.

Furthermore, according to the embodiments shown in Figure 25 to 29, since it is possible to accurately position the stimulable phosphor sheet 180 on the biochemical analysis unit 130 so that each of a number of the absorptive regions 134 formed in substrate 131 of the biochemical analysis unit 130 faces the corresponding stimulable phosphor layer region 182 among a number of the stimulable phosphor layer regions 182 formed in the support 141 of the stimulable phosphor sheet 180 in the same pattern as that of a number of the absorptive regions 134 by placing the biochemical analysis unit 130 including the two through-holes 133a and 133b in which no nylon-6 is charged on the base plate 18 of the exposure device, producing image data of the biochemical analysis unit 130 including the two through-holes in which no nylon-6 is charged using the CCD area sensor 150 to display the visible image of the biochemical analysis unit 130 including the two through-holes in which no nylon-6 is charged on the screen of the CRT 175 based thereon, placing the stimulable phosphor sheet 180 including the two through-holes 143a and 143b in which no stimulable phosphor is charged at positions corresponding to those of the two through-holes 133a and 133b of the biochemical analysis unit 130 in which no nylon-6 is charged on the biochemical analysis unit 130 placed on the base plate 18 of the exposure device, producing image data of the stimulable phosphor sheet 180 including the two through-holes 143a and 143b in which no stimulable phosphor is charged to display the visible image of the stimulable phosphor sheet 180 including the two through-holes 143a and 143b in which no stimulable phosphor is charged on the screen of the CRT 175 based thereon together with the visible image of the biochemical analysis unit 130 including the two through-holes in which no nylon-6 is charged and moving the stimulable phosphor sheet 180 on the biochemical analysis unit 130 so that the images of two through-holes 143a and 143b in which no stimulable phosphor is charged overlap with the images of the two through-holes 133a and 133b in which no nylon-6 is charged, chemiluminescence emission released from the individual absorptive regions 134 of the biochemical analysis unit 130 can be selectively impinged onto the corresponding stimulable phosphor layer regions 182 of the stimulable phosphor sheet 180 and the individual stimulable phosphor layer regions 182 of the stimulable phosphor sheet 180 can be exposed to chemiluminescence emission released from the corresponding absorptive regions 134 of the biochemical analysis unit 130. Therefore, it is possible to produce biochemical analysis data having an excellent quantitative characteristic with high resolution by scanning a number of the stimulable phosphor layer regions 182 exposed to chemiluminescence emission with the laser beam 24 and photoelectrically detecting stimulated emission 45 released from a number of the stimulable phosphor layer regions 182.

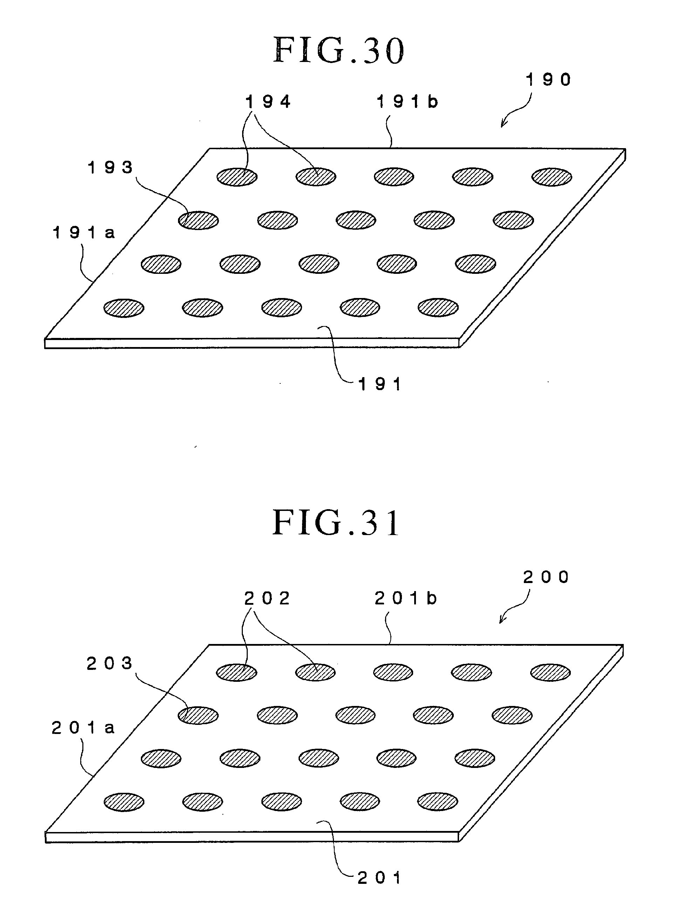

Figure 30 is a schematic perspective view showing a biochemical analysis unit which is a further preferred embodiment of the present invention.

As shown in Figure 30, a biochemical analysis unit 190 according to this embodiment includes a substrate 191 made of stainless steel and regularly formed with a number of through-holes 193 and a number of absorptive regions 194 are formed by charging nylon-6 in a number of the through-holes 193.

Although not accurately shown in Figure 30, in this embodiment, about 10,000 substantially circular through-holes 193 having a size of about 0.01 mm² are formed in a regular pattern at a density of about 5,000 per cm² in the substrate 191.

A number of the absorptive regions 194 are formed by charging nylon-6 in a number of the through-holes 193 in such a manner that the surfaces thereof lie at the same height level as that of the surface of the substrate 191.

In this embodiment, each of a number of the through-holes 193, and, therefore, each of a number of the absorptive regions 194, is formed in a predetermined positional relationship to a first reference edge 191a of the substrate 191 of the biochemical analysis unit 190 and a second reference edge 191b of the substrate 191 perpendicular to first reference edge 191a.

In this embodiment, similarly to the biochemical analysis unit 1 shown in Figure 1, a solution containing specific binding substances such as cDNAs is spotted using the spotting device shown in Figure 2 onto a number of the absorptive regions 194 formed in the biochemical analysis unit 190 and the specific binding substances are absorbed in a number of the absorptive regions 194.

Further, as shown in Figure 3, the biochemical analysis unit 190 is set in the hybridization reaction vessel 8 accommodating a hybridization reaction solution 9 containing a substance derived from a living organism and labeled with a radioactive labeling substance, a substance derived from a living organism and labeled with a labeling substance which generates chemiluminescence emission when it contacts a chemiluminescent substrate and a substance derived from a living organism and labeled with a fluorescent substance such as a fluorescent dye and specific binding substances absorbed in a number of the absorptive regions 194 of the biochemical analysis unit 190 are selectively hybridized with a substance derived from a living organism, labeled with a radioactive labeling substance and contained in the hybridization reaction solution 9, a substance derived from a living organism, labeled with a labeling substance which generates chemiluminescence emission when it contacts a chemiluminescent substrate and contained in the hybridization reaction solution 9 and a substance derived from a living organism, labeled with a fluorescent substance such as a fluorescent dye and contained in the hybridization reaction solution 9.

Thus, radiation data, chemiluminescence data and fluorescence data are recorded in a number of the absorptive regions 194 formed in the biochemical analysis unit 190.

Similarly to the previous embodiments, fluorescence data recorded in a number of the absorptive regions 134 of the biochemical analysis unit 130 are read by the scanner shown in Figures 7 to 14 to produce biochemical analysis data.

On the other hand, radiation data recorded in a number of the absorptive regions 194 of the biochemical analysis unit 190 are transferred onto a stimulable phosphor sheet and chemiluminescence data recorded in a number of the absorptive regions 194 of the biochemical analysis unit 190 are transferred onto another stimulable phosphor sheet.

Figure 31 is a schematic perspective view showing a stimulable phosphor sheet onto which radiation data are to be transferred, which is a further preferred embodiment of the present invention.

As shown in Figure 31, a stimulable phosphor sheet 200 according to this embodiment includes a support 201 made of stainless steel and regularly formed with a number of substantially circular through-holes 203 and a number of stimulable phosphor layer regions 202 are formed by charging BaFX system stimulable phosphor (where X is at least one halogen atom selected from the group consisting of Cl, Br and I) capable of absorbing and storing radiation energy in a number of the through-holes 203.

A number of the through-holes 203 are formed in the support 201 in the same pattern as that of a number of the through-holes 193 formed in the substrate 191 of the biochemical analysis unit 190 so that each of them has the same size as that of each of the through-holes 193 formed in the substrate 191 of the biochemical analysis unit 190.

In this embodiment, each of a number of the through-holes 203, and, therefore, each of a number of the stimulable phosphor layer regions 202, is formed in the same positional relationship to a first reference edge 201a and a second reference edge 201b of the support 201 of the stimulable phosphor sheet 200 as that of a number of the absorptive regions 194 to the first reference edge 191a of the substrate 191 of the biochemical analysis unit 190 and the second reference edge 191b of the substrate 191 perpendicular to the first reference edge 191a.

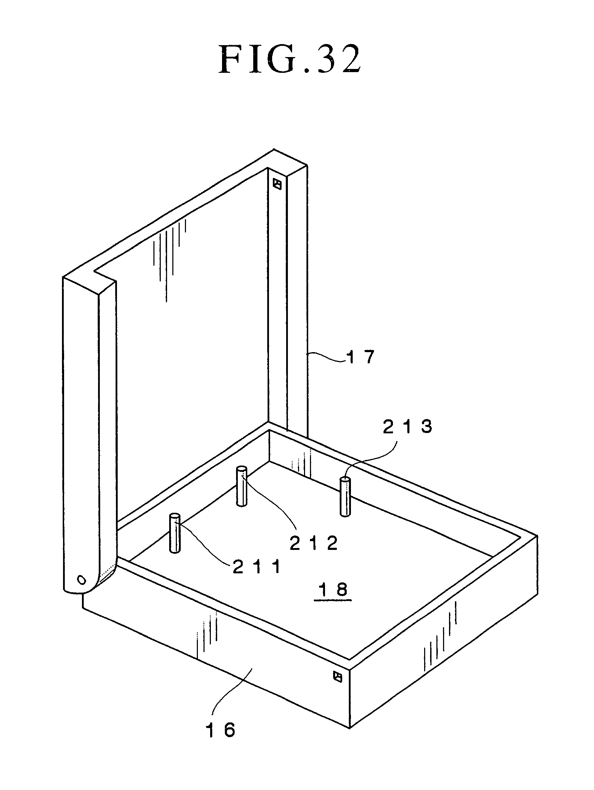

Figure 32 is a schematic cross sectional view showing an exposure device used for a method for exposing a stimulable phosphor sheet which is a further preferred embodiment of the present invention.

As shown in Figure 32, an exposure device according to this embodiment includes a first reference pin 211, a second reference pin 212 and a third erect reference pin 213 formed on the base plate 18. The third reference pin 213 is formed on the base plate 18 so as to be located on a line perpendicular to a line connecting the first reference pin 211 and the second reference pin 212.

When a number of the stimulable phosphor layer regions 202 formed in the stimulable phosphor sheet 200 are to be exposed to a radioactive labeling substance selectively contained in a number of the absorptive regions 194 formed in the biochemical analysis unit 190 to transfer radiation data recorded in a number of the absorptive regions 194 of the biochemical analysis unit 190 onto a number of the stimulable phosphor layer regions 202 of the stimulable phosphor sheet 200, the biochemical analysis unit 190 is first placed on the base plate 18 of the exposure device in such a manner that the first reference edge 191a of the substrate 191 of the biochemical analysis unit 190 aligns with the first erect reference pin 211 and the second erect reference pin 212 formed on the base plate 18 of the exposure device and the second reference edge 191b of the substrate 191 of the biochemical analysis unit 190 abuts against the third erect reference pin 213 formed on the base plate 18 of the exposure device.

The stimulable phosphor sheet 200 is then superposed on the biochemical analysis unit 190 placed on the base plate 18 of the exposure device in such a manner that the first reference edge 201a of the stimulable phosphor sheet 200 aligns with the first erect reference pin 211 and the second erect reference pin 212 formed on the base plate 18 of the exposure device and the second reference edge 201b of the stimulable phosphor sheet 200 abuts against the third erect reference pin 213 formed on the base plate 18 of the exposure device.

In this embodiment, since each of a number of the through-holes 193, and, therefore each of a number of the absorptive regions 194, is formed in a predetermined positional relationship to a first reference edge 191a of the substrate 191 of the biochemical analysis unit 190 and a second reference edge 191b of the substrate 191 perpendicular to first reference edge 191a, while each of a number of the through-holes 203, and, therefore, each of a number of the stimulable phosphor layer regions 202, is formed in the same positional relationship to a first reference edge 201a and a second reference edge 201b of the support 201 of the stimulable phosphor sheet 200 as that of a number of the absorptive regions 194 to the first reference edge 191a of the substrate 191 of the biochemical analysis unit 190 and the second reference edge 191b of the substrate 191 perpendicular to athe first reference edge 191a, it is possible to accurately position the biochemical analysis unit 190 and the stimulable phosphor sheet 200 so that each of a number of the absorptive regions 194 formed in the substrate 191 of the biochemical analysis unit 190 faces the corresponding stimulable phosphor layer region 202 among a number of the stimulable phosphor layer regions 202 formed in the support 201 of the stimulable phosphor sheet 200 in the same pattern as that of a number of the absorptive regions 194 by placing the biochemical analysis unit 190 on the base plate 18 of the exposure device in such a manner that the first reference edge 191a of the substrate 191 of the biochemical analysis unit 190 aligns with the first erect reference pin 211 and the second erect reference pin 212 formed on the base plate 18 of the exposure device and the second reference edge 191b of the substrate 191 of the biochemical analysis unit 190 abuts against the third erect reference pin 213 formed on the base plate 18 of the exposure device and superposing the stimulable phosphor sheet 200 on the biochemical analysis unit 190 in such a manner that the first reference edge 201a of the stimulable phosphor sheet 200 aligns with the first erect reference pin 211 and the second erect reference pin 212 formed on the base plate 18 of the exposure device and the second reference edge 201b of the stimulable phosphor sheet 200 abuts against the third erect reference pin 213 formed on the base plate 18 of the exposure device. Therefore, since the individual stimulable phosphor layer regions 202 formed in the support 201 of the stimulable phosphor sheet 200 can be exposed to electron beams (β rays) released from the corresponding absorptive regions 194 formed in the substrate 191 of the biochemical analysis unit 190, it is possible to produce biochemical analysis data having an excellent quantitative characteristic with high resolution by scanning a number of the stimulable phosphor layer regions 202 exposed to a radioactive labeling substance with the laser beam 24 and photoelectrically detecting stimulated emission 45 released from a number of the stimulable phosphor layer regions 202.

In this manner, radiation data recorded in a number of the absorptive regions 194 of the biochemical analysis unit 190 are transferred onto a number of the stimulable phosphor layer regions 202 of the stimulable phosphor sheet 200 and similarly to the previous embodiments, the radiation data transferred onto a number of the stimulable phosphor layer regions 202 of the stimulable phosphor sheet 200 are read by the scanner shown in Figures 7 to 14, thereby producing biochemical analysis data.

On the other hand, chemiluminescence data recorded in a number of the absorptive regions 194 of the biochemical analysis unit 190 are transferred onto another stimulable phosphor sheet and biochemical analysis data are produced by reading chemiluminescence data transferred onto the stimulable phosphor sheet.

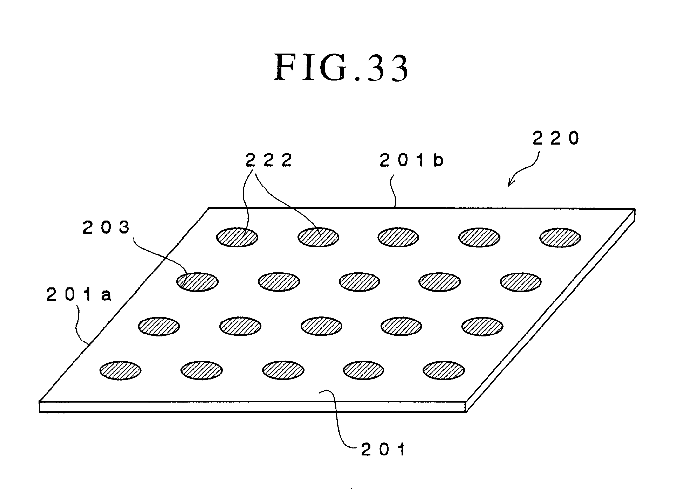

Figure 33 is a schematic perspective view showing a stimulable phosphor sheet onto which chemiluminescence data are to be transferred, which is a further preferred embodiment of the present invention.

As shown in Figure 33, a stimulable phosphor sheet 220 according to this embodiment has the same configuration as that of the stimulable phosphor sheet 200 shown in Figure 31 except that a number of stimulable phosphor layer regions 222 are formed by charging SrS system stimulable phosphor capable of absorbing and storing light energy in a number of the through-holes 203 formed in the support 201 made of stainless steel.

Therefore, each of a number of the stimulable phosphor layer regions 222 is formed in the same positional relationship to a first reference edge 201a and a second reference edge 201b of the support 201 of the stimulable phosphor sheet 220 as that of a number of the absorptive regions 194 to the first reference edge 191a of the substrate 191 of the biochemical analyisis unit 190 and the second reference edge 191b of the substrate 191 perpendicular to the first reference edge 191a.

When chemiluminescence data recorded in a number of the absorptive regions 194 of the biochemical analysis unit 190 are to be transferred onto a number of the stimulable phosphor layer regions 222 of the stimulable phosphor sheet 220, a number of the absorptive regions 194 of the biochemical analysis unit 190 are first brought into contact with a chemiluminescent substrate.

As a result, chemiluminescence emission in a wavelength of visible light is selectively released from a number of the absorptive regions 194 of the biochemical analysis unit 190.

The biochemical analysis unit 190 releasing chemiluminescence emission is then placed on the base plate 18 of the exposure device in such a manner that the first reference edge 191a of the substrate 191 of the biochemical analysis unit 190 aligns with the first erect reference pin 211 and the second erect reference pin 212 formed on the base plate 18 of the exposure device and the second reference edge 191b of the substrate 191 of the biochemical analysis unit 190 abuts against the third erect reference pin 213 formed on the base plate 18 of the exposure device.

The stimulable phosphor sheet 220 is then superposed on the biochemical analysis unit 190 placed on the base plate 18 of the exposure device in such a manner that the first reference edge 201a of the stimulable phosphor sheet 220 aligns with the first erect reference pin 211 and the second erect reference pin 212 formed on the base plate 18 of the exposure device and the second reference edge 201b of the stimulable phosphor sheet 220 abuts against the third erect reference pin 213 formed on the base plate 18 of the exposure device.

In this embodiment, since each of a number of the through-holes 193, and, therefore each of a number of the absorptive regions 194, is formed in a predetermined positional relationship to a first reference edge 191a of the substrate 191 of the biochemical analysis unit 190 and a second reference edge 191b of the substrate 191 perpendicular to the first reference edge 191a, while each of a number of the through-holes 203, and, therefore each of a number of the stimulable phosphor layer regions 222 is formed in the same positional relationship to a first reference edge 201a and a second reference edge 201b of the support 201 of the stimulable phosphor sheet 220 as that of a number of the absorptive regions 194 to the first reference edge 191a of the substrate 191 of the biochemical analysis unit 190 and the second reference edge 191b of the substrate 191 perpendicular to the first reference edge 191a, it is possible to accurately position the biochemical analysis unit 190 and the stimulable phosphor sheet 220 so that each of a number of the absorptive regions 194 formed in the substrate 191 of the biochemical analysis unit 190 faces the corresponding stimulable phosphor layer region 222 among a number of the stimulable phosphor layer regions 222 formed in the support 201 of the stimulable phosphor sheet 220 in the same pattern as that of a number of the absorptive regions 194 by placing the biochemical analysis unit 190 on the base plate 18 of the exposure device in such a manner that the first reference edge 191a of the substrate 191 of the biochemical analysis unit 190 aligns with the first erect reference pin 211 and the second erect reference pin 212 formed on the base plate 18 of the exposure device and the second reference edge 191b of the substrate 191 of the biochemical analysis unit 190 abuts against the third erect reference pin 213 formed on the base plate 18 of the exposure device and superposing the stimulable phosphor sheet 220 on the biochemical analysis unit 190 in such a manner that the first reference edge 201a of the stimulable phosphor sheet 220 aligns with the first erect reference pin 211 and the second erect reference pin 212 formed on the base plate 18 of the exposure device and the second reference edge 201b of the stimulable phosphor sheet 220 abuts against the third erect reference pin 213 formed on the base plate 18 of the exposure device. Therefore, since the individual stimulable phosphor layer regions 222 formed in the support 201 of the stimulable phosphor sheet 220 can be exposed to chemiluminescence emission released from the corresponding absorptive regions 194 formed in the substrate 191 of the biochemical analysis unit 190, it is possible to produce biochemical analysis data having an excellent quantitative characteristic with high resolution by scanning a number of the stimulable phosphor layer regions 222 exposed to chemiluminescence emission with the laser beam 24 and photoelectrically detecting stimulated emission 45 released from a number of the stimulable phosphor layer regions 222.

In this manner, chemiluminescence data recorded in a number of the absorptive regions 194 of the biochemical analysis unit 190 are transferred onto a number of the stimulable phosphor layer regions 222 of the stimulable phosphor sheet 220 and similarly to the previous embodiments, the chemiluminescence data transferred onto a number of the stimulable phosphor layer regions 222 of the stimulable phosphor sheet 220 are read by the scanner shown in Figures 16 to 18, thereby producing biochemical analysis data.

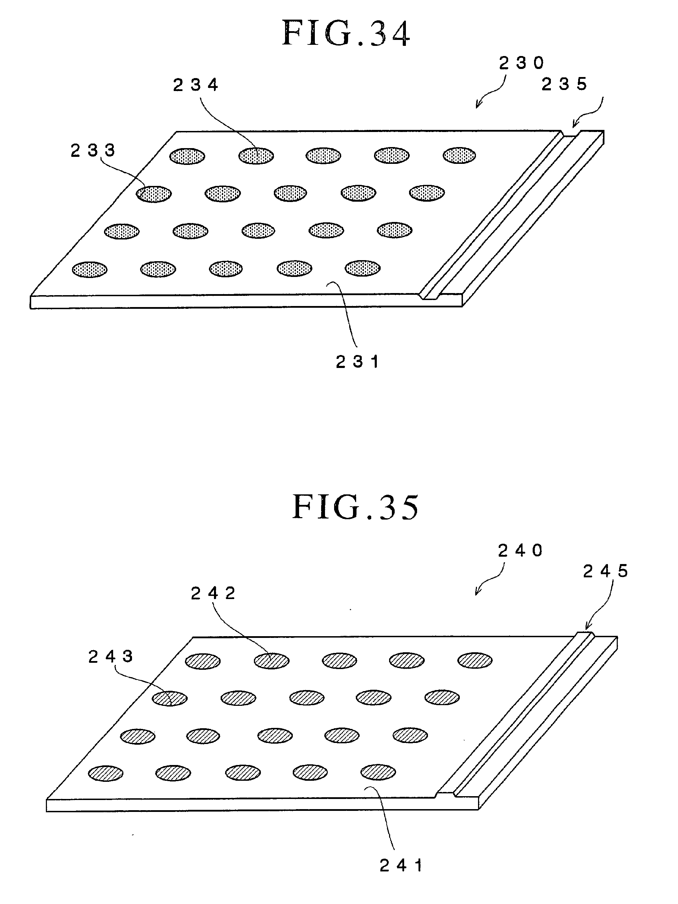

Figure 34 is a schematic perspective view showing a biochemical analysis unit which is a further preferred embodiment of the present invention.

As shown in Figure 34, a biochemical analysis unit 230 according to this embodiment includes a substrate 231 made of stainless steel and regularly formed with a number of through-holes 233 and a number of absorptive regions 234 are formed by charging nylon-6 in a number of the through-holes 233.

Although not accurately shown in Figure 34, in this embodiment, about 10,000 substantially circular through-holes 233 having a size of about 0.01 mm² are formed in a regular pattern at a density of about 5,000 per cm² in the substrate 231.

A number of the absorptive regions 234 are formed by charging nylon-6 in a number of the through-holes 233 in such a manner that the surfaces thereof lie at the same height level as that of the surface of the substrate 231.

As shown in Figure 34, the substrate 231 of the biochemical analysis unit 230 is formed with a recess 235 extending in parallel with one edge portion of the substrate 231.

In this embodiment, similarly to the biochemical analysis unit 1 shown in Figure 1, a solution containing specific binding substances such as cDNAs is spotted using the spotting device shown in Figure 2 onto a number of the absorptive regions 234 formed in the biochemical analysis unit 230 and the specific binding substances are absorbed in a number of the absorptive regions 234.

Further, as shown in Figure 3, the biochemical analysis unit 230 is set in the hybridization reaction vessel 8 accommodating a hybridization reaction solution 9 containing a substance derived from a living organism and labeled with a radioactive labeling substance, a substance derived from a living organism and labeled with a labeling substance which generates chemiluminescence emission when it contacts a chemiluminescent substrate and a substance derived from a living organism and labeled with a fluorescent substance such as a fluorescent dye and specific binding substances absorbed in a number of the absorptive regions 234 of the biochemical analysis unit 230 are selectively hybridized with a substance derived from a living organism, labeled with a radioactive labeling substance and contained in the hybridization reaction solution 9, a substance derived from a living organism, labeled with a labeling substance which generates chemiluminescence emission when it contacts a chemiluminescent substrate and contained in the hybridization reaction solution 9 and a substance derived from a living organism, labeled with a fluorescent substance such as a fluorescent dye and contained in the hybridization reaction solution 9.

Thus, radiation data, chemiluminescence data and fluorescence data are recorded in a number of the absorptive regions 234 formed in the biochemical analysis unit 230.

Similarly to the previous embodiments, fluorescence data recorded in a number of the absorptive regions 234 of the biochemical analysis unit 230 are read by the scanner shown in Figures 7 to 14 to produce biochemical analysis data.

On the other hand, radiation data recorded in a number of the absorptive regions 234 of the biochemical analysis unit 230 are transferred onto a stimulable phosphor sheet and chemiluminescence data recorded in a number of the absorptive regions 234 of the biochemical analysis unit 230 are transferred onto another stimulable phosphor sheet.

Figure 35 is a schematic perspective view showing a stimulable phosphor sheet onto which radiation data are to be transferred, which is a further preferred embodiment of the present invention.

As shown in Figure 35, a stimulable phosphor sheet 240 according to this embodiment includes a support 241 made of stainless steel and regularly formed with a number of substantially circular through-holes 243 and a number of stimulable phosphor layer regions 242 are formed by charging BaFX system stimulable phosphor (where X is at least one halogen atom selected from the group consisting of Cl, Br and I) capable of absorbing and storing radiation energy in a number of the through-holes 243.

A number of the through-holes 243 are formed in the support 241 in the same pattern as that of a number of the through-holes 233 formed in the substrate 231 of the biochemical analysis unit 230 so that each of them has the same size as that of each of the through-holes 233 formed in the substrate 231 of the biochemical analysis unit 230.

Therefore, although not accurately shown in Figure 35, in this embodiment, about 10,000 substantially circular stimulable phosphor layer regions 242 having a size of about 0.01 mm² are dot-like formed in a regular pattern at a density of about 5,000 per cm² in the support 241 of the stimulable phosphor sheet 240 in the same pattern as that of a number of the absorptive regions 234 formed in the substrate 231 of the biochemical analysis unit 230.

As shown in Figure 35, the support 241 of the stimulable phosphor sheet 240 is formed with a convex portion 245 extending in parallel with one edge portion of the support 241 at a position corresponding to that of the recess 235 formed in the substrate 231 of the biochemical analysis unit 230 so as to have a complementary shape to that of the recess 235 formed in the substrate 231 of the biochemical analysis unit 230.

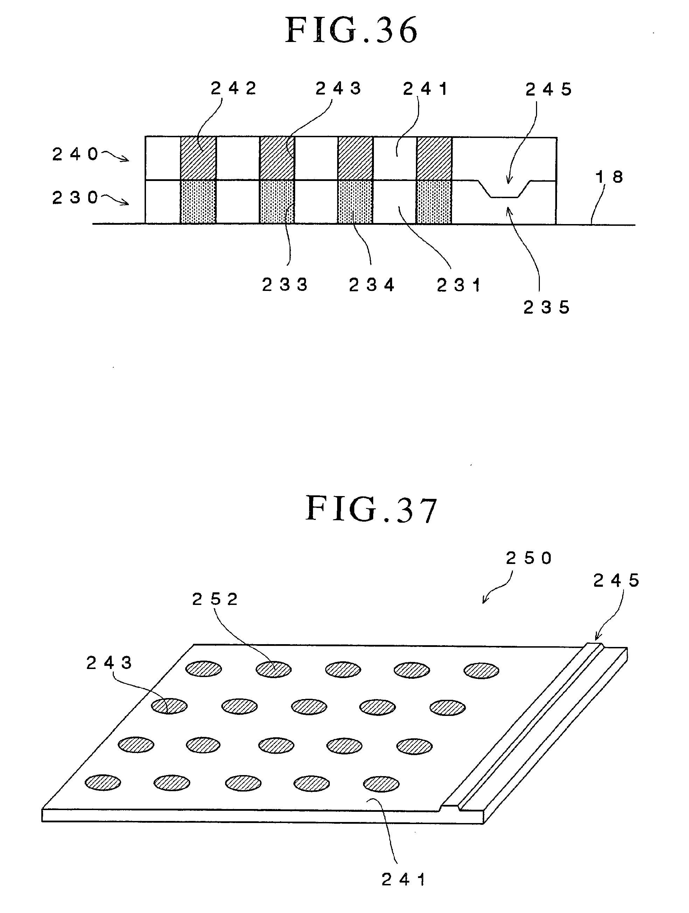

Figure 36 is a schematic cross sectional view showing a method for exposing a number of the stimulable phosphor layer regions 242 formed in the support 241 of the stimulable phosphor sheet 240 to a radioactive labeling substance selectively contained in a number of the absorptive regions 234 formed in the substrate 231 of the biochemical analysis unit 230.

As shown in Figure 36, when a number of the stimulable phosphor layer regions 242 formed in the support 241 of the stimulable phosphor sheet 240 are to be exposed to a radioactive labeling substance selectively contained in a number of the absorptive regions 234 formed in the substrate 231 of the biochemical analysis unit 230 and radiation data recorded in a number of the absorptive regions 234 formed in the substrate 231 of the biochemical analysis unit 230 are to be transferred onto a number of the stimulable phosphor layer regions 242 formed in the support 241 of the stimulable phosphor sheet 240, the biochemical analysis unit 230 is first set on the base plate 18 of the exposure device so that the recess 235 formed in the substrate 231 is directed upward.

The stimulable phosphor sheet 240 is then superposed on the biochemical analysis unit 230 set on the base plate 18 of the exposure device so that the convex portion 245 formed in the support 241 is fitted into the recess 235 formed in the substrate 231 of the biochemical analysis unit 230.

In this embodiment, since the convex portion 245 of the stimulable phosphor sheet 240 is formed at the position corresponding to that of the recess 235 formed in the substrate 231 of the biochemical analysis unit 230 so as to have a complementary shape to that of the recess 235 formed in the substrate 231 of the biochemical analysis unit 230, it is possible to accurately position the biochemical analysis unit 230 and the stimulable phosphor sheet 240 so that each of a number of the absorptive regions 234 formed in the substrate 231 of the biochemical analysis unit 230 faces the corresponding stimulable phosphor layer region 242 among a number of the stimulable phosphor layer regions 242 formed in the support 241 of the stimulable phosphor sheet 240 in the same pattern as that of a number of the absorptive regions 234 by superposing the stimulable phosphor sheet 240 on the biochemical analysis unit 230 in such a manner that the convex portion 245 of the stimulable phosphor sheet 240 is fitted into the recess 235 formed in the substrate 231 of the biochemical analysis unit 230. Therefore, since the individual stimulable phosphor layer regions 242 formed in the support 241 of the stimulable phosphor sheet 240 can be exposed to electron beams (β rays) released from the corresponding absorptive regions 234 formed in the substrate 231 of the biochemical analysis unit 230, it is possible to produce biochemical analysis data having an excellent quantitative characteristic with high resolution by scanning a number of the stimulable phosphor layer regions 242 exposed to a radioactive labeling substance with the laser beam 24 and photoelectrically detecting stimulated emission 45 released from a number of the stimulable phosphor layer regions 242.

In this manner, radiation data recorded in a number of the absorptive regions 234 of the biochemical analysis unit 230 are transferred onto a number of the stimulable phosphor layer regions 242 of the stimulable phosphor sheet 240 and similarly to the previous embodiments, the radiation data transferred onto a number of the stimulable phosphor layer regions 242 of the stimulable phosphor sheet 240 are read by the scanner shown in Figures 7 to 14, thereby producing biochemical analysis data.

On the other hand, chemiluminescence data recorded in a number of the absorptive regions 234 of the biochemical analysis unit 230 are transferred onto another stimulable phosphor sheet and biochemical analysis data are produced by reading chemiluminescence data transferred onto the stimulable phosphor sheet.

Figure 37 is a schematic perspective view showing a stimulable phosphor sheet onto which chemiluminescence data are to be transferred, which is a further preferred embodiment of the present invention.

As shown in Figure 37, a stimulable phosphor sheet 250 according to this embodiment has the same configuration as that of the stimulable phosphor sheet 240 shown in Figure 35 except that a number of stimulable phosphor layer regions 252 are formed by charging SrS system stimulable phosphor capable of absorbing and storing light energy in a number of the through-holes 243 formed in the support 241 made of stainless steel.

Therefore, similarly to the stimulable phosphor sheet 240 shown in Figure 35, the support 241 of the stimulable phosphor sheet 250 is formed with a convex portion 245 extending in parallel with one edge portion of the support 241 at a position corresponding to that of the recess 235 formed in the substrate 231 of the biochemical analysis unit 230 so as to have a complementary shape to that of the recess 235 formed in the substrate 231 of the biochemical analysis unit 230.

When chemiluminescence data recorded in a number of the absorptive regions 234 of the biochemical analysis unit 230 are to be transferred onto a number of the stimulable phosphor layer regions 252 of the stimulable phosphor sheet 250, a number of the absorptive regions 234 of the biochemical analysis unit 230 are first brought into contact with a chemiluminescent substrate.

As a result, chemiluminescence emission in a wavelength of visible light is selectively released from a number of the absorptive regions 234 of the biochemical analysis unit 230.

Then, the biochemical analysis unit 230 releasing chemiluminescence emission is set on the base plate 18 of the exposure device so that the recess 235 formed in the substrate 231 is directed upward.

The stimulable phosphor sheet 250 is further superposed on the biochemical analysis unit 230 set on the base plate 18 of the exposure device so that the convex portion 245 formed in the support 241 is fitted into the recess 235 formed in the substrate 231 of the biochemical analysis unit 230.

In this embodiment, since the convex portion 245 of the stimulable phosphor sheet 250 is formed at the position corresponding to that of the recess 235 formed in the substrate 231 of the biochemical analysis unit 230 so as to have a complementary shape to that of the recess 235 formed in the substrate 231 of the biochemical analysis unit 230, it is possible to accurately position the biochemical analysis unit 230 and the stimulable phosphor sheet 250 so that each of a number of the absorptive regions 234 formed in the substrate 231 of the biochemical analysis unit 230 faces the corresponding stimulable phosphor layer region 252 among a number of the stimulable phosphor layer regions 252 formed in the support 241 of the stimulable phosphor sheet 250 in the same pattern as that of a number of the absorptive regions 234 by superposing the stimulable phosphor sheet 250 on the biochemical analysis unit 230 in such a manner that the convex portion 245 of the stimulable phosphor sheet 250 is fitted into the recess 235 formed in the substrate 231 of the biochemical analysis unit 230. Therefore, since the individual stimulable phosphor layer regions 252 formed in the support 241 of the stimulable phosphor sheet 250 can be exposed to chemiluminescence emission released from the corresponding absorptive regions 234 formed in the substrate 231 of the biochemical analysis unit 230, it is possible to produce biochemical analysis data having an excellent quantitative characteristic with high resolution by scanning a number of the stimulable phosphor layer regions 252 exposed to chemiluminescence emission with the laser beam 24 and photoelectrically detecting stimulated emission 45 released from a number of the stimulable phosphor layer regions 252.

In this manner, chemiluminescence data recorded in a number of the absorptive regions 234 of the biochemical analysis unit 230 are transferred onto a number of the stimulable phosphor layer regions 252 of the stimulable phosphor sheet 250 and similarly to the previous embodiments, the chemiluminescence data transferred onto a number of the stimulable phosphor layer regions 252 of the stimulable phosphor sheet 250 are read by the scanner shown in Figures 16 to 18, thereby producing biochemical analysis data.

The present invention has thus been shown and described with reference to specific embodiments. However, it should be noted that the present invention is in no way limited to the details of the described arrangements but changes and modifications may be made without departing from the scope of the appended claims.

For example, in the above described embodiments, although about 10,000 substantially circular absorptive regions 4, 104, 134, 194, 234 having a size of about 0.01 mm² are formed in the biochemical analysis unit 1, 100, 130, 190, 230 in a regular pattern at a density of about 5,000 per cm², the shape of each of the absorptive regions 4, 104, 134, 194, 234 is not limited to substantially a circular shape but may be formed in an arbitrary shape, for example, a rectangular shape.

Further, in the above described embodiments, although about 10,000 substantially circular absorptive regions 4, 104, 134, 194, 234 having a size of about 0.01 mm² are formed in the biochemical analysis unit 1, 100, 130, 190, 230 in a regular pattern at a density of about 5,000 per cm², the number or size of the absorptive regions 4, 104, 134, 194, 234 may be arbitrarily selected in accordance with the purpose. Preferably, 10 or more of the absorptive regions 4, 104, 134, 194, 234 having a size of 5 mm² or less are formed in the biochemical analysis unit 1, 100, 130, 190, 230 at a density of 10/ cm² or greater.

Furthermore, in the above described embodiments, although about 10,000 substantially circular stimulable phosphor layer regions 12, 82, 115, 125, 142, 182, 202, 222, 242, 252 having a size of about 0.01 mm² are regularly formed in the stimulable phosphor sheet 10, 80, 110, 120, 140, 180, 200, 220, 240, 250 at a density of about 5,000 per cm² in the same pattern as that of the absorptive regions 4, 104, 134, 194, 234 formed in the biochemical analysis unit 1, 100, 130, 190, 230, the shape of each of the stimulable phosphor layer regions 12, 82, 115, 125, 142, 182, 202, 222, 242, 252 is not limited to substantially a circular shape but may be formed in an arbitrary shape, for example, a rectangular shape.

Moreover, in the above described embodiments, although about 10,000 substantially circular stimulable phosphor layer regions 12, 82, 115, 125, 142, 182, 202, 222, 242, 252 having a size of about 0.01 mm² are regularly formed in the stimulable phosphor sheet 10, 80, 110, 120, 140, 180, 200, 220, 240, 250 at a density of about 5,000 per cm² in the same pattern as that of the absorptive regions 4, 104, 134, 194, 234 formed in the biochemical analysis unit 1, 100, 130, 190, 230, the number or size of the stimulable phosphor layer regions 12, 82, 115, 125, 142, 182, 202, 222, 242, 252 may be arbitrarily selected in accordance with the purpose. Preferably, 10 or more of the stimulable phosphor layer regions 12, 82, 115, 125, 142, 182, 202, 222, 242, 252 having a size of 5 mm² or less are formed in the stimulable phosphor sheet 10, 80, 110, 120, 140, 180, 200, 220, 240, 250 at a density of 10/ cm² or greater.

Further, in the above described embodiments, although about 10,000 substantially circular stimulable phosphor layer regions 12, 82, 115, 125, 142, 182, 202, 222, 242, 252 having a size of about 0.01 mm² are regularly formed in the stimulable phosphor sheet 10, 80, 110, 120, 140, 180, 200, 220, 240, 250 at a density of about 5,000 per cm² in the same pattern as that of the absorptive regions 4, 104, 134, 194, 234 formed in the biochemical analysis unit 1, 100, 130, 190, 230, it is not absolutely necessary to form the absorptive regions 4, 104, 134, 194, 234 of the biochemical analysis unit 1, 100, 130, 190, 230 in a regular pattern. Therefore, it is not absolutely necessary to form the stimulable phosphor layer regions 12, 82, 115, 125, 142, 182, 202, 222, 242, 252 in the stimulable phosphor sheet 10, 80, 110, 120, 140, 180, 200, 220, 240, 250 in a regular pattern and it is sufficient for the stimulable phosphor layer regions 12, 82, 115, 125, 142, 182, 202, 222, 242, 252 to be formed in the stimulable phosphor sheet 10, 80, 110, 120, 140, 180, 200, 220, 240, 250 in the same pattern as that of the absorptive regions 4, 104, 134, 194, 234 formed in the biochemical analysis unit 1, 100, 130, 190, 230.

Furthermore, in the above described embodiments, although each of the stimulable phosphor layer regions 12, 82, 115, 125, 142, 182, 202, 222, 242, 252 of the stimulable phosphor sheet 10, 80, 110, 120, 140, 180, 200, 220, 240, 250 is formed so as to have the same size as that of each of the absorptive regions 4, 104, 134, 194, 234 of the biochemical analysis unit 1, 100, 130, 190, 230, it is not absolutely necessary to form each of the stimulable phosphor layer regions 12, 82, 115, 125, 142, 182, 202, 222, 242, 252 in the stimulable phosphor sheet 10, 80, 110, 120, 140, 180, 200, 220, 240, 250 so as to have the same size as that of each of the absorptive regions 4, 104, 134, 194, 234 of the biochemical analysis unit 1, 100, 130, 190, 230 and each of the stimulable phosphor layer regions 12, 82, 115, 125, 142, 182, 202, 222, 242, 252 is preferably formed in the stimulable phosphor sheet 10, 80, 110, 120, 140, 180, 200, 220, 240, 250 so as to have a size equal to or lager than that of each of the absorptive regions 4, 104, 134, 194, 234 of the biochemical analysis unit 1, 100, 130, 190, 230.

Moreover, in the above described embodiments, as specific binding substances, cDNAs each of which has a known base sequence and is different from the others are used. However, specific binding substances usable in the present invention are not limited to cDNAs but all specific binding substances capable of specifically binding with a substance derived from a living organism such as a cell, virus, hormone, tumor marker, enzyme, antibody, antigen, abzyme, other protein, a nuclear acid, cDNA, DNA, RNA or the like and whose sequence, base length, composition and the like are known, can be employed in the present invention as a specific binding substance.

Further, the biochemical analysis unit 1, 130, 190, 230 includes a number of the absorptive regions 4, 134, 194, 234 formed by charging nylon-6 in a number of the through-holes 3, 133, 193, 233 formed in the substrate 2, 131, 191, 231 made of stainless steel in the embodiments shown in Figures 1, 25, 30 and 34 and the biochemical analysis unit 100 includes a number of the absorptive regions 104 formed by pressing the absorptive membrane 103 formed of nylon-6 into a number of the through-holes 102 formed in the substrate 101 made of stainless steel in the embodiment shown in Figure 21. However, it is not absolutely necessary to form a number of the absorptive regions 4, 104, 134, 194, 234 of the biochemical analysis unit 1, 100, 130, 190, 230 of nylon-6 but a number of the absorptive regions 4, 104, 134, 194, 234 of the biochemical analysis unit 1, 100, 130, 190, 230 may be formed of other absorptive material. A porous material or a fiber material may be preferably used as the absorptive material for forming a number of the absorptive regions 4, 104, 134, 194, 234 of the biochemical analysis unit 1, 100, 130, 190, 230 and a number of the absorptive regions 4, 104, 134, 194, 234 of the biochemical analysis unit 1, 100, 130, 190, 230 may be formed by combining a porous material and a fiber material. A porous material for forming a number of the absorptive regions 4, 104, 134, 194, 234 of the biochemical analysis unit 1, 100, 130, 190, 230 may be any type of an organic material or an inorganic material and may be an organic/inorganic composite material. An organic porous material used for forming a number of the absorptive regions 4, 104, 134, 194, 234 of the biochemical analysis unit 1, 100, 130, 190, 230 is not particularly limited but a carbon porous material such as an activated carbon or a porous material capable of forming a membrane filter can be preferably used. Illustrative examples of porous materials capable of forming a membrane filter include nylons such as nylon-6, nylon-6,6, nylon-4,10; cellulose derivatives such as nitrocellulose, acetyl cellulose, butyric-acetyl cellulose; collagen; alginic acids such as alginic acid, calcium alginate, alginic acid/poly-L-lysine polyionic complex; polyolefins such as polyethylene, polypropylene; polyvinyl chloride; polyvinylidene chloride; polyfluoride such as polyvinylidene fluoride, polytetrafluoride; and copolymers or composite materials thereof. An inorganic porous material used for forming a number of the absorptive regions 4, 104, 134, 194, 234 of the biochemical analysis unit 1, 100, 130, 190, 230 is not particularly limited. Illustrative examples of inorganic porous materials preferably usable in the present invention include metals such as platinum, gold, iron, silver, nickel, aluminum and the like; metal oxides such as alumina, silica, titania, zeolite and the like; metal salts such as hydroxy apatite, calcium sulfate and the like; and composite materials thereof. A fiber material used for forming a number of the absorptive regions 4, 104, 134, 194, 234 of the biochemical analysis unit 1, 100, 130, 190, 230 is not particularly limited. Illustrative examples of fiber materials preferably usable in the present invention include nylons such as nylon-6, nylon-6,6, nylon-4,10; and cellulose derivatives such as nitrocellulose, acetyl cellulose, butyric-acetyl cellulose.

Furthermore, although a number of the absorptive regions 104 of the biochemical analysis unit 100 are formed by pressing the absorptive membrane 103 formed of nylon-6 into a number of the through-holes 102 formed in the substrate 101 made of stainless steel using the calender processing apparatus in the embodiment shown in Figure 21, it is possible to press the absorptive membrane 103 into a number of the through-holes 102 formed in the substrate 101 by other means such as a heat press apparatus. Further, a number of the absorptive regions 104 may be formed by charging the absorptive membrane 103 into a number of the through-holes 102 formed in the substrate 101 by an appropriate method other than pressing.

Moreover, although a number of the absorptive regions 104 of the biochemical analysis unit 100 are formed by pressing the absorptive membrane 103 formed of nylon-6 into a number of the through-holes 102 formed in the substrate 101 made of stainless steel, the absorptive membrane 103 may be pressed into a number of the through-holes 102 formed in the substrate 101 via an adhesive agent for improving the strength of the biochemical analysis unit 100.

Further, in the above described embodiments, although the substrate 2, 101, 131, 191, 231 of the biochemical analysis unit 1, 100, 130, 190, 230 is made of stainless steel, it is not absolutely necessary to make the substrate 2, 101, 131, 191, 231 of the biochemical analysis unit 1, 100, 130, 190, 230 of stainless steel but the substrate 2, 101, 131, 191, 231 of the biochemical analysis unit 1, 100, 130, 190, 230 may be made of other kinds of material. The substrate 2, 101, 131, 191, 231 of the biochemical analysis unit 1, 100, 130, 190, 230 is preferably made of material capable of attenuating radiation energy and/or light energy but the material for forming the substrate 2, 101, 131, 191, 231 of the biochemical analysis unit 1, 100, 130, 190, 230 is not particularly limited. The substrate 2, 101, 131, 191, 231 of the biochemical analysis unit 1, 100, 130, 190, 230 can be formed of either inorganic compound material or organic compound material and is preferably formed of a metal material, a ceramic material or a plastic material. Illustrative examples of inorganic compound materials usable for forming the substrate 2, 101, 131, 191, 231 of the biochemical analysis unit 1, 100, 130, 190, 230 and capable of attenuating radiation energy and/or light energy include metals such as gold, silver, copper, zinc, aluminum, titanium, tantalum, chromium, steel, nickel, cobalt, lead, tin, selenium and the like; alloys such as brass, stainless, bronze and the like; silicon materials such as silicon, amorphous silicon, glass, quartz, silicon carbide, silicon nitride and the like; metal oxides such as aluminum oxide, magnesium oxide, zirconium oxide and the like; and inorganic salts such as tungsten carbide, calcium carbide, calcium sulfate, hydroxy apatite, gallium arsenide and the like. High molecular compounds are preferably used as organic compound material for forming the substrate 2, 101, 131, 191, 231 of the biochemical analysis unit 1, 100, 130, 190, 230 and capable of attenuating radiation energy and/or light energy and illustrative examples thereof include polyolefins such as polyethylene, polypropylene and the like; acrylic resins such as polymethyl methacrylate, polybutylacrylate/polymethyl methacrylate copolymer and the like; polyacrylonitrile; polyvinyl chloride; polyvinylidene chloride; polyvinylidene fluoride; polytetrafluoroethylene; polychlorotrifluoroethylene; polycarbonate; polyesters such as polyethylene naphthalate, polyethylene terephthalate and the like; nylons such as nylon-6, nylon-6,6, nylon-4, 10 and the like; polyimide; polysulfone; polyphenylene sulfide; silicon resins such as polydiphenyl siloxane and the like; phenol resins such as novolac and the like; epoxy resin; polyurethane; polystyrene, butadiene-styrene copolymer; polysaccharides such as cellulose, acetyl cellulose, nitrocellulose, starch, calcium alginate, hydroxypropyl methyl cellulose and the like; chitin; chitosan; urushi (Japanese lacquer); polyamides such as gelatin, collagen, keratin and the like; and copolymers of these high molecular materials. These may be a composite compound, and metal oxide particles, glass fiber or the like may be added thereto as occasion demands. Further, an organic compound material may be blended therewith.

Furthermore, although a number of the absorptive regions 4, 134, 194, 234 of the biochemical analysis unit 1, 130, 190, 230 are formed by charging nylon-6 in a number of the through-holes 3, 133, 193, 233 formed in the substrate 2, 131, 191, 231 made of stainless steel in the embodiments shown in Figures 1, 25, 30 and 34 and a number of the absorptive regions 104 of the biochemical analysis unit 100 are formed by pressing the absorptive membrane 103 formed of nylon-6 into a number of the through-holes 102 formed in the substrate 101 made of stainless steel in the embodiment shown in Figure 21, a number of absorptive regions of a biochemical analysis unit may be formed to be spaced apart from each other by closely contacting a perforated plate formed with a number of through-holes onto one surface of an absorptive substrate.

Moreover, although a number of the absorptive regions 4, 194, 234 of the biochemical analysis unit 1, 190, 230 are formed by charging nylon-6 in a number of the through-holes 3, 193, 233 formed in the substrate 2, 191, 231, it is possible to form a number of recesses in the substrate 2, 191, 231 instead of the through-holes 3, 193, 233 and to form a number of absorptive regions by charging nylon-6 in a number of the recesses formed in the substrate 2, 191, 231.

Further, a number of the stimulable phosphor layer regions 12, 82, 142, 182, 202, 222, 242, 252 of the stimulable phosphor sheet 10, 80, 140, 180, 200, 220, 240, 250 are formed by charging stimulable phosphor in a number of the through-holes 13, 143, 203, 243 formed in the support 11, 141, 201, 241 made of stainless steel in the embodiments shown in Figures 4, 15, 26, 29, 31, 33, 35 and 37 and a number of the stimulable phosphor layer regions 115, 125 of the stimulable phosphor sheet 110, 120 are formed by pressing the stimulable phosphor membrane 111, 121 into a number of the through-holes 112 formed in the support 113 made of stainless steel in the embodiments shown in Figures 22 and 24. However, it is not absolutely necessary to make the support 11, 113, 141, 201, 241 of the stimulable phosphor sheet 10, 80, 110, 120, 140, 180, 200, 220, 240, 250 of stainless steel but the support 11, 113, 141, 201, 241 of the stimulable phosphor sheet 10, 80, 110, 120, 140, 180, 200, 220, 240, 250 can be made of other material. The support 11, 113, 141, 201, 241 of the stimulable phosphor sheet 10, 80, 110, 120, 140, 180, 200, 220, 240, 250 is preferably made of material capable of attenuating radiation energy and/or light energy but the material for forming the support 11, 113, 141, 201, 241 of the stimulable phosphor sheet 10, 80, 110, 120, 140, 180, 200, 220, 240, 250 is not particularly limited. The support 11, 113, 141, 201, 241 of the stimulable phosphor sheet 10, 80, 110, 120, 140, 180, 200, 220, 240, 250 can be formed of either inorganic compound material or organic compound material and is preferably formed of a metal material, a ceramic material or a plastic material. Illustrative examples of inorganic compound materials preferably usable for forming the support 11, 113, 141, 201, 241 of the stimulable phosphor sheet 10, 80, 110, 120, 140, 180, 200, 220, 240, 250 include metals such as gold, silver, copper, zinc, aluminum, titanium, tantalum, chromium, steel, nickel, cobalt, lead, tin, selenium and the like; alloys such as brass, stainless, bronze and the like; silicon materials such as silicon, amorphous silicon, glass, quartz, silicon carbide, silicon nitride and the like; metal oxides such as aluminum oxide, magnesium oxide, zirconium oxide and the like; and inorganic salts such as tungsten carbide, calcium carbide, calcium sulfate, hydroxy apatite, gallium arsenide and the like. High molecular compounds are preferably used as organic compound material usable for forming the support 11, 113, 141, 201, 241 of the stimulable phosphor sheet 10, 80, 110, 120, 140, 180, 200, 220, 240, 250 and illustrative examples thereof include polyolefins such as polyethylene, polypropylene and the like; acrylic resins such as polymethyl methacrylate, polybutylacrylate/polymethyl methacrylate copolymer and the like; polyacrylonitrile; polyvinyl chloride; polyvinylidene chloride; polyvinylidene fluoride; polytetrafluoroethylene; polychlorotrifluoroethylene; polycarbonate; polyesters such as polyethylene naphthalate, polyethylene terephthalate and the like; nylons such as nylon-6, nylon-6,6, nylon-4,10 and the like; polyimide; polysulfone; polyphenylene sulfide; silicon resins such as polydiphenyl siloxane and the like; phenol resins such as novolac and the like; epoxy resin; polyurethane; polystyrene, butadiene-styrene copolymer; polysaccharides such as cellulose, acetyl cellulose, nitrocellulose, starch, calcium alginate, hydroxypropyl methyl cellulose and the like; chitin; chitosan; urushi (Japanese lacquer); polyamides such as gelatin, collagen, keratin and the like; and copolymers of these high molecular materials. These may be a composite compound, and metal oxide particles, glass fiber or the like may be added thereto as occasion demands. Further, an organic compound material may be blended therewith.

Furthermore, although a number of the stimulable phosphor layer regions 115, 125 of the stimulable phosphor sheet 110, 120 are formed by pressing the stimulable phosphor membrane 111, 121 into a number of the through-holes 112 formed in the support 113 made of stainless steel using the calender processing apparatus in the embodiments shown in Figures 22 and 24, it is possible to press the stimulable phosphor membrane 111, 121 into a number of the through-holes 112 formed in the support 113 by other means such as a heat press apparatus. Further, a number of the stimulable phosphor layer regions 115, 125 may be formed by charging the stimulable phosphor membrane 111, 121 into a number of the through-holes 112 formed in the support 113 by an appropriate method other than pressing.

Moreover, although a number of the stimulable phosphor layer regions 115, 125 of the stimulable phosphor sheet 110, 120 are formed by pressing the stimulable phosphor membrane 111, 121 into a number of the through-holes 112 formed in the support 113 made of stainless steel in the embodiments shown in Figures 22 and 24, the stimulable phosphor membrane 111, 121 may be pressed into a number of the through-holes 112 formed in the support 113 via an adhesive agent for improving the strength of the stimulable phosphor sheet 110, 120.

Further, although a number of the stimulable phosphor layer regions 12, 82, 202, 222, 242, 252 of the stimulable phosphor sheet 10, 80, 200, 220, 240, 250 are formed by charging stimulable phosphor in a number of the through-holes 13, 203, 243 formed in the support 11, 201. 241 in the embodiments shown in Figures 4, 15, 31, 33, 35 and 37, it is possible to form a number of recesses in the support 11, 201. 241 instead of the through-holes 13, 203, 243 and to form a number of stimulable phosphor layer regions by charging stimulable phosphor in a number of the recesses formed in the support 11, 201, 241.

Moreover, in the embodiments, a hybridization reaction solution 9 containing a substance derived from a living organism and labeled with a radioactive labeling substance, a substance derived from a living organism and labeled with a labeling substance which generates chemiluminescence emission when it contacts a chemiluminescent substrate and a substance derived from a living organism and labeled with a fluorescent substance such as a fluorescent dye is prepared. However, it is not absolutely necessary for the hybridization reaction solution 9 to contain a substance derived from a living organism and labeled with a radioactive labeling substance, a substance derived from a living organism and labeled with a labeling substance which generates chemiluminescence emission when it contacts a chemiluminescent substrate and a substance derived from a living organism and labeled with a fluorescent substance such as a fluorescent dye but it is sufficient for the hybridization reaction solution 9 to contain at least one of a substance derived from a living organism and labeled with a radioactive labeling substance and a substance derived from a living organism and labeled with a labeling substance which generates chemiluminescence emission when it contacts a chemiluminescent substrate.

Further, in the above described embodiments, specific binding substances are hybridized with substances derived from a living organism and labeled with a radioactive labeling substance, a substance derived from a living organism and labeled with a labeling substance which generates chemiluminescence emission when it contacts a chemiluminescent substrate and a fluorescent substance. However, it is not absolutely necessary to hybridize substances derived from a living organism with specific binding substances and substances derived from a living organism may be specifically bound with specific binding substances by means of antigen-antibody reaction, receptor-ligand reaction or the like instead of hybridization.

Furthermore, the biochemical analysis unit 1 formed with the two positioning through-holes 5, 5 and the stimulable phosphor sheet 10, 80 formed with two positioning through-holes 15, 15 are positioned using the two erect positioning pins 19, 19 formed on the base plate 18 of the exposure device, thereby exposing a number of the stimulable phosphor layer regions 12, 82 of the stimulable phosphor sheet 10, 80 in the embodiments shown in Figures 1 to 18 and the biochemical analysis unit 1 formed with the two positioning through-holes 5, 5 and the stimulable phosphor sheet 10, 80 formed with two positioning through-holes 15, 15 are positioned using the photo-sensor 77 provided in the exposure device, thereby exposing a number of the stimulable phosphor layer regions 12, 82 of the stimulable phosphor sheet 10, 80 in the embodiments shown in Figures 19 and 20. However, it is possible to produce images of the biochemical analysis unit 1 formed with the two positioning through-holes 5, 5 and the stimulable phosphor sheet 10, 80 formed with two positioning through-holes 15, 15 using the CCD area sensor 150 shown in Figures 19 and 20 and to position the biochemical analysis unit 1 and the stimulable phosphor sheet 10, 80 based on the thus produced images.

Moreover, in the embodiments shown in Figures 19 and 20, when the level of signal intensity level of light detected by the photo-sensor 77 is equal to or higher than the threshold value, it is judged that the two positioning through-holes 5, 5 formed in the substrate 2 of the biochemical analysis unit 1 and the two positioning through-holes 15, 15 formed in the support 11 of the stimulable phosphor sheet 10, 80 have completely aligned with each other. However, it is possible to form the two positioning through-holes 5, 5 and the two positioning through-holes 15, 15 sufficiently small and judge that the two positioning through-holes 5, 5 formed in the substrate 2 of the biochemical analysis unit 1 and the two positioning through-holes 15, 15 formed in the support 11 of the stimulable phosphor sheet 10, 80 have completely aligned with each other, when the photo-sensor 77 detects light emitted from the fluorescent lamp 76.

Further, in the embodiments shown in Figures 1 to 18, although the two positioning through-holes 5, 5 are formed in the substrate 2 of the biochemical analysis unit 1 and the two positioning through-holes 15, 15 are formed in the support 11 of the stimulable phosphor sheet 10, 80, it is possible to form three or more positioning through-holes 5 in the substrate 2 of the biochemical analysis unit 1, form three or more positioning through-holes 15 in the support 11 of the stimulable phosphor sheet 10, 80 and form positioning pins 19 in a number corresponding to the number of the positioning through-holes 5 formed in the substrate 2 of the biochemical analysis unit 1 and the number of the positioning through-holes 15 formed in the support 11 of the stimulable phosphor sheet 10, 80 on the base plate 18 of the exposure device.

Furthermore, in the embodiments shown in Figures 21 to 24, the biochemical analysis unit 100 formed with the two positioning notches 105, 105 and the stimulable phosphor sheet 110, 120 formed with two positioning notches 116, 116 are positioned using the two erect positioning pins 19, 19 formed on the base plate 18 of the exposure device, thereby exposing a number of the stimulable phosphor layer regions 115, 125 of the stimulable phosphor sheet 110, 120. However, it is possible to position the biochemical analysis unit 100 and the stimulable phosphor sheet 110, 120 by abutting the side portion of the biochemical analysis unit 100 formed with the two positioning notches 105, 105 and the side portion of the stimulable phosphor sheet 110, 120 formed with the two positioning notches 116, 116 against the inner surface of the exposure device shown in Figure 19 on the side of the fluorescent lamp 76 and detecting light emitted from the fluorescent lamp 76 with the photo-sensor 77 and it is further possible to produce images of the biochemical analysis unit 100 and the stimulable phosphor sheet 110, 120 using the CCD area sensor 150 shown in Figure 27 and to position the biochemical analysis unit 100 and the stimulable phosphor sheet 110, 120 based on the thus produced images.

Moreover, in the embodiments shown in Figures 21 to 24, although the two positioning notches 105, 105 are formed in the substrate 101 of the biochemical analysis unit 100 and the two positioning notches 116, 116 are formed in the support 111 of the stimulable phosphor sheet 110, 120, it is possible to form three or more positioning notches 105 in the substrate 101 of the biochemical analysis unit 100, form three or more positioning notches 116 in the support 111 of the stimulable phosphor sheet 110, 120 and form positioning pins 19 in a number corresponding to the number of the positioning notches 105 formed in the substrate 101 of the biochemical analysis unit 100 and the number of the positioning notches 116 formed in the support 111 of the stimulable phosphor sheet 110, 120 on the base plate 18 of the exposure device.

Further, in the embodiments shown in Figures 25 to 29, images of the biochemical analysis unit 130 including the two through-holes 133a and 133b in which no nylon-6 is charged and the stimulable phosphor sheet 140, 180 including the two through-holes 143a and 143b in which no stimulable phosphor is charged are produced using the CCD area sensor 150 and the biochemical analysis unit 130 and the stimulable phosphor sheet 140, 180 are positioned. However, it is possible to position the biochemical analysis unit 130 and the stimulable phosphor sheet 140, 180 by inserting the two erect positioning pins 19, 19 formed on the base plate 18 of the exposure device shown in Figure 5 into the two through-holes 133a and 133b of the biochemical analysis unit 130 in which no nylon-6 is charged and the two through-holes 143a and 143b of the stimulable phosphor sheet 140, 180 in which no stimulable phosphor is charged and it is further possible to position the biochemical analysis unit 130 and the stimulable phosphor sheet 140, 180 using the photo-sensor 77 shown in Figure 20.

Furthermore, in the embodiments shown in Figures 25 to 29, although the biochemical analysis unit 130 is formed with the two through-holes 133a and 133b in which no nylon-6 is charged and the stimulable phosphor sheet 140, 180 is formed with the two through-holes 143a and 143b in which no stimulable phosphor is charged, three or more through-holes in which no nylon-6 is charged may be formed in the biochemical analysis unit 130 and three or more through-holes in which no stimulable phosphor is charged may be formed in the stimulable phosphor sheet 140, 180.

Moreover, in the embodiments shown in Figures 30 to 33, although the biochemical analysis unit 190 and the stimulable phosphor sheet 200, 220 are positioned using the first reference pin 211, the second reference pin 212 and the third reference pin 213, it is possible to produce images of the biochemical analysis unit 190 and the stimulable phosphor sheet 200, 220 using the CCD area sensor 150 shown in Figure 27 and to position the biochemical analysis unit 190 and the stimulable phosphor sheet 200, 220 based on the thus produced images. In this case, it is sufficient for a number of the absorptive regions 194 of the biochemical analysis unit 190 and a number of the stimulable phosphor layer regions 202, 222 of the stimulable phosphor sheet 200, 220 to be formed in the same pattern and it is not absolutely necessary to form each of a number of the absorptive regions 194 in the substrate 191 of the biochemical analysis unit 190 in a predetermined positional relationship to the first reference edge 191a of the biochemical analysis unti 190 and the second reference edge 191b of the substrate 191 perpendicular to the first reference edge 191a and to form each of a number of the stimulable phosphor layer regions 202, 222 in the support 201 of the stimulable phosphor sheet 200, 220 in the same positional relationship to the first reference edge 201a and the second reference edge 201b of the support 201 of the stimulable phosphor sheet 200 as that of a number of the absorptive regions 194 to the first reference edge 191a of the substrate 191 of the biochemical analysis unit 190 and the second reference edge 191b of the substrate 191 perpendicular to the first reference edge 191a.

Further, in the embodiments shown in Figures 34 to 37, although the biochemical analysis unit 230 and the stimulable phosphor sheet 240, 250 are positioned by forming the recess 235 in the substrate 231 of the biochemical analysis unit 230, forming the convex portion 245 in the support 241 of the stimulable phosphor sheet 240, 250 at a position corresponding to the recess 235 of the biochemical analysis unit 230 so as to have a complementary shape to that of the recess 235 and fitting the convex portion 245 formed in the support 241 of the stimulable phosphor sheet 240, 250 into the recess 235 formed in the substrate 231 of the biochemical analysis unit 230, the biochemical analysis unit 230 and the stimulable phosphor sheet 240, 250 may be positioned by forming a convex portion in the substrate 231 of the biochemical analysis unit 230, forming a recess in the support 241 of the stimulable phosphor sheet 240, 250 at a position corresponding to the convex portion of the biochemical analysis unit 230 so as to have a complementary shape to that of the convex portion and fitting the convex portion formed in the substrate 231 of the biochemical analysis unit 230 into the recess formed in the support 241 of the stimulable phosphor sheet 240, 250.

Furthermore, in the embodiments shown in Figures 34 to 37, although the biochemical analysis unit 230 and the stimulable phosphor sheet 240, 250 are positioned by forming the recess 235 extending in parallel with one edge portion of the substrate 231 in the substrate 231 of the biochemical analysis unit 230, forming the convex portion 245 in the support 241 of the stimulable phosphor sheet 240, 250 at a position corresponding to the recess 235 of the biochemical analysis unit 230 so as to have a complementary shape to that of the recess 235 and fitting the convex portion 245 formed in the support 241 of the stimulable phosphor sheet 240, 250 into the recess 235 formed in the substrate 231 of the biochemical analysis unit 230, it is not absolutely necessary to form the recess 235 in the substrate 231 of the biochemical analysis unit 230 in parallel with one edge portion of the substrate 231 and it is possible to form a recess 235 having an arbitrary shape in the substrate 231 of the biochemical analysis unit 230 and a convex portion 245 having the corresponding shape to that of the recess 235 in the support 241 of the stimulable phosphor sheet 240, 250. In the case where a recess is formed circular in the substrate 231 of the biochemical analysis unit 230, two or more recesses 235 are formed in the substrate 231 of the biochemical analysis unit 230 and two or more convex portions 245 are formed in the support 241 of the stimulable phosphor sheet 240, 250.

Further, in the above described embodiments, although the stimulable phosphor sheet 10, 80, 110, 120, 140, 180, 200, 220, 240, 250 are superposed on the biochemical analysis unit 1, 100, 130, 190, 230, it is possible superpose the biochemical analysis unit 1, 100, 130, 190, 230 on the stimulable phosphor sheet 10, 80, 110, 120, 140, 180, 200, 220, 240, 250.

Moreover, in the above described embodiments, the scanner shown in Figures 7 to 14 is constituted so as to read radiation data of a radioactive labeling substance recorded in a number of the stimulable phosphor layer regions 12 formed in the support 11 of the stimulable phosphor sheet 10 and fluorescence data of a fluorescent substance such as a fluorescent dye recorded in a number of absorptive regions 4 formed in the substrate 2 of the biochemical analysis unit 1, thereby producing biochemical analysis data and includes the first laser stimulating ray source 21 for emitting a laser beam 24 having a wavelength of 640 nm, the second laser stimulating ray source 22 for emitting a laser beam 24 having a wavelength of 532 nm and the third laser stimulating ray source 23 for emitting a laser beam 24 having a wavelength of 473 nm. However, it is not absolutely necessary to read radiation data of a radioactive labeling substance and fluorescence data of a fluorescent substance by a single scanner but radiation data of a radioactive labeling substance and fluorescence data of a fluorescent substance may be read by separate scanners to produce biochemical analysis data. Therefore, it is not absolutely necessary for the scanner to include three laser stimulating ray sources.

Further, in the above described embodiments, the on and off operation of the first laser stimulating ray source 21, the second laser stimulating ray source 22 or the fourth laser stimulating ray source 85 is controlled by the control unit 70 in synchronism with the intermittent movement of the optical head 35. However, if the moving speed of the optical head 35 is determined so that the laser beam 24 quickly passes portions between neighboring stimulable phosphor layer regions 12, 82 of the stimulable phosphor sheet 10, 80 or neighboring absorptive regions 4 of the biochemical analysis unit 1 in the main scanning direction, biochemical analysis data may be produced by merely intermittently moving the optical head 35 while the first laser stimulating ray source 21, the fourth laser stimulating ray source 85 or the second laser stimulating ray source 22 is kept on, thereby sequentially scanning a number of the stimulable phosphor layer regions 12, 82 of the stimulable phosphor sheet 10, 80 or a number of the absorptive regions 4 of the biochemical analysis unit 1 with the laser beam 24 and photoelectrically detecting stimulated emission released from the stimulable phosphor layer regions 12, 82 or fluorescence emission released from the absorptive regions 4.

Furthermore, in the above described embodiments, the scanner shown in Figures 7 to 14 includes the first laser stimulating ray source 21 for emitting a laser beam 24 having a wavelength of 640 nm, the second laser stimulating ray source 22 for emitting a laser beam 24 having a wavelength of 532 nm and the third laser stimulating ray source 23 for emitting a laser beam 24 having a wavelength of 473 nm, and the scanner shown in Figures 16 to 18 includes the first laser stimulating ray source 21 for emitting a laser beam 24 having a wavelength of 640 nm, the second laser stimulating ray source 22 for emitting a laser beam 24 having a wavelength of 532 nm and the fourth laser stimulating ray source 85 for emitting a laser beam 24 having a wavelength of 980 nm. However, it is not absolutely necessary to employ a laser stimulating ray source as a stimulating ray source and an LED (light emitting diode) light source may be employed as a stimulating ray source instead of a laser stimulating ray source. Further, it is possible to employ a halogen lamp as a stimulating ray source and to provide a spectral filter to cut wavelength components which cannot contribute to the excitation.

Moreover, in the above described embodiments, the scanner shown in Figures 7 to 14 and the scanner shown in Figures 16 to 18 are constituted so that all of the stimulable phosphor layer regions 12, 82 formed in the support 11 of the stimulable phosphor sheet 10, 80 or all of the absorptive regions 4 formed in the substrate 2 of the biochemical analysis unit 1 are scanned with a laser beam 24 to excite stimulable phosphor or a fluorescent substance such as a fluorescent dye by moving the optical head 35 using a scanning mechanism in the main scanning direction indicated by the arrow X direction and the sub-scanning direction indicated by the arrow Y in Figure 13. However, all of the stimulable phosphor layer regions 12, 82 formed in the support 11 of the stimulable phosphor sheet 10, 80 or all of the absorptive regions 4 formed in the substrate 2 of the biochemical analysis unit 1 may be scanned with a laser beam 24 to excite stimulable phosphor or a fluorescent substance such as a fluorescent dye by moving the stage 40 in the main scanning direction indicated by the arrow X direction and the sub-scanning direction indicated by the arrow Y in Figure 13, while holding the optical head 35 stationary, or moving the optical head 35 in the main scanning direction indicated by the arrow X direction or the sub-scanning direction indicated by the arrow Y in Figure 13 and moving the stage 40 in the sub-scanning direction indicated by the arrow Y or the main scanning direction indicated by the arrow X in Figure 12.

Further, in the above described embodiments, the scanner shown in Figures 7 to 14 and the scanner shown in Figures 16 to 18 are constituted so as to photoelectrically detect stimulated emission and fluorescence emission are detected using the photomultiplier 50 as a light detector. However, it is sufficient for the light detector used in the present invention to be able to photoelectrically detect fluorescence emission or stimulated emission and it is possible to employ a light detector such as a line CCD or a two-dimensional CCD instead of the photomultiplier 50.

Furthermore, in the above-described embodiments, a solution containing specific binding substances such as cDNAs are spotted using the spotting device including an injector 6 and a CCD camera 7 so that when the tip end portion of the injector 6 and the center of the absorptive region 4, 85 into which a solution containing specific binding substances is to be spotted are determined to coincide with each other as a result of viewing them using the CCD camera 7, the solution containing the specific binding substances such as cDNA is spotted from the injector 6. However, the solution containing specific binding substances such as cDNAs can be spotted by detecting the positional relationship between a number of the absorptive regions 4, 84 formed in the biochemical analysis unit 1, 80 and the tip end portion of the injector 6 in advance and two-dimensionally moving the biochemical analysis unit 1, 80 or the tip end portion of the injector 6 so that the tip end portion of the injector 6 coincides with each of the absorptive regions 4, 84.

According to the present invention, it is possible to provide a biochemical analysis kit and a method for exposing a stimulable phosphor sheet, which can prevent noise caused by the scattering of electron beams (β rays) released from a radioactive labeling substance selectively contained in a plurality of spot-like regions of a biochemical analysis unit from being generated in biochemical analysis data and produce biochemical analysis data having an excellent quantitative characteristic by reading radiation data with high resolution even in the case of forming a plurality of spot-like regions containing specific binding substances, which can specifically bind with a substance derived from a living organism and whose sequence, base length, composition and the like are known, in the biochemical analysis unit at a high density, selectively labeling the plurality of spot-like regions of the biochemical analysis unit with a radioactive labeling substance to record radiation data therein, facing the thus prepared biochemical analysis unit toward a stimulable phosphor layer of a stimulable phosphor sheet to expose the stimulable phosphor layer to a radioactive labeling substance, irradiating the thus exposed stimulable phosphor layer with a stimulating ray, and photoelectrically detecting stimulated emission released from the stimulable phosphor layer to produce biochemical analysis data, and can also prevent noise caused by the scattering of chemiluminescence emission selectively released from a plurality of spot-like regions of a biochemical analysis unit from being generated in biochemical analysis data and produce biochemical analysis data having an excellent quantitative characteristic by reading radiation data with high resolution even in the case of forming a plurality of spot-like regions containing specific binding substances, which can specifically bind with a substance derived from a living organism and whose sequence, base length, composition and the like are known, in the biochemical analysis unit at a high density, selectively labeling the plurality of spot-like regions of the biochemical analysis unit with a labeling substance which generates chemiluminescence emission when it contacts a chemiluminescent substrate to record chemiluminescence data therein, bringing the plurality of spot-like regions of the biochemical analysis unit into contact with a chemiluminescent substrate, thereby causing the plurality of spot-like regions of the biochemical analysis unit to release chemiluminescence emission, facing the biochemical analysis unit releasing chemiluminescence emission toward a stimulable phosphor layer of a stimulable phosphor sheet to expose the stimulable phosphor layer to chemiluminescence emission, irradiating the thus exposed stimulable phosphor layer with a stimulating ray, and photoelectrically detecting stimulated emission released from the stimulable phosphor layer to produce biochemical analysis data.

## Claims

1. A biochemical analysis kit comprising a biochemical analysis unit including a substrate formed with a plurality of absorptive regions to be spaced apart from each other and a stimulable phosphor sheet including a support formed with a plurality of stimulable phosphor layer regions to be spaced apart from each other in substantially the same pattern as that of the plurality of absorptive regions formed in the substrate of the biochemical analysis unit.

2. A biochemical analysis kit in accordance with Claim 1 wherein the substrate of the biochemical analysis unit is formed with the plurality of absorptive regions in a regular pattern.

3. A biochemical analysis kit in accordance with Claim 1 or 2 wherein the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit.

4. A biochemical analysis kit in accordance with Claim 1 or 2 wherein the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit.

5. A biochemical analysis kit in accordance with Claim 1 or 2 wherein at least one positioning convex portion is formed on the surface of the substrate of the biochemical analysis unit and at least one positioning recess having a complementary shape to that of the at least one positioning convex portion is formed on the surface of the support of the stimulable phosphor sheet at a position corresponding to that of the at least one positioning convex portion.

6. A biochemical analysis kit in accordance with Claim 1 or 2 wherein at least one positioning recess is formed on the surface of the substrate of the biochemical analysis unit and at least one positioning convex portion having a complementary shape to that of the at least one positioning recess is formed on the surface of the support of the stimulable phosphor sheet at a position corresponding to that of the at least one positioning recess.

7. A biochemical analysis kit in accordance with Claim 1 or 2 wherein the plurality of absorptive regions are formed in the substrate of the biochemical analysis unit in a predetermined positional relationship to a first reference edge of the substrate of the biochemical analysis unit and a second reference edge of the substrate of the biochemical analysis unit perpendicular to the first reference edge and the plurality of stimulable phosphor layer regions are formed in the support of the stimulable phosphor sheet in a predetermined positional relationship to a first reference edge of the support of the stimulable phosphor sheet corresponding to the first reference edge of the substrate of the biochemical analysis unit and a second reference edge of the support of the stimulable phosphor sheet corresponding to the substrate of the biochemical analysis unit.

8. A biochemical analysis kit in accordance with any one of Claims 1 to 7 wherein the plurality of absorptive regions are formed by charging an absorptive material in a plurality of holes formed in the substrate of the biochemical analysis unit.

9. A biochemical analysis kit in accordance with Claim 8 wherein the plurality of absorptive regions are formed by charging an absorptive material in a plurality of through-holes formed in the substrate of the biochemical analysis unit.

10. A biochemical analysis kit in accordance with Claim 9 wherein the plurality of absorptive regions are formed by pressing an absorptive membrane containing an absorptive material into the plurality of through-holes formed in the substrate of the biochemical analysis unit.

11. A biochemical analysis kit in accordance with any one of Claims 1 to 10 wherein the plurality of stimulable phosphor layer regions are formed by charging stimulable phosphor in a plurality of holes formed in the support of the stimulable phosphor sheet.

12. A biochemical analysis kit in accordance with Claim 11 wherein the plurality of stimulable phosphor layer regions are formed by charging stimulable phosphor in a plurality of through-holes formed in the support of the stimulable phosphor sheet.

13. A biochemical analysis kit in accordance with Claim 12 wherein the plurality of stimulable phosphor layer regions are formed by pressing a stimulable phosphor membrane containing stimulable phosphor and a binder into the plurality of through-holes formed in the support of the stimulable phosphor sheet.

14. A biochemical analysis kit in accordance with Claim 12 or 13 wherein the at least two positioning through-holes of the biochemical analysis unit are formed by through-holes in which no absorptive material is charged among the plurality of through-holes formed in the substrate of the biochemical analysis unit and the at least two positioning through-holes of the stimulable phosphor sheet are formed by through-holes formed at positions corresponding to the at least two positioning through-holes of the biochemical analysis unit and in which no stimulable phosphor is charged among the plurality of through-holes formed in the support of the stimulable phosphor sheet.

15. A biochemical analysis kit in accordance with any one of Claims 1 to 14 wherein the substrate of the biochemical analysis unit is formed with 10 or more absorptive regions.

16. A biochemical analysis kit in accordance with Claim 15 wherein the substrate of the biochemical analysis unit is formed with 1,000 or more absorptive regions.

17. A biochemical analysis kit in accordance with any one of Claims 1 to 16 wherein each of the plurality of absorptive regions formed in the substrate of the biochemical analysis unit has a size of less than 5 mm².

18. A biochemical analysis kit in accordance with Claim 17 wherein each of the plurality of absorptive regions formed in the substrate of the biochemical analysis unit has a size of less than 0.5 mm².

19. A biochemical analysis kit in accordance with any one of Claims 1 to 18 wherein the plurality of absorptive regions are formed in the substrate of the biochemical analysis unit at a density of 10 or more per cm².

20. A biochemical analysis kit in accordance with Claim 19 wherein the plurality of absorptive regions are formed in the substrate of the biochemical analysis unit at a density of 1,000 or more per cm².

21. A biochemical analysis kit in accordance with any one of Claims 1 to 20 wherein the substrate of the biochemical analysis unit has a property of attenuating radiation energy and/or light energy.

22. A biochemical analysis kit in accordance with Claim 21 wherein the substrate of the biochemical analysis unit has a property of reducing the energy of radiation and/or the energy of light to 1/5 or less when the radiation and or light travels in the substrate by a distance equal to that between neighboring absorptive regions.

23. A biochemical analysis kit in accordance with Claim 22 wherein the substrate of the biochemical analysis unit has a property of reducing the energy of radiation and/or the energy of light to 1/100 or less when the radiation and/or light travels in the substrate by a distance equal to that between neighboring absorptive regions.

24. A biochemical analysis kit in accordance with any one of Claims 1 to 23 wherein the substrate of the biochemical analysis unit is made of a material selected from a group consisting of a metal material, a ceramic material and a plastic material.

25. A biochemical analysis kit in accordance with any one of Claims 1 to 24 wherein the plurality of absorptive regions of the biochemical analysis unit are made of a porous carbon material or a porous material capable of forming a membrane filter.

26. A biochemical analysis kit in accordance with any one of Claims 1 to 24 wherein the plurality of absorptive regions of the biochemical analysis unit are made of a bundle of a plurality of fibers.

27. A biochemical analysis kit in accordance with any one of Claims 1 to 26 wherein specific binding substances whose sequence, base length, composition and the like are known are absorbed in the plurality of absorptive regions of the biochemical analysis unit.

28. A biochemical analysis kit in accordance with any one of Claims 1 to 27 wherein each of the plurality of stimulable phosphor layer regions is formed in the stimulable phosphor sheet to have a size of less than 5 mm².

29. A biochemical analysis kit in accordance with Claim 28 wherein each of the plurality of stimulable phosphor layer regions is formed in the stimulable phosphor sheet to have a size of less than 0.5 mm².

30. A biochemical analysis kit in accordance with any one of Claims 1 to 29 wherein the support of the stimulable phosphor sheet has a property of attenuating radiation energy and/or light energy.

31. A biochemical analysis kit in accordance with Claim 30 wherein the support of the stimulable phosphor sheet has a property of reducing the radiation energy and/or the energy of light to 1/5 or less when the radiation and/or light travels in the support by a distance equal to that between neighboring stimulable phosphor layer regions.

32. A biochemical analysis kit in accordance with Claim 31 wherein the support of the stimulable phosphor sheet has a property of reducing the radiation energy and/or the energy of light to 1/100 or less when the radiation and/or light travels in the support by a distance equal to that between neighboring stimulable phosphor layer regions.

33. A biochemical analysis kit in accordance with any one of Claims 1 to 34 wherein the support of the stimulable phosphor sheet is made of a material selected from a group consisting of a metal material, a ceramic material and a plastic material.

34. A method for exposing a stimulable phosphor sheet comprising the steps of superposing a biochemical analysis unit including a substrate capable of attenuating radiation energy and formed with a plurality of absorptive regions spaced apart from each other and selectively labeled with a radioactive labeling substance and the stimulable phosphor sheet including a support formed with a plurality of stimulable phosphor layer regions spaced apart from each other so that each of the plurality of absorptive regions of the biochemical analysis unit faces the corresponding stimulable layer region of the stimulable phosphor sheet and exposing the plurality of stimulable phosphor layer regions of the stimulable phosphor sheet to the radioactive labeling substance selectively contained in the plurality of absorptive regions of the biochemical analysis unit.

35. A method for exposing a stimulable phosphor sheet in accordance with Claim 34 wherein the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit, and which comprises the steps of superposing the biochemical analysis unit and the stimulable phosphor sheet so that at least two erect positioning pins formed on a substrate of an exposure device at positions corresponding to the at least two positioning through-holes formed in the substrate of the biochemical analysis unit and the at least two positioning through-holes formed in the support of the stimulable phosphor sheet are inserted into the at least two positioning through-holes formed in the substrate of the biochemical analysis unit and the at least two positioning through-holes formed in the support of the stimulable phosphor sheet, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

36. A method for exposing a stimulable phosphor sheet in accordance with Claim 34 wherein the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit, and which comprises the steps of superposing the biochemical analysis unit and the stimulable phosphor sheet, positioning the biochemical analysis unit and the stimulable phosphor sheet by detecting light emitted from a light emitting means and transmitted through the at least two positioning through-holes formed in the substrate of the biochemical analysis unit and the at least two positioning through-holes formed in the support of the stimulable phosphor sheet with a light receiving means, and exposing the plurality of stimulable phosphor layer regions of the stimulable phosphor sheet to a radioactive labeling substrate selectively contained in the plurality of absorptive regions of the biochemical analysis unit.

37. A method for exposing a stimulable phosphor sheet in accordance with Claim 34 wherein the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit, and which comprises the steps of setting one of the biochemical analysis unit and the stimulable phosphor sheet on a substrate of an exposure device, producing an image of the at least two positioning through-holes formed in the one of the biochemical analysis unit and the stimulable phosphor sheet using an imaging device, storing data corresponding to the image in a memory, superposing the other one of the biochemical analysis unit and the stimulable phosphor sheet on the surface of the one of the biochemical analysis unit and the stimulable phosphor sheet, producing an image of the at least two positioning through-holes formed in the other one of the biochemical analysis unit and the stimulable phosphor sheet using the imaging device, comparing the thus produced image with the image of the at least two positioning through-holes formed in the one of the biochemical analysis unit and the stimulable phosphor sheet based on the data stored in the memory, moving the other one of the biochemical analysis unit and the stimulable phosphor sheet so that the at least two positioning through-holes formed in the substrate of the biochemical analysis unit and the at least two positioning through-holes formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

38. A method for exposing a stimulable phosphor sheet in accordance with Claim 34 wherein the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit, and which comprises the steps of superposing the biochemical analysis unit and the stimulable phosphor sheet so that at least two erect positioning pins formed on a substrate of an exposure device at positions corresponding to the at least two positioning notches formed in the substrate of the biochemical analysis unit and the at least two positioning notches formed in the support of the stimulable phosphor sheet are inserted into the at least two positioning notches formed in the substrate of the biochemical analysis unit and the at least two positioning notches formed in the support of the stimulable phosphor sheet, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

39. A method for exposing a stimulable phosphor sheet in accordance with Claim 34 wherein the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit, and which comprises the steps of superposing the biochemical analysis unit and the stimulable phosphor sheet, positioning the biochemical analysis unit and the stimulable phosphor sheet by detecting light emitted from a light emitting means and transmitted through the at least two positioning notches formed in the substrate of the biochemical analysis unit and the at least two positioning notches formed in the support of the stimulable phosphor sheet with a light receiving means, and exposing the plurality of stimulable phosphor layer regions of the stimulable phosphor sheet to a radioactive labeling substrate selectively contained in the plurality of absorptive regions of the biochemical analysis unit.

40. A method for exposing a stimulable phosphor sheet in accordance with Claim 34 wherein the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit, and which comprises the steps of setting one of the biochemical analysis unit and the stimulable phosphor sheet on a substrate of an exposure device, producing an image of the at least two positioning notches formed in the one of the biochemical analysis unit and the stimulable phosphor sheet using an imaging device, storing data corresponding to the image in a memory, superposing the other one of the biochemical analysis unit and the stimulable phosphor sheet on the surface of the one of the biochemical analysis unit and the stimulable phosphor sheet, producing an image of the at least two positioning notches formed in the other one of the biochemical analysis unit and the stimulable phosphor sheet using the imaging device, comparing the thus produced image with the image of the at least two positioning notches formed in the one of the biochemical analysis unit and the stimulable phosphor sheet based on the data stored in the memory, moving the other one of the biochemical analysis unit and the stimulable phosphor sheet so that the at least two positioning notches formed in the substrate of the biochemical analysis unit and the at least two positioning notches formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

41. A method for exposing a stimulable phosphor sheet in accordance with Claim 34 wherein at least one positioning convex portion on the surface of the substrate of the biochemical analysis unit and at least one positioning recess having a complementary shape to that of the at least one positioning convex portion at a position corresponding to that of the at least one positioning convex portion on the surface of the support of the stimulable phosphor sheet, and which comprises the steps of fitting the at least one positioning convex portion formed on the surface of the substrate of the biochemical analysis unit into the at least one positioning recess formed on the surface of the support of the stimulable phosphor sheet, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

42. A method for exposing a stimulable phosphor sheet in accordance with Claim 34 wherein at least one positioning recess on the surface of the substrate of the biochemical analysis unit and at least one positioning convex portion having a complementary shape to that of the at least one positioning recess at a position corresponding to that of the at least one positioning recess on the surface of the support of the stimulable phosphor sheet, and which comprises the steps of fitting the at least one positioning convex portion formed on the surface of the support of the stimulable phosphor sheet into the at least one positioning recess formed on the surface of the substrate of the biochemical analysis unit, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

43. A method for exposing a stimulable phosphor sheet in accordance with Claim 34 wherein the plurality of absorptive regions are formed in the substrate of the biochemical analysis unit in a predetermined positional relationship to a first reference edge of the substrate of the biochemical analysis unit and a second reference edge of the substrate of the biochemical analysis unit perpendicular to the first reference edge and the plurality of stimulable phosphor layer regions are formed in the support of the stimulable phosphor sheet in a predetermined positional relationship to a first reference edge of the support of the stimulable phosphor sheet corresponding to the first reference edge of the substrate of the biochemical analysis unit and a second reference edge of the support of the stimulable phosphor sheet corresponding to the substrate of the biochemical analysis unit, and which comprises the steps of superposing the biochemical analysis unit and the stimulable phosphor sheet so that the first reference edge of the substrate of the biochemical analysis unit and the first reference edge of the support of the stimulable phosphor sheet align with one of two erect reference pins formed on the support of an exposure device and that the second reference edge of the substrate of the biochemical analysis unit and the second reference edge of the support of the stimulable phosphor sheet align with the other one of the two erect reference pins formed on the support of an exposure device, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

44. A method for exposing a stimulable phosphor sheet in accordance with Claim 34 wherein the plurality of absorptive regions of the biochemical analysis unit are formed by charging an absorptive material in a number of through-holes formed in the substrate and the plurality of stimulable phosphor layer regions of the stimulable phosphor sheet are formed by charging stimulable phosphor in a number of through-holes formed in the support so that the at least two positioning through-holes of the biochemical analysis unit are formed by through-holes in which no absorptive material is charged among the plurality of through-holes formed in the substrate of the biochemical analysis unit and that the at least two positioning through-holes of the stimulable phosphor sheet are formed by through-holes formed at positions corresponding to the at least two positioning through-holes of the biochemical analysis unit and in which no stimulable phosphor is charged among the plurality of through-holes formed in the support of the stimulable phosphor sheet, and which comprises the steps of inserting at least two erect positioning pins formed on a substrate of an exposure device at positions corresponding to the at least two positioning through-holes formed in the substrate of the biochemical analysis unit and the at least two positioning through-holes formed in the support of the stimulable phosphor sheet into the at least two positioning through-holes formed in the substrate of the biochemical analysis unit and the at least two positioning through-holes formed in the support of the stimulable phosphor sheet, superposing the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

45. A method for exposing a stimulable phosphor sheet in accordance with Claim 34 wherein the plurality of absorptive regions of the biochemical analysis unit are formed by charging an absorptive material in a number of through-holes formed in the substrate and the plurality of stimulable phosphor layer regions of the stimulable phosphor sheet are formed by charging stimulable phosphor in a number of through-holes formed in the support so that the at least two positioning through-holes of the biochemical analysis unit are formed by through-holes in which no absorptive material is charged among the plurality of through-holes formed in the substrate of the biochemical analysis unit and that the at least two positioning through-holes of the stimulable phosphor sheet are formed by through-holes formed at positions corresponding to the at least two positioning through-holes of the biochemical analysis unit and in which no stimulable phosphor is charged among the plurality of through-holes formed in the support of the stimulable phosphor sheet, and which comprises the steps of superposing the biochemical analysis unit and the stimulable phosphor sheet, positioning the biochemical analysis unit and the stimulable phosphor sheet by detecting light emitted from a light emitting means and transmitted through the at least two positioning through-holes formed in the substrate of the biochemical analysis unit and the at least two positioning through-holes formed in the support of the stimulable phosphor sheet with a light receiving means, and exposing the plurality of stimulable phosphor layer regions of the stimulable phosphor sheet to a radioactive labeling substrate selectively contained in the plurality of absorptive regions of the biochemical analysis unit.

46. A method for exposing a stimulable phosphor sheet in accordance with Claim 34 wherein the plurality of absorptive regions of the biochemical analysis unit are formed by charging an absorptive material in a number of through-holes formed in the substrate and the plurality of stimulable phosphor layer regions of the stimulable phosphor sheet are formed by charging stimulable phosphor in a number of through-holes formed in the support so that the at least two positioning through-holes of the biochemical analysis unit are formed by through-holes in which no absorptive material is charged among the plurality of through-holes formed in the substrate of the biochemical analysis unit and that the at least two positioning through-holes of the stimulable phosphor sheet are formed by through-holes formed at positions corresponding to the at least two positioning through-holes of the biochemical analysis unit and in which no stimulable phosphor is charged among the plurality of through-holes formed in the support of the stimulable phosphor sheet, and which comprises the steps of setting one of the biochemical analysis unit and the stimulable phosphor sheet on a substrate of an exposure device, producing an image of the at least two positioning through-holes formed in the one of the biochemical analysis unit and the stimulable phosphor sheet using an imaging device, storing data corresponding to the image in a memory, superposing the other one of the biochemical analysis unit and the stimulable phosphor sheet on the surface of the one of the biochemical analysis unit and the stimulable phosphor sheet, producing an image of the at least two positioning through-holes formed in the other one of the biochemical analysis unit and the stimulable phosphor sheet using the imaging device, comparing the thus produced image with the image of the at least two positioning through-holes formed in the one of the biochemical analysis unit and the stimulable phosphor sheet based on the data stored in the memory, moving the other one of the biochemical analysis unit and the stimulable phosphor sheet so that the at least two positioning through-holes formed in the substrate of the biochemical analysis unit and the at least two positioning through-holes formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

47. A method for exposing a stimulable phosphor sheet in accordance with Claim 34 which comprises the steps of setting one of the biochemical analysis unit and the stimulable phosphor sheet on a substrate of an exposure device, producing an image of the one of the biochemical analysis unit and the stimulable phosphor sheet using an imaging device, storing data corresponding to the image in a memory, superposing the other one of the biochemical analysis unit and the stimulable phosphor sheet on the surface of the one of the biochemical analysis unit and the stimulable phosphor sheet, producing an image of the other one of the biochemical analysis unit and the stimulable phosphor sheet, comparing the thus produced image with the image of the at least two positioning through-holes formed in the one of the biochemical analysis unit and the stimulable phosphor sheet based on the data stored in the memory, moving the other one of the biochemical analysis unit and the stimulable phosphor sheet so that the plurality of absorptive regions formed in the substrate of the biochemical analysis unit and the plurality of stimulable phosphor layer regions formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to a radioactive labeling substrate selectively contained in the plurality of absorptive regions of the biochemical analysis unit.

48. A method for exposing a stimulable phosphor sheet comprising the steps of bringing a biochemical analysis unit including a substrate capable of attenuating light energy and formed with a plurality of absorptive regions spaced apart from each other and selectively labeled with a labeling substance which generates chemiluminescence emission when it contacts a chemiluminescent substrate into contact with a chemiluminescent substrate, thereby causing the plurality of absorptive regions to selectively release chemiluminescence emission, superposing the biochemical analysis unit releasing the chemiluminescence emission and the stimulable phosphor sheet including a support formed with a plurality of stimulable phosphor layer regions spaced apart from each other so that each of the plurality of absorptive regions of the biochemical analysis unit faces the corresponding stimulable layer region of the stimulable phosphor sheet and exposing the plurality of stimulable phosphor layer regions of the stimulable phosphor sheet to the chemiluminescence emission selectively released from the plurality of absorptive regions of the biochemical analysis unit.

49. A method for exposing a stimulable phosphor sheet in accordance with Claim 48 wherein the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit, and which comprises the steps of superposing the biochemical analysis unit releasing chemiluminescence emission and the stimulable phosphor sheet so that at least two erect positioning pins formed on a substrate of an exposure device at positions corresponding to the at least two positioning through-holes formed in the substrate of the biochemical analysis unit and the at least two positioning through-holes formed in the support of the stimulable phosphor sheet are inserted into the at least two positioning through-holes formed in the substrate of the biochemical analysis unit and the at least two positioning through-holes formed in the support of the stimulable phosphor sheet, thereby positioning the biochemical analysis unit and the stimulable phosphor sheet, and exposing the plurality of stimulable phosphor layer regions of the stimulable phosphor sheet to chemiluminescence emission selectively released from the plurality of absorptive regions of the biochemical analysis unit.

50. A method for exposing a stimulable phosphor sheet in accordance with Claim 48 wherein the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit, and which comprises the steps of superposing the biochemical analysis unit releasing chemiluminescence emission and the stimulable phosphor sheet, positioning the biochemical analysis unit and the stimulable phosphor sheet by detecting light emitted from a light emitting means and transmitted through the at least two positioning through-holes formed in the substrate of the biochemical analysis unit and the at least two positioning through-holes formed in the support of the stimulable phosphor sheet with a light receiving means, and exposing the plurality of stimulable phosphor layer regions of the stimulable phosphor sheet to chemiluminescence emission selectively released from the plurality of absorptive regions of the biochemical analysis unit.

51. A method for exposing a stimulable phosphor sheet in accordance with Claim 48 wherein the substrate of the biochemical analysis unit is formed with at least two positioning through-holes and the support of the stimulable phosphor sheet is formed with at least two positioning through-holes at positions corresponding to those of the at least two positioning through-holes formed in the substrate of the biochemical analysis unit, and which comprises the steps of setting one of the biochemical analysis unit releasing chemiluminescence emission and the stimulable phosphor sheet on a substrate of an exposure device, producing an image of the at least two positioning through-holes formed in the one of the biochemical analysis unit and the stimulable phosphor sheet using an imaging device, storing data corresponding to the image in a memory, superposing the other one of the biochemical analysis unit releasing chemiluminescence emission and the stimulable phosphor sheet on the surface of the one of the biochemical analysis unit and the stimulable phosphor sheet, producing an image of the at least two positioning through-holes formed in the other one of the biochemical analysis unit and the stimulable phosphor sheet using the imaging device, comparing the thus produced image with the image of the at least two positioning through-holes formed in the one of the biochemical analysis unit and the stimulable phosphor sheet based on the data stored in the memory, moving the other one of the biochemical analysis unit and the stimulable phosphor sheet so that the at least two positioning through-holes formed in the substrate of the biochemical analysis unit and the at least two positioning through-holes formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to chemiluminescence emission selectively released from the plurality of absorptive regions of the biochemical analysis unit.

52. A method for exposing a stimulable phosphor sheet in accordance with Claim 48 wherein the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit, and which comprises the steps of superposing the biochemical analysis unit releasing chemiluminescence emission and the stimulable phosphor sheet so that at least two erect positioning pins formed on a substrate of an exposure device at positions corresponding to the at least two positioning notches formed in the substrate of the biochemical analysis unit and the at least two positioning notches formed in the support of the stimulable phosphor sheet are inserted into the at least two positioning notches formed in the substrate of the biochemical analysis unit and the at least two positioning notches formed in the support of the stimulable phosphor sheet, thereby positioning the biochemical analysis unit and the stimulable phosphor sheet, and exposing the plurality of stimulable phosphor layer regions of the stimulable phosphor sheet to chemiluminescence emission selectively released from the plurality of absorptive regions of the biochemical analysis unit.

53. A method for exposing a stimulable phosphor sheet in accordance with Claim 48 wherein the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit, and which comprises the steps of superposing the biochemical analysis unit releasing chemiluminescence emission and the stimulable phosphor sheet, positioning the biochemical analysis unit and the stimulable phosphor sheet by detecting light emitted from a light emitting means and transmitted through the at least two positioning notches formed in the substrate of the biochemical analysis unit and the at least two positioning notches formed in the support of the stimulable phosphor sheet with a light receiving means, and exposing the plurality of stimulable phosphor layer regions of the stimulable phosphor sheet to chemiluminescence emission selectively released from the plurality of absorptive regions of the biochemical analysis unit.

54. A method for exposing a stimulable phosphor sheet in accordance with Claim 48 wherein the substrate of the biochemical analysis unit is formed with at least two positioning notches and the support of the stimulable phosphor sheet is formed with at least two positioning notches at positions corresponding to those of the at least two positioning notches formed in the substrate of the biochemical analysis unit, and which comprises the steps of setting one of the biochemical analysis unit releasing chemiluminescence emission and the stimulable phosphor sheet on a substrate of an exposure device, producing an image of the at least two positioning notches formed in the one of the biochemical analysis unit and the stimulable phosphor sheet using an imaging device, storing data corresponding to the image in a memory, superposing the other one of the biochemical analysis unit releasing chemiluminescence emission and the stimulable phosphor sheet on the surface of the one of the biochemical analysis unit and the stimulable phosphor sheet, producing an image of the at least two positioning notches formed in the other one of the biochemical analysis unit and the stimulable phosphor sheet using the imaging device, comparing the thus produced image with the image of the at least two positioning notches formed in the one of the biochemical analysis unit and the stimulable phosphor sheet based on the data stored in the memory, moving the other one of the biochemical analysis unit and the stimulable phosphor sheet so that the at least two positioning notches formed in the substrate of the biochemical analysis unit and the at least two positioning notches formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to chemiluminescence emission selectively released from the plurality of absorptive regions of the biochemical analysis unit.

55. A method for exposing a stimulable phosphor sheet in accordance with Claim 48 wherein at least one positioning convex portion on the surface of the substrate of the biochemical analysis unit and at least one positioning recess having a complementary shape to that of the at least one positioning convex portion at a position corresponding to that of the at least one positioning convex portion on the surface of the support of the stimulable phosphor sheet, and which comprises the steps of fitting the at least one positioning convex portion formed on the surface of the substrate of the biochemical analysis unit into the at least one positioning recess formed on the surface of the support of the stimulable phosphor sheet, thereby positioning the biochemical analysis unit releasing chemiluminescence emission and the stimulable phosphor sheet, and exposing the plurality of stimulable phosphor layer regions of the stimulable phosphor sheet to chemiluminescence emission substrate selectively released from the plurality of absorptive regions of the biochemical analysis unit.

56. A method for exposing a stimulable phosphor sheet in accordance with Claim 48 wherein at least one positioning recess on the surface of the substrate of the biochemical analysis unit and at least one positioning convex portion having a complementary shape to that of the at least one positioning recess at a position corresponding to that of the at least one positioning recess on the surface of the support of the stimulable phosphor sheet, and which comprises the steps of fitting the at least one positioning convex portion formed on the surface of the support of the stimulable phosphor sheet into the at least one positioning recess formed on the surface of the substrate of the biochemical analysis unit, thereby positioning the biochemical analysis unit releasing chemiluminescence emission and the stimulable phosphor sheet, and exposing the plurality of stimulable phosphor layer regions of the stimulable phosphor sheet to chemiluminescence emission selectively released from the plurality of absorptive regions of the biochemical analysis unit.

57. A method for exposing a stimulable phosphor sheet in accordance with Claim 48 wherein the plurality of absorptive regions are formed in the substrate of the biochemical analysis unit in a predetermined positional relationship to a first reference edge of the substrate of the biochemical analysis unit and a second reference edge of the substrate of the biochemical analysis unit perpendicular to the first reference edge and the plurality of stimulable phosphor layer regions are formed in the support of the stimulable phosphor sheet in a predetermined positional relationship to a first reference edge of the support of the stimulable phosphor sheet corresponding to the first reference edge of the substrate of the biochemical analysis unit and a second reference edge of the support of the stimulable phosphor sheet corresponding to the substrate of the biochemical analysis unit, and which comprises the steps of superposing the biochemical analysis unit releasing chemiluminescence emission and the stimulable phosphor sheet so that the first reference edge of the substrate of the biochemical analysis unit and the first reference edge of the support of the stimulable phosphor sheet align with one of two erect reference pins formed on the support of an exposure device and that the second reference edge of the substrate of the biochemical analysis unit and the second reference edge of the support of the stimulable phosphor sheet align with the other one of the two erect reference pins formed on the support of an exposure device, thereby positioning the biochemical analysis unit and the stimulable phosphor sheet, and exposing the plurality of stimulable phosphor layer regions of the stimulable phosphor sheet to chemiluminescence emission selectively released from the plurality of absorptive regions of the biochemical analysis unit.

58. A method for exposing a stimulable phosphor sheet in accordance with Claim 48 wherein the plurality of absorptive regions of the biochemical analysis unit are formed by charging an absorptive material in a number of through-holes formed in the substrate and the plurality of stimulable phosphor layer regions of the stimulable phosphor sheet are formed by charging stimulable phosphor in a number of through-holes formed in the support so that the at least two positioning through-holes of the biochemical analysis unit are formed by through-holes in which no absorptive material is charged among the plurality of through-holes formed in the substrate of the biochemical analysis unit and that the at least two positioning through-holes of the stimulable phosphor sheet are formed by through-holes formed at positions corresponding to the at least two positioning through-holes of the biochemical analysis unit and in which no stimulable phosphor is charged among the plurality of through-holes formed in the support of the stimulable phosphor sheet, and which comprises the steps of inserting at least two erect positioning pins formed on a substrate of an exposure device at positions corresponding to the at least two positioning through-holes formed in the substrate of the biochemical analysis unit and the at least two positioning through-holes formed in the support of the stimulable phosphor sheet into the at least two positioning through-holes formed in the substrate of the biochemical analysis unit and the at least two positioning through-holes formed in the support of the stimulable phosphor sheet, superposing the biochemical analysis unit releasing chemiluminescence emission and the stimulable phosphor sheet, and exposing the plurality of stimulable phosphor layer regions of the stimulable phosphor sheet to chemiluminescence emission selectively released from the plurality of absorptive regions of the biochemical analysis unit.

59. A method for exposing a stimulable phosphor sheet in accordance with Claim 48 wherein the plurality of absorptive regions of the biochemical analysis unit are formed by charging an absorptive material in a number of through-holes formed in the substrate and the plurality of stimulable phosphor layer regions of the stimulable phosphor sheet are formed by charging stimulable phosphor in a number of through-holes formed in the support so that the at least two positioning through-holes of the biochemical analysis unit are formed by through-holes in which no absorptive material is charged among the plurality of through-holes formed in the substrate of the biochemical analysis unit and that the at least two positioning through-holes of the stimulable phosphor sheet are formed by through-holes formed at positions corresponding to the at least two positioning through-holes of the biochemical analysis unit and in which no stimulable phosphor is charged among the plurality of through-holes formed in the support of the stimulable phosphor sheet, and which comprises the steps of superposing the biochemical analysis unit releasing chemiluminescence emission and the stimulable phosphor sheet, positioning the biochemical analysis unit and the stimulable phosphor sheet by detecting light emitted from a light emitting means and transmitted through the at least two positioning through-holes formed in the substrate of the biochemical analysis unit and the at least two positioning through-holes formed in the support of the stimulable phosphor sheet with a light receiving means, and exposing the plurality of stimulable phosphor layer regions of the stimulable phosphor sheet to chemiluminescence emission selectively released from the plurality of absorptive regions of the biochemical analysis unit.

60. A method for exposing a stimulable phosphor sheet in accordance with Claim 48 wherein the plurality of absorptive regions of the biochemical analysis unit are formed by charging an absorptive material in a number of through-holes formed in the substrate and the plurality of stimulable phosphor layer regions of the stimulable phosphor sheet are formed by charging stimulable phosphor in a number of through-holes formed in the support so that the at least two positioning through-holes of the biochemical analysis unit are formed by through-holes in which no absorptive material is charged among the plurality of through-holes formed in the substrate of the biochemical analysis unit and that the at least two positioning through-holes of the stimulable phosphor sheet are formed by through-holes formed at positions corresponding to the at least two positioning through-holes of the biochemical analysis unit and in which no stimulable phosphor is charged among the plurality of through-holes formed in the support of the stimulable phosphor sheet, and which comprises the steps of setting one of the biochemical analysis unit releasing chemiluminescence emission and the stimulable phosphor sheet on a substrate of an exposure device, producing an image of the at least two positioning through-holes formed in the one of the biochemical analysis unit and the stimulable phosphor sheet using an imaging device, storing data corresponding to the image in a memory, superposing the other one of the biochemical analysis unit releasing chemiluminescence emission and the stimulable phosphor sheet on the surface of the one of the biochemical analysis unit and the stimulable phosphor sheet, producing an image of the at least two positioning through-holes formed in the other one of the biochemical analysis unit and the stimulable phosphor sheet using the imaging device, comparing the thus produced image with the image of the at least two positioning through-holes formed in the one of the biochemical analysis unit and the stimulable phosphor sheet based on the data stored in the memory, moving the other one of the biochemical analysis unit and the stimulable phosphor sheet so that the at least two positioning through-holes formed in the substrate of the biochemical analysis unit and the at least two positioning through-holes formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to chemiluminescence emission selectively released from the plurality of absorptive regions of the biochemical analysis unit.

61. A method for exposing a stimulable phosphor sheet in accordance with Claim 48 which comprises the steps of setting one of the biochemical analysis unit releasing chemiluminescence emission and the stimulable phosphor sheet on a substrate of an exposure device, producing an image of the one of the biochemical analysis unit and the stimulable phosphor sheet using an imaging device, storing data corresponding to the image in a memory, superposing the other one of the biochemical analysis unit releasing chemiluminescence emission and the stimulable phosphor sheet on the surface of the one of the biochemical analysis unit and the stimulable phosphor sheet, producing an image of the other one of the biochemical analysis unit and the stimulable phosphor sheet, comparing the thus produced image with the image of the at least two positioning through-holes formed in the one of the biochemical analysis unit and the stimulable phosphor sheet based on the data stored in the memory, moving the other one of the biochemical analysis unit and the stimulable phosphor sheet so that the plurality of absorptive regions formed in the substrate of the biochemical analysis unit and the plurality of stimulable phosphor layer regions formed in the support of the stimulable phosphor sheet coincide with each other in images, thereby positioning the biochemical analysis unit and the stimulable phosphor sheet, and exposing the plurality of stimulable phosphor layer regions of the stimulable phosphor sheet to chemiluminescence emission selectively released from the plurality of absorptive regions of the biochemical analysis unit

62. A method for exposing a stimulable phosphor sheet in accordance with any one of Claims 34 to 61 wherein the substrate of the biochemical analysis unit is formed with 10 or more absorptive regions.

63. A method for exposing a stimulable phosphor sheet in accordance with any one of Claims 34 to 62 wherein each of the plurality of absorptive regions formed in the substrate of the biochemical analysis unit has a size of less than 5 mm².

64. A method for exposing a stimulable phosphor sheet in accordance with any one of Claims 34 to 63 wherein the plurality of absorptive regions are formed in the substrate of the biochemical analysis unit at a density of 10 or more per cm².

65. A method for exposing a stimulable phosphor sheet in accordance with any one of Claims 34 to 47 wherein the substrate of the biochemical analysis unit has a property of reducing the energy of radiation to 1/5 or less when the radiation travels in the substrate by a distance equal to that between neighboring absorptive regions.

66. A method for exposing a stimulable phosphor sheet in accordance with any one of Claims 48 to 61 wherein the substrate of the biochemical analysis unit has a property of reducing the energy of light to 1/5 or less when the light travels in the substrate by a distance equal to that between neighboring absorptive regions.

67. A method for exposing a stimulable phosphor sheet in accordance with any one of Claims 34 to 66 wherein the substrate of the biochemical analysis unit is made of a material selected from a group consisting of a metal material, a ceramic material and a plastic material.

68. A method for exposing a stimulable phosphor sheet in accordance with any one of Claims 34 to 67 wherein the plurality of absorptive regions of the biochemical analysis unit are made of a porous carbon material or a porous material capable of forming a membrane filter.

69. A method for exposing a stimulable phosphor sheet in accordance with any one of Claims 34 to 67 wherein the plurality of absorptive regions of the biochemical analysis unit are made of a bundle of a plurality of fibers.

70. A method for exposing a stimulable phosphor sheet in accordance with any one of Claims 34 to 47 and 62 to 69 wherein the support of the stimulable phosphor sheet has a property of attenuating radiation energy.

71. A method for exposing a stimulable phosphor sheet in accordance with Claim 70 wherein the support of the stimulable phosphor sheet has a property of reducing the radiation energy to 1/5 or less when the radiation travels in the support by a distance equal to that between neighboring stimulable phosphor layer regions.

72. A method for exposing a stimulable phosphor sheet in accordance with any one of Claims 48 to 71 wherein the support of the stimulable phosphor sheet has a property of attenuating light energy.

73. A method for exposing a stimulable phosphor sheet in accordance with Claim 72 wherein the support of the stimulable phosphor sheet has a property of reducing the energy of light to 1/5 or less when the light travels in the support by a distance equal to that between neighboring stimulable phosphor layer regions.

74. A method for exposing a stimulable phosphor sheet in accordance with any one of Claims 34 to 73 wherein the support of the stimulable phosphor sheet is made of a material selected from a group consisting of a metal material, a ceramic material and a plastic material.
